# EUROPEAN PATENT APPLICATION

(11) **EP 4 108 257 A1**
(43) Date of publication of application: **28.12.2022**
(21) Application number: 21382557.3
(22) Date of filing: 23.06.2021
(51) Int. Cl.: A61K 39/12, A61K 39/215, A61K 39/285, C07K 14/005, C12N 15/00, A61P 31/14

(54) **MVA-BASED VACCINE AGAINST COVID-19 EXPRESSING A PREFUSION-STABILIZED SARS-COV-2 S PROTEIN**

(71) Applicant: Consejo Superior De Investigaciones Científicas, 28006 Madrid (ES)
(72) Inventor: GARCÍA ARRIAZA, Juan Francisco, 28006 Madrid (ES); ESTEBAN RODRÍGUEZ, Mariano, 28006 Madrid (ES); PÉREZ RAMÍREZ, Patricia, 28006 Madrid (ES); LÁZARO FRÍAS, Adrian, 28006 Madrid (ES)
(74) Representative: Hoffmann Eitle

(57) **Abstract**

The present invention is directed a vaccine composition comprising an immunologically effective amount of a modified vaccinia virus Ankara (MVA) vector comprising at least one nucleic acid encoding the Spike (S) protein, or a fragment of said S protein comprising at least one epitope, of at least one SARS-CoV-2 variant, wherein said S protein or fragment thereof, comprises at least the substitutions R682G, R683S, R685S, A942P, K986P and V987P, and wherein the MVA vector regulates the expression of the nucleic acid encoding the S protein, or the fragment thereof. The present invention also relates to combination of vaccines and uses thereof.

## Description

### TECHNICAL FIELD OF THE INVENTION

The present invention is directed to a recombinant modified vaccinia virus Ankara (MVA) vector comprising a nucleic acid sequence coding for a prefusion-stabilized full-length spike (S) protein of at least one SARS-CoV-2 variant that is useful for vaccinating against SARS-CoV-2. The present invention is further directed to a vaccine composition containing said recombinant MVA as well as to a method for generating a protective immune response in a mammal against at least one SARS-CoV-2 variant.

### BACKGROUND OF THE INVENTION

The emergence of the severe acute respiratory syndrome coronavirus 2 (SARS-CoV-2) and its high impact on global health have made imperative the development of safe and effective vaccines for this virus, the causal agent of the pandemic coronavirus disease 2019 (COVID-19). SARS-CoV-2 now adds to the list of coronavirus diseases that have threatened global health, along with the severe acute respiratory syndrome (SARS) and Middle East respiratory syndrome (MERS) coronaviruses that emerged in 2002/2003 and 2012, respectively.

Coronavirus virions are decorated with a spike (S) glycoprotein that binds to host cell receptors and mediates cell entry via fusion of the host and viral membranes. This protein is also the main target of neutralizing antibodies. However, the SARS-CoV-2 S ectodomain is unstable and difficult to produce reliably in mammalian cells, hampering the development of S-based vaccines. Prefusion stabilization techniques are aimed at increasing the recombinant expression of viral fusion glycoproteins, possibly by preventing triggering or misfolding that results from a tendency to adopt the more stable post-fusion structure. Furthermore, prefusion-stabilized viral glycoproteins are also superior immunogens to their wild-type counterparts, since they represent the primary target of neutralizing antibodies.

Here, we provide an effective MVA-based, as well as vaccine regimens, capable of producing a prefusion-stabilized S protein from at least one SARS-CoV-2 variant virus that at the same time controls infection and disease progression caused by SARS-CoV-2/COVID-19.

### BRIEF DESCRIPTION OF THE FIGURES

### DESCRIPTION OF THE INVENTION

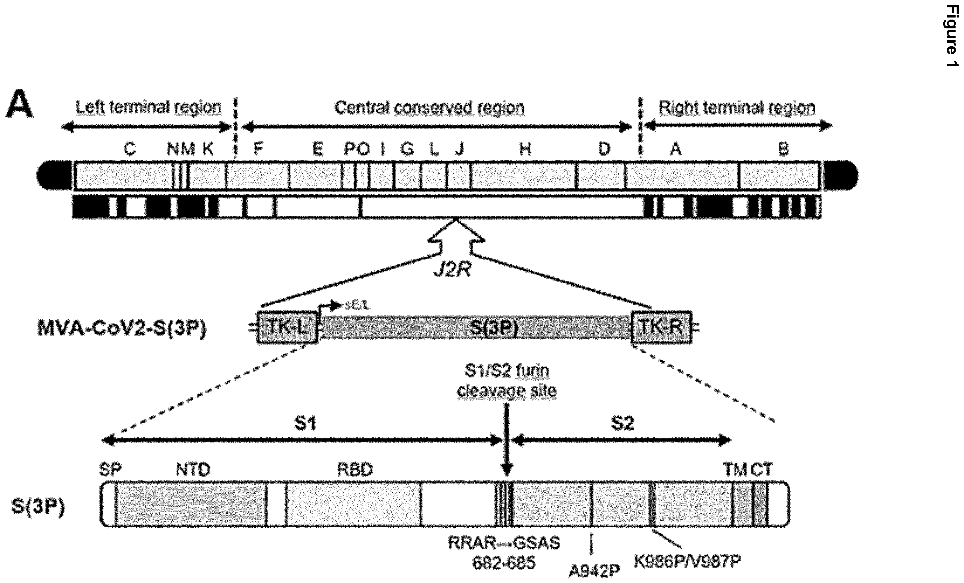

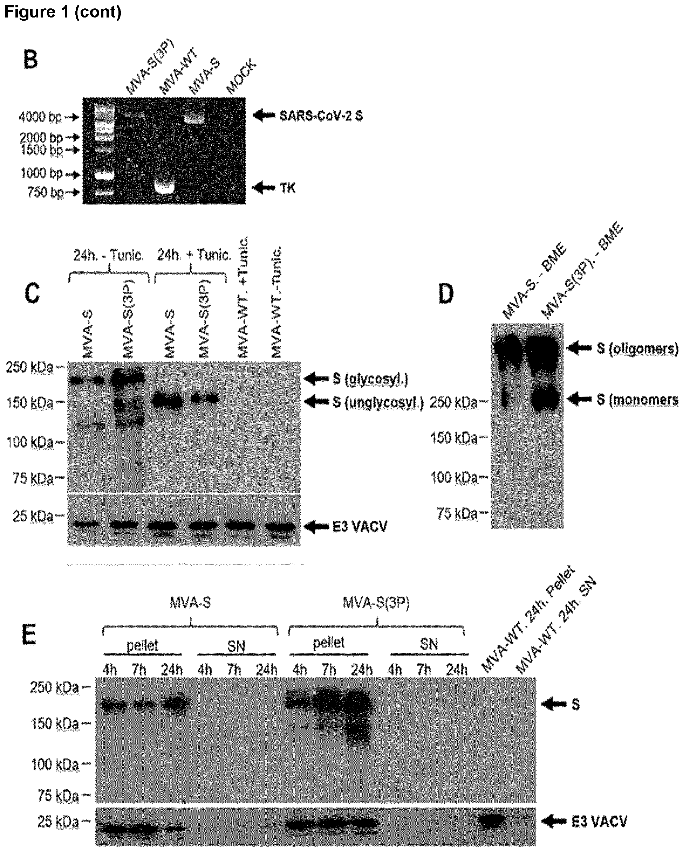

**Figure 1****. Design, generation, and in vitro characterization of MVA-S(3P) vaccine candidate in permissive DF-1 cells. (A)** Genome map of the vaccine candidate MVA-S(3P) expressing a prefusion-stabilized coronavirus SARS-CoV-2 S protein. The different regions of the MVA genome are indicated with capital letters and the central conserved region and the left and right terminal regions are shown. Below the map, the deleted or fragmented MVA genes are depicted as black boxes. The coronavirus SARS-CoV-2 S gene inserted within the MVA TK viral locus (J2R) of MVA-WT parental virus and driven by the sE/L virus promoter are indicated. TK-L: TK left; TK-R: TK right. An scheme of the prefusion-stabilized full-length S protein inserted in the MVA genomes is included. (B) PCR analysis of MVA TK locus. Viral DNA was extracted from DF-1 cells that were not infected (mock) or infected with 5 PFU/cell of vaccine candidates MVA-S and MVA-S(3P), or MVA-WT. Oligonucleotides which hybridize in the flanking regions of the TK locus were used for the PCR analysis of the coronavirus SARS-CoV-2 S gene inserted in the TK locus. A molecular weight marker (1 kb) with the corresponding bp sizes is indicated on the left side of the images, and the amplified DNA products are indicated with arrows on the right side. **(C)** Expression of the coronavirus SARS-CoV-2 S-protein. DF-1 cells were infected for 24 hours (in the absence or presence of tunicamycin) at 5 PFU/cell with vaccine candidates MVA-S and MVA-S(3P) and MVA-WT. At 24 hours post-infection cells were lysed in Laemmli 1X + β-mercaptoethanol buffer, fractionated by 7% SDS-PAGE, and the presence of the S protein was analyzed by Western blotting using a rabbit polyclonal antibody against SARS-CoV-2 (against SARS-CoV-2 S1 region). A rabbit anti-VACV E3 protein antibody was used as a loading control for the amount of viral proteins. The arrows on the right side of the images indicate the S protein (glycosylated or unglycosylated), together with the VACV E3 protein, whereas the sizes of standards (in kilodaltons [kDa]) (Precision Plus protein standards; Bio-Rad Laboratories) are indicated on the left side. **(D)** Expression of SARS-CoV-2 S protein under nonreducing conditions. DF-1 cells were infected with MVA-S and MVA-S(3P) vaccine candidates and at 24 hpi, cells were lysed under nonreducing conditions (Laemmli 1X - β-mercaptoethanol buffer), fractionated by 7% SDS-PAGE, and analyzed by Western blotting using a rabbit polyclonal antibody against SARS-CoV-2 (against SARS-CoV-2 S1 region). Arrows on the right indicates the SARS-CoV-2 S protein in the form of monomers or oligomers. The sizes of standards (in kilodaltons [kDa]) (Precision Plus protein standards; Bio-Rad Laboratories) are indicated on the left. **(E)** Kinetics of expression of the coronavirus SARS-CoV-2 S protein in cell extracts (pellet) and supernatants (SN) from cells infected with vaccine candidates MVA-S and MVA-S(3P). DF-1 cells were infected with vaccine candidates MVA-S and MVA-S(3P), and MVA-WT, at 5 PFU/cell. The cell extracts and the supernatants were collected 4, 7 and 24 hours post-infection (the supernatants were first precipitated with 10% TCA), lysed in Laemmli 1X + β-mercaptoethanol buffer, fractioned with 7% SDS-PAGE, and analyzed by Western blotting using a rabbit polyclonal antibody against SARS-CoV-2 (against SARS-CoV-2 S1 region). The arrows on the right side indicate the S protein. The sizes of standards (in kilodaltons [kDa]) (Precision Plus protein standards; Bio-Rad Laboratories) are indicated on the left.

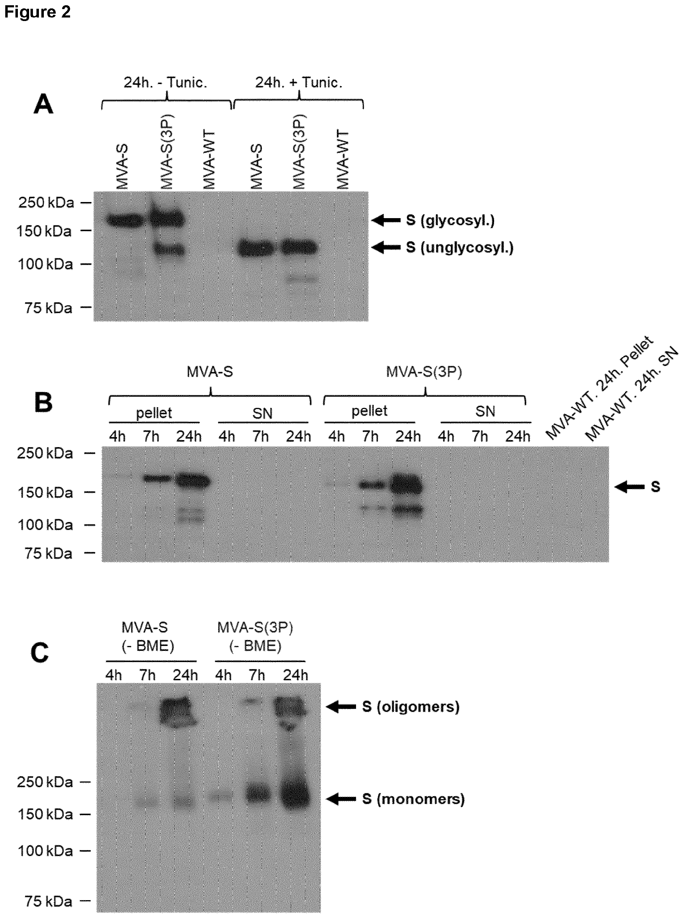

**Figure 2****. In vitro characterization of vaccine candidate MVA-S(3P) in nonpermissive human HeLa cells. (A)** Expression of the coronavirus SARS-CoV-2 S-protein. HeLa cells were infected for 24 hours (in the absence or presence of tunicamycin) at 5 PFU/cell with vaccine candidates MVA-S and MVA-S(3P) and MVA-WT. At 24 hours post-infection cells were lysed in Laemmli 1X + β-mercaptoethanol buffer, fractionated by 7% SDS-PAGE, and the presence of the S protein was analyzed by Western blotting using a rabbit polyclonal antibody against SARS-CoV-2 (against SARS-CoV-2 S1 region). The arrows on the right side of the images indicate the S protein (glycosylated or unglycosylated), whereas the sizes of standards (in kilodaltons [kDa]) (Precision Plus protein standards; Bio-Rad Laboratories) are indicated on the left side. (B) Kinetics of expression of the coronavirus SARS-CoV-2 S protein in cell extracts (pellet) and supernatants (SN) from HeLa cells infected with vaccine candidates MVA-S and MVA-S(3P). DF-1 cells were infected with vaccine candidates MVA-S and MVA-S(3P), and MVA-WT, at 5 PFU/cell. The cell extracts and the supernatants were collected 4, 7 and 24 hours post-infection (the supernatants were first precipitated with 10% TCA), lysed in Laemmli 1X + β-mercaptoethanol buffer, fractioned with 7% SDS-PAGE, and analyzed by Western blotting using a rabbit polyclonal antibody against SARS-CoV-2 (against SARS-CoV-2 S1 region). The arrows on the right side indicate the S protein. The sizes of standards (in kilodaltons [kDa]) (Precision Plus protein standards; Bio-Rad Laboratories) are indicated on the left. **(C)** Expression of SARS-CoV-2 S protein under nonreducing conditions. HeLa cells were infected with MVA-S and MVA-S(3P) vaccine candidates and at 4, 7 and 24 hpi, cells were lysed under nonreducing conditions (Laemmli 1X - β-mercaptoethanol buffer), fractionated by 7% SDS-PAGE, and analyzed by Western blotting using a rabbit polyclonal antibody against SARS-CoV-2 (against SARS-CoV-2 S1 region). Arrows on the right indicates the SARS-CoV-2 S protein in the form of monomers or oligomers. The sizes of standards (in kilodaltons [kDa]) (Precision Plus protein standards; Bio-Rad Laboratories) are indicated on the left.

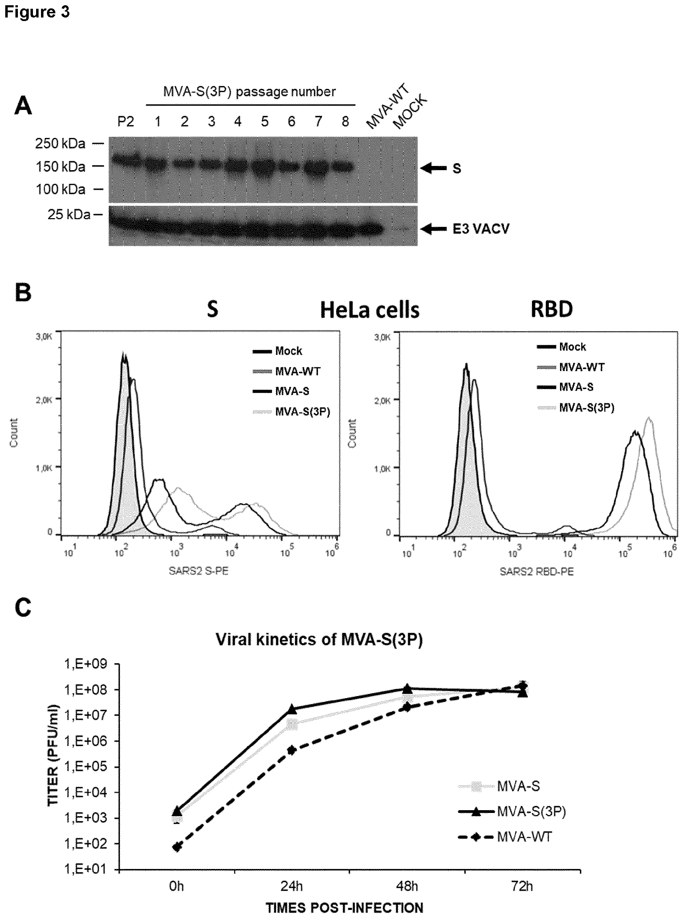

**Figure 3****. MVA-S(3P) is stable, express S in the membrane of infected cells and replicates in permissive DF-1 cells. (A)** Genetic stability of vaccine candidate MVA-S(3P). DF-1 cell monolayers were infected with the P2 stock of MVA-S(3P) at a low MOI for 8 serial passages. Then, chick DF-1 cells were the left uninfected (*mock*) or infected with vaccine candidate MVA-S(3P) from the different passages, or with MVA-WT, and the cells were lysed at 24 hours post-infection in Laemmli 1X + β-mercaptoethanol buffer, fractionated by 7% SDS-PAGE, and analyzed by Western Blotting using a rabbit polyclonal antibody against SARS-CoV-2 (against SARS-CoV-2 S1 region). A rabbit anti-VACV E3 protein antibody was used as a loading control for the amount of viral proteins. The arrows on the right side indicate the position of the S protein or the VACV E3 protein. The sizes of standards (in kilodaltons [kDa]) (Precision Plus protein standards; Bio-Rad Laboratories) are indicated on the left. **(B)** Flow cytometry analysis of S expression levels in cells infected with MVA-S(3P). HeLa cells were mock infected or infected at 5 PFU/cell with MVA-S, MVA-S(3P) and MVA-WT, and at 24 hpi, cells were collected and analyzed by flow cytometry using mouse polyclonal antibodies against S and RBD proteins, followed by an anti-mouse PE-conjugated antibody. The cell surface expression of S is represented in the histogram plots. **(C)** Viral growth kinetics of vaccine candidate MVA-S(3P). DF-1 cell monolayers were infected with MVA-WT, MVA-S, and MVA-S(3P) at 0.01 PFU/cell. Cells were collected at different times post-infection (0, 24, 48, and 72 hours) and the viral titers in the cell lysates were quantified by a plaque immunostaining assay with anti-VACV antibodies. The means and standard deviations of the results obtained from two independent experiments are shown.

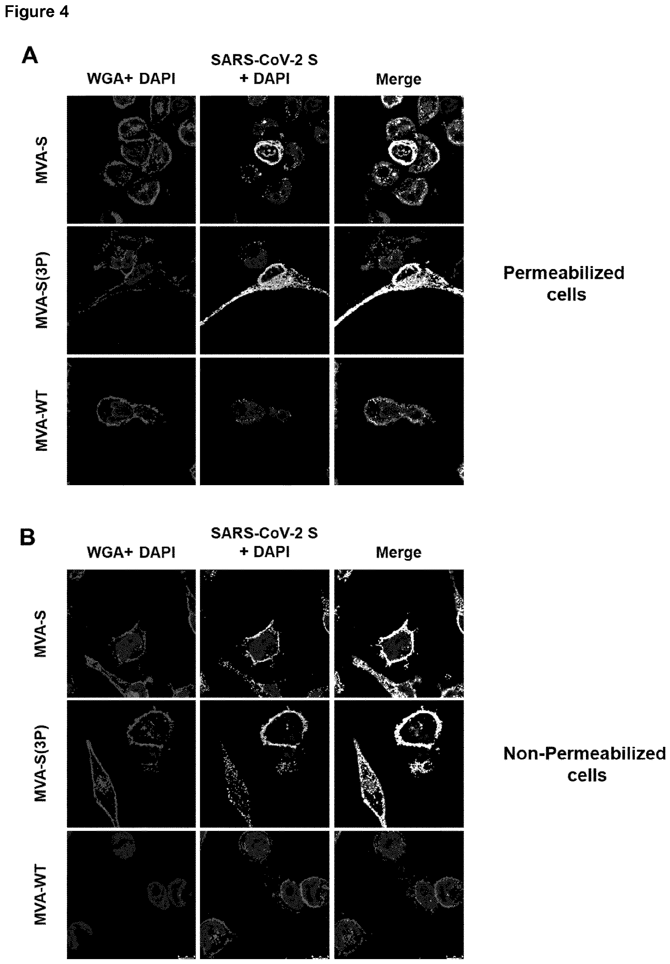

**Figure 4****. Expression of the S protein in the cell membrane of HeLa cells infected with MVA-CoV2-S(3P), and analyzed by immunofluorescence.** Subcellular distribution of the SARS-CoV-2 S protein in cells infected with MVA-S and MVA-S(3P). Confocal immunofluorescence microscopy of infected and permeabilized (A) or nonpermeabilized **(B)** HeLa cells. Cells were infected with 0.5 PFU/cell with the indicated viruses, fixed at 24 hpi, permeabilized or nonpermeabilized, and stained with Alexa Fluor 594-conjugated WGA probe (red) and with a rabbit polyclonal anti-S antibody further detected with a rabbit Alexa Fluor 488-conjugated antibody (green). Cell nuclei were stained using DAPI (blue).

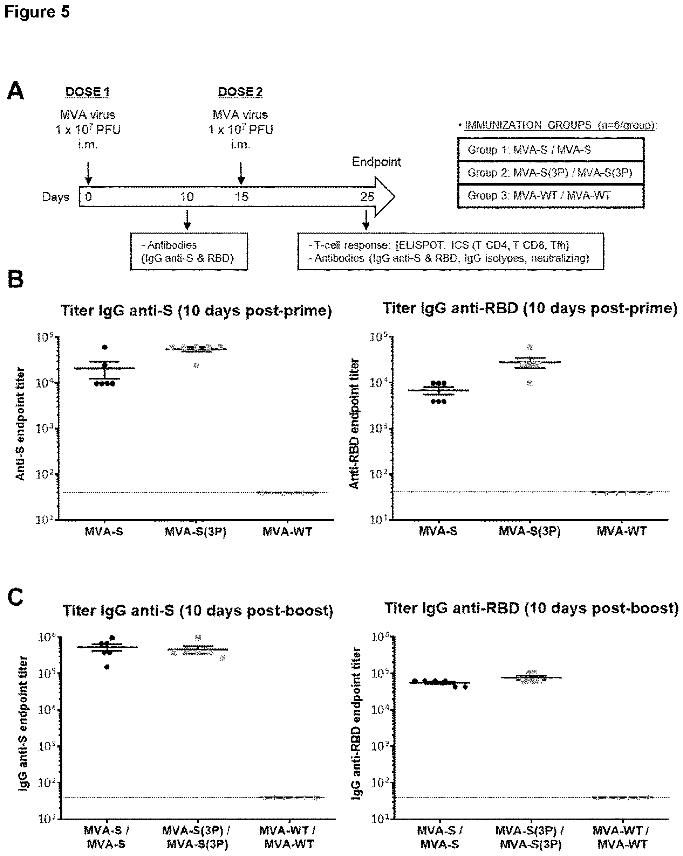

**Figure 5****. Immunization schedule and titers of IgG anti-S and anti-RBD antibodies induced in mice after one or two doses of MVA-S and MVA-S(3P) vaccine candidates. (A)** Immunization schedule. Six C57BL/6 mice per group were inoculated at days 0 and 15 (prime/boost) with the different indicated vaccine candidates (MVA-S or MVA-S(3P)). Immunization groups are indicated (n=6 mice per group), together with the time points at which animals were immunized (dose and route of administration are shown) and sacrificed to analyze the adaptive SARS-CoV-2-specific T cell and humoral immune responses. At day 10 post-prime mice were bleed to obtain serum samples. The mice were sacrificed 10 days after the boost (day 25) and the following was obtained: i) spleens of the mice for studying adaptive T cell-mediated immune responses against SARS-CoV-2 by means of ELISpot and ICS, and ii) serum samples originating from blood for studying adaptive humoral immune responses against SARS-CoV-2, by means of ELISA and a neutralization assay. PFU: Plaque forming units; i.m.: intramuscular. **(B and C)** Titers of binding IgG antibodies specific for the S and RBD proteins at 10 days post-prime **(B)** or 10 days post-boost **(C).** Titers were determined by ELISA in individual mice serum samples collected 10 days post-prime or 10 days post-boost immunization, and were calculated as the serum dilution (Y axis) at which the absorbance at 450 nm was three times higher than the naive serum value in each of the indicated immunization regimens, shown in the X axis. Dotted line represented the detection limit.

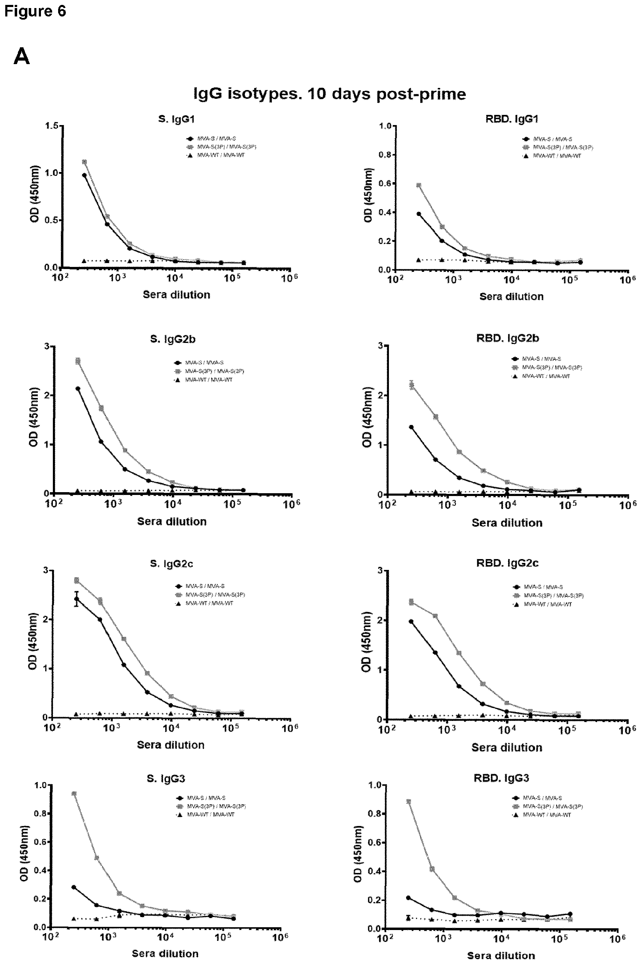

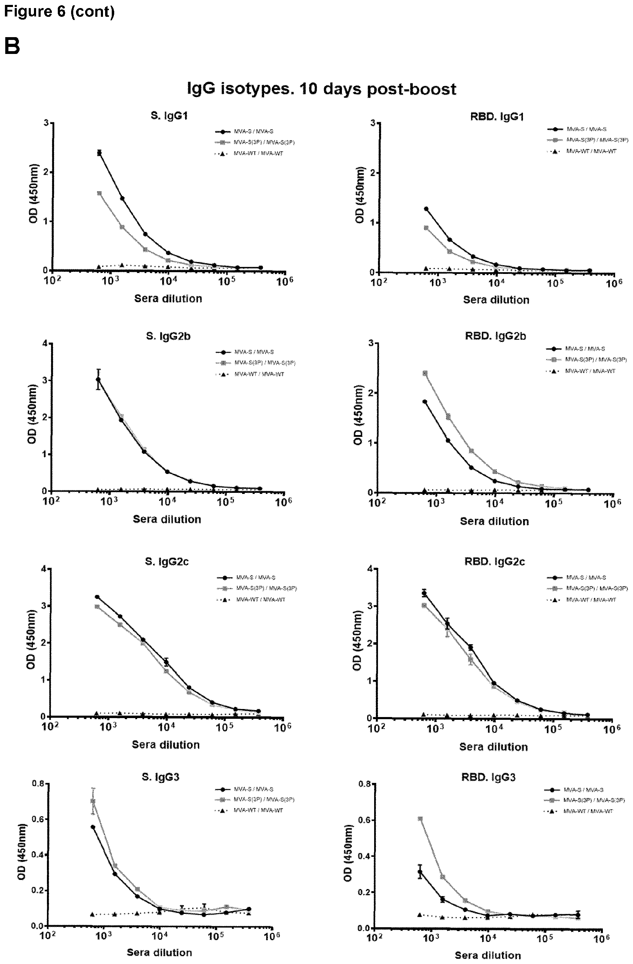

**Figure 6****. SARS-CoV-2 S-specific and RBD-specific IgG1, IgG2b, IgG2c, and IgG3 antibodies induced by vaccine candidate MVA-S(3P) in C57BL/6 mice immunized.** Groups of C57BL/6 mice (n=6) were immunized as represented in Figure 5A. At 10 days post-prime **(A)** and 10 days post-boost **(B),** serum samples were collected, and the levels of total coronavirus SARS-CoV-2 S-specific and RBD-specific IgG1, IgG2b, IgG2c, and IgG3 in pooled sera from mice derived from each immunization group were analyzed by ELISA. Mean absorbance values (measured at 450 nm) and standard deviations of duplicate pooled serum dilutions are represented.

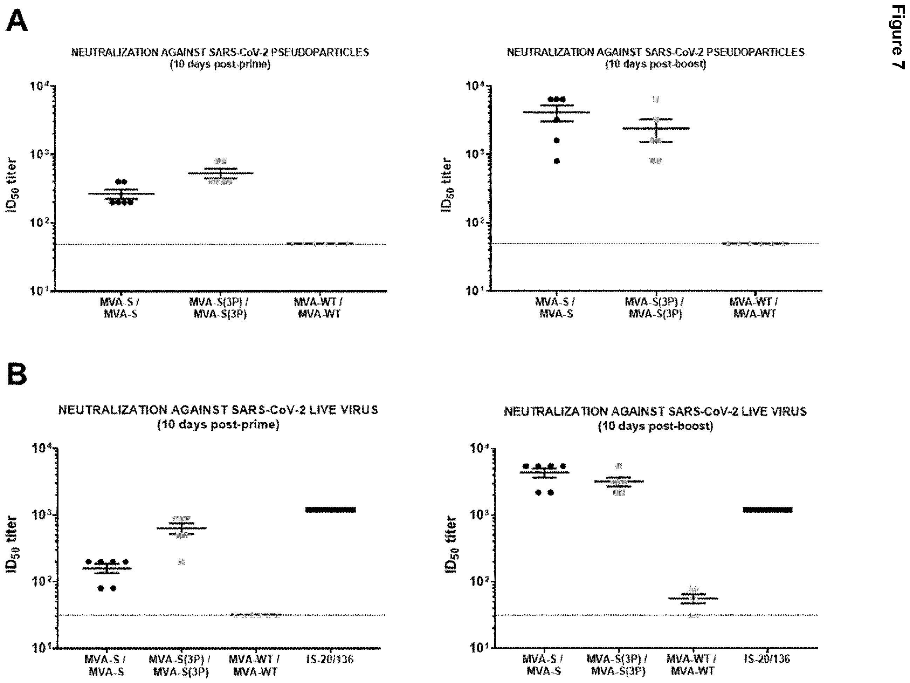

**Figure 7****. Neutralizing antibody titers against SARS-CoV-2 induced by vaccine candidate MVA-S(3P) in C57BL/6 immunized mice.** Groups of C57BL/6 mice (n=6) were immunized as represented in Figure 5A. At 10 days post-prime (left panels) and 10 days post-boost (right panels), individual serum samples were collected, and the ID50 neutralizing antibody titers were evaluated using retrovirus-based pseudoparticles expressing the SARS-CoV-2 S protein **(A)** or live-virus microneutralization **(B)** assays. The ID50 titers in the retrovirus-based pseudoparticle assay were determined as the highest serum dilution which resulted in a 50% reduction of luciferase units compared with pseudotyped viruses not incubated with serum. The ID50 titers in the live-virus microneutralization assay were calculated as the reciprocal dilution resulting in 50% inhibition of cell death. Mean ID50 values and SEM for each immunization group is represented. Dotted line represented the detection limit. The NIBSC 20/136 international standard containing pooled plasma obtained from eleven individuals recovered from SARS-CoV-2 infection is included.

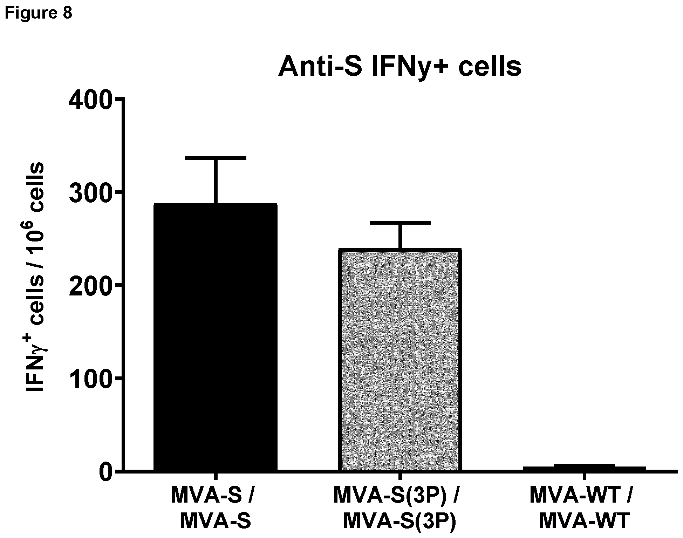

**Figure 8****. ELISpot analysis of the adaptive SARS-CoV-2 S-specific cell-mediated immune responses induced by vaccine candidate MVA-S(3P) in C57BL/6 mice.** Six mice per group were sacrificed at 10 days post-boost, and the splenic SARS-CoV-2-specific cells secreting IFN-γ were evaluated by an ELISpot assay, as described in Materials and Methods. Samples from each group of mice were analysed in triplicate, and the bars indicate the mean values and the standard deviation. The responses obtained have been obtained by subtracting the RPMI values for each condition and are represented as IFN-γ positive cells per million of splenocytes. The magnitude of SARS-CoV-2-specific cells secreting IFN-γ and directed against SARS-CoV-2 S1 and S2 peptide pools is represented.

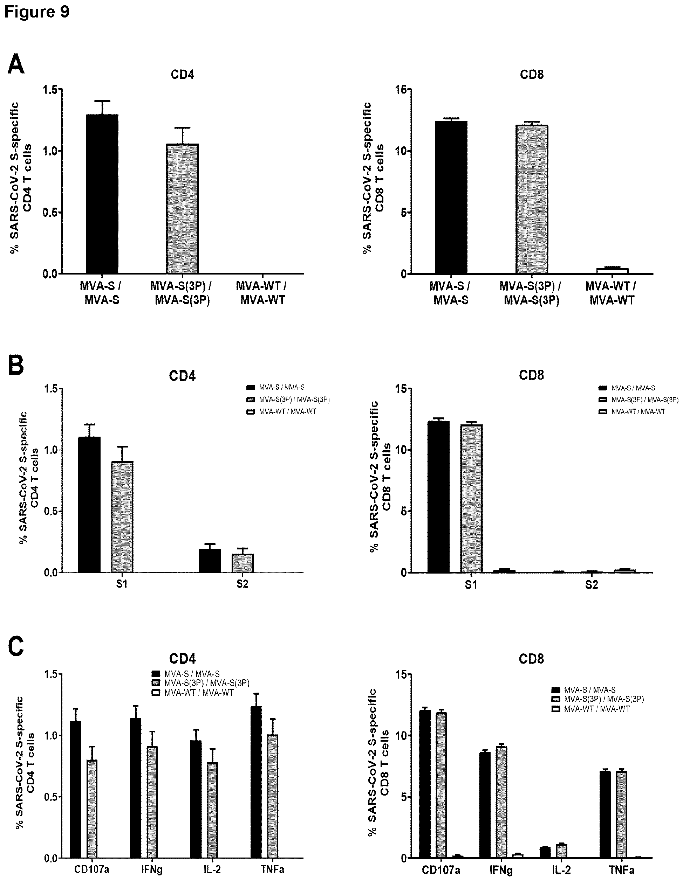

**Figure 9****. SARS-CoV-2 S-specific adaptive CD4+ (left) and CD8+ (right) T cell-mediated immune responses induced by vaccine candidate MVA-S(3P) in C57BL/6 mice.** CD4+ and CD8+ T cell-mediated responses in the splenocytes of mice (n=6) immunized with homologous (MVA/MVA) prime-boost protocol were analysed by ICS, as described in Materials and Methods 10 days after the boost. The data is depicted with its corresponding confidence intervals. **(A)** The total magnitude of SARS-CoV-2 S (S1 + S2)-specific adaptive CD4+ T cell-mediated (left) and CD8+ T cell-mediated (right) immune responses. The y-axis shows the percentage measured as the sum of SARS-CoV-2 S (mixture of S1 + S2 peptide pools)-specific T cells expressing CD107a and/or producing IFN-γ and/or TNF-α and/or IL-2. **(B)** Breadth of SARS-CoV-2-specific CD4+ and CD8+ T cell adaptive immune responses. S1 or S2 peptide pool-specific adaptive CD4+ T cell-mediated (left) and CD8+ T cell-mediated (right) immune responses. The y-axis shows the percentage measured as the sum of SARS-CoV-2 S1 or S2 peptide pool-specific T cells expressing CD107a and/or producing IFN-γ and/or TNF-α and/or IL-2. (C) Adaptive CD4+ T cell-mediated (left) and CD8+ T cell-mediated (right) immune responses shown as the percentage of SARS-CoV-2 S (S1 + S2)-specific cells expressing CD107a or producing IFN-γ or TNF-α or IL-2.

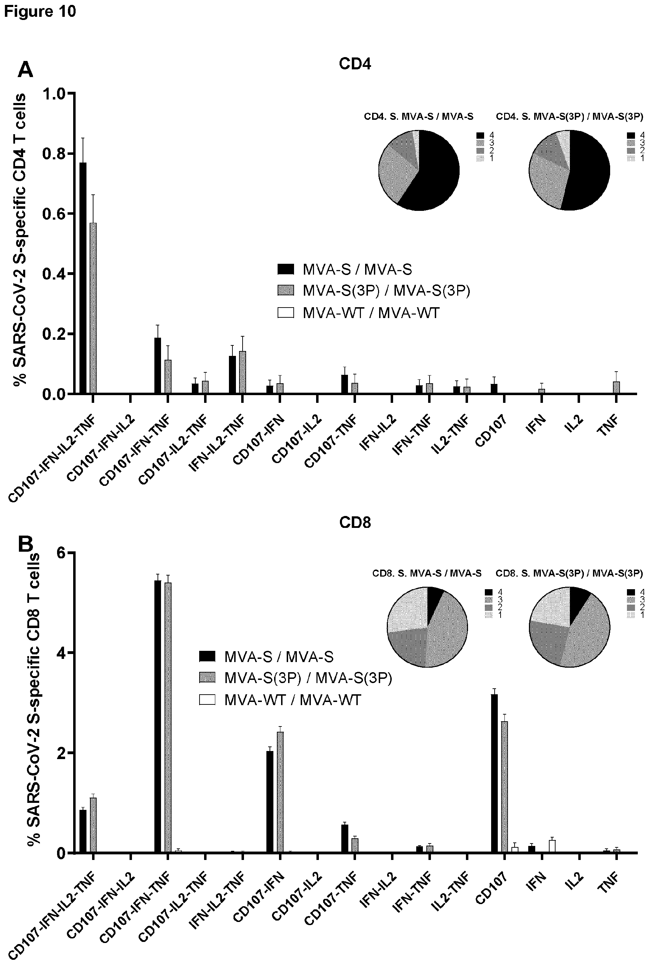

**Figure 10****. Polyfunctionality of SARS-CoV-2 S-specific adaptive CD4+ and CD8+ T cell-mediated immune responses induced by vaccine candidate MVA-S(3P) in C57BL/6 mice.** Six mice per group were sacrificed at 10 days post-boost, and the splenic SARS-CoV-2-specific CD4+ and CD8+ T cell immune responses were analysed by ICS, as described in Materials and Methods. Polyfunctional profiles of total SARS-CoV-2-specific CD4+ **(A)** and CD8+ **(B)** T cell adaptive immune responses directed against a mixture of S1, and S2 SARS-CoV-2 S peptide pools. All of the possible combinations of responses are shown on the x axis, while the percentages of T cells expressing CD107a and/or IFN-γ and/or TNF-α and/or IL-2 against S1 plus S2 peptide pools are shown on the y axis. The pie charts summarize the data.

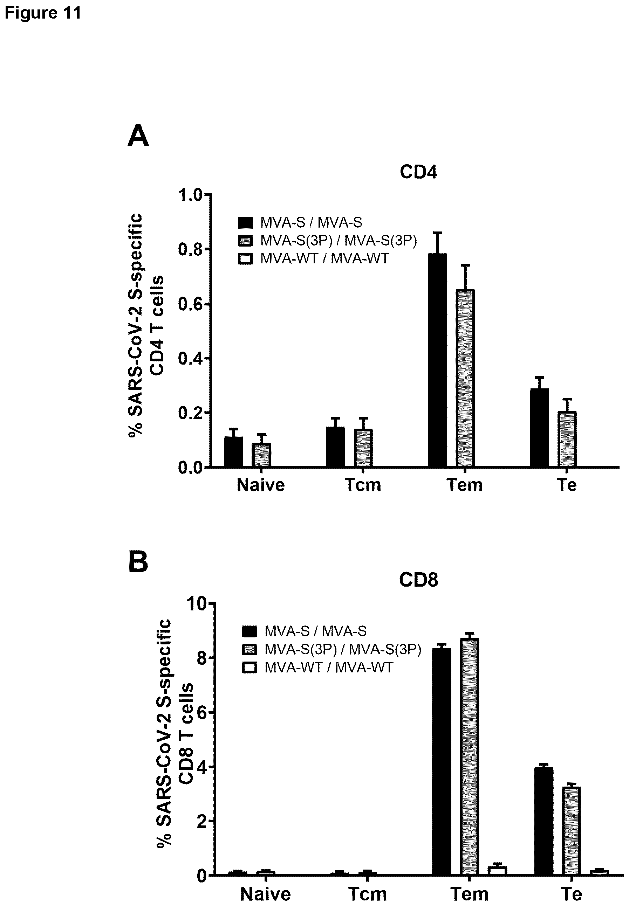

**Figure 11****. Phenotype profile of SARS-CoV-2 S-specific adaptive CD4+ and CD8+ T cells induced by vaccine candidate MVA-S(3P) in C57BL/6 mice.** Splenocytes of mice (n=6) immunized with a homologous (MVA/MVA) prime-boost protocol were obtained 10 days after the boost, and SARS-CoV-2 S (S1 +S2 peptide pools)-specific CD4+ T cell-mediated **(A)** and CD8+ T cell-mediated **(B)** immune responses were analysed by ICS. The y-axis indicates the percentage of SARS-CoV-2 S (S1 +S2)-specific CD4+ and CD8+ central memory T cells (TCM; CD127+, CD62L+), effector memory T cells (TEM; CD127+, CD62L-), and effector T cells (TE; CD127-, CD62L-) expressing CD107a and/or IFN-γ and/or TNF-α and/or IL-2 (total response). The data is depicted with its corresponding confidence intervals.

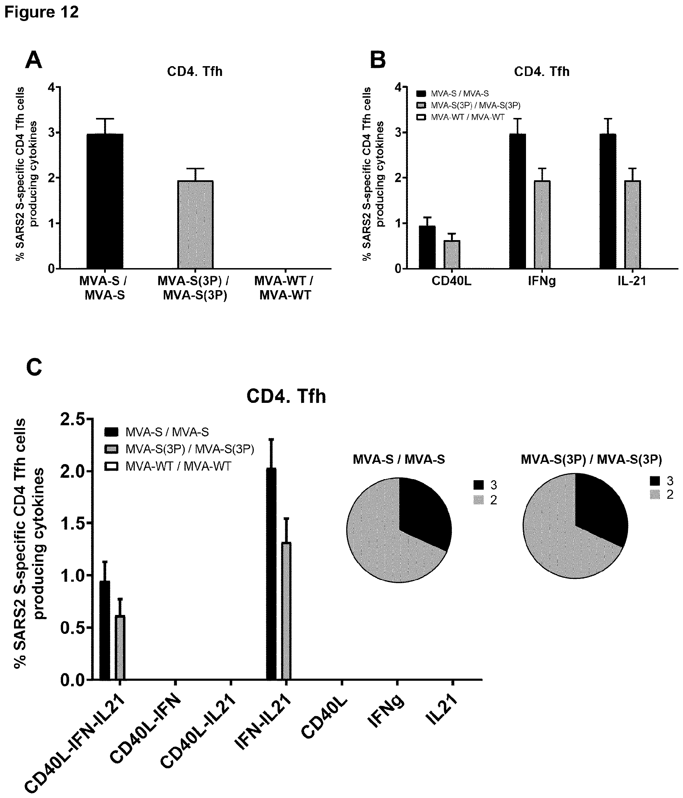

**Figure 12****. SARS-CoV-2 S-specific adaptive CD4+ T follicular helper (Tfh) cell-mediated immune responses induced by vaccine candidate MVA-S(3P) in C57BL/6 mice.** Splenocytes of mice (n=6) immunized with a homologous (MVA/MVA) prime-boost protocol were obtained 10 days after the boost, and SARS-CoV-2 S (S protein plus S1 +S2 peptide pools)-specific CD4+ Tfh cell-mediated immune responses were analysed by ICS. The data is depicted with its corresponding confidence intervals. **(A)** Magnitude of total SARS-CoV-2-specific adaptive CD4+ Tfh cell immune responses directed against SARS-CoV-2 S protein plus S1 and S2 peptide pools. Percentages of CD4+ Tfh cells (CXCR5+, PD1+) expressing CD40L and/or producing IFN-γ and/or IL-21 against a mixture of SARS-CoV-2 S protein plus S1, and S2 SARS-CoV-2 S peptide pools are represented. **(B)** Percentages of total SARS-CoV-2-specific CD4+ Tfh cells expressing CD40L or producing IFN-γ or IL-21 against a mixture of SARS-CoV-2 S protein plus S1, and S2 SARS-CoV-2 S peptide pools. **(C)** Polyfunctionality of total SARS-CoV-2-specific adaptive CD4+ Tfh cells directed against a mixture of SARS-CoV-2 S protein plus S1 and S2 peptide pools. All of the possible combinations of responses are shown on the x axis, while the percentages of T cells expressing CD40L and/or IFN-γ and/or IL-21 against SARS-CoV-2 S protein plus S1 and S2 peptide pools are shown on the y axis.

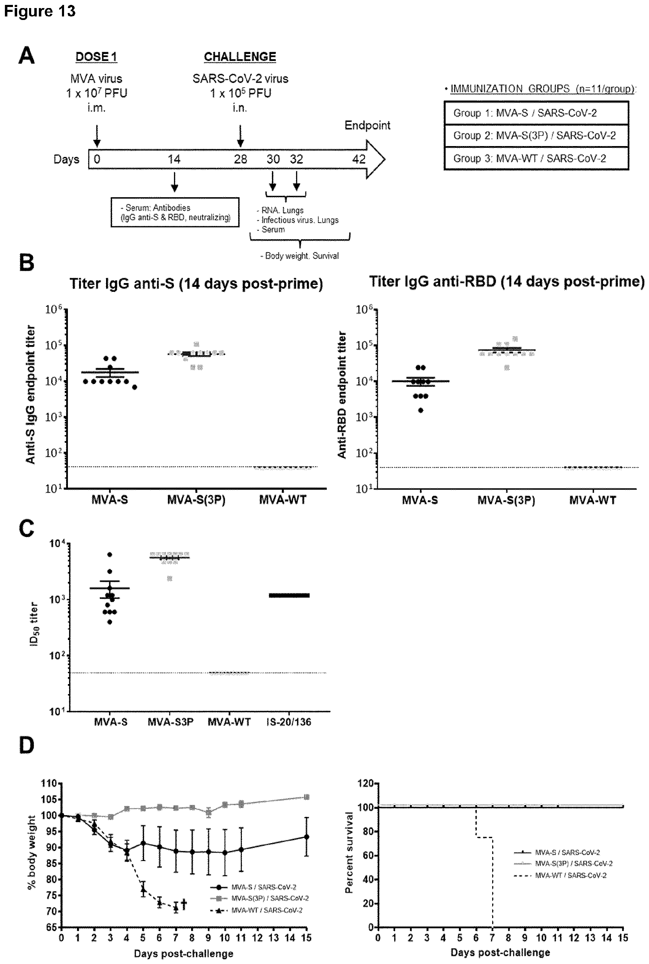

**Figure 13****. One dose of MVA-S(3P) is highly immunogenic and fully protects humanized K18-hACE2 mice from SARS-CoV-2 infection. (A)** Efficacy study schedule. K18-hACE2 mice (n=11 per group) were immunized with one dose of 1 × 10⁷ PFU of MVA-S(3P), MVA-S or MVA-WT by intramuscular route at week 0. At week 4 mice were intranasally (i.n.) challenged with 1 × 10⁵ PFU of SARS-CoV-2 (MAD6 isolate). At 14 days post-immunization mice were bleed to obtain serum samples. **(B)** Titers of IgG antibodies against S and RBD, after one dose of MVA-S and MVA-S(3P) vaccine candidate, at 14 days post-immunization. **(C)** Neutralizing antibody titers against live SARS-CoV-2 virus after one dose of MVA-S and MVA-S(3P) vaccine candidates, at 14 days post-immunization. The ID50 titers were evaluated in individual mouse serum samples using a live-virus microneutralization assay. The ID50 titers were calculated as the reciprocal dilution resulting in 50% inhibition of cell death. Mean ID50 values and SEM for each immunization group is represented. Dotted line represented the detection limit. The NIBSC 20/136 international standard containing pooled plasma obtained from eleven individuals recovered from SARS-CoV-2 infection is included. **(D)** Morbidity and mortality. The challenged mice were monitored for change of body weights (left) and mortality (right) for 15 days. Mice not vaccinated loss more than 25% of the initial body weight and have to be sacrificed at days 6-7 post-challenge.

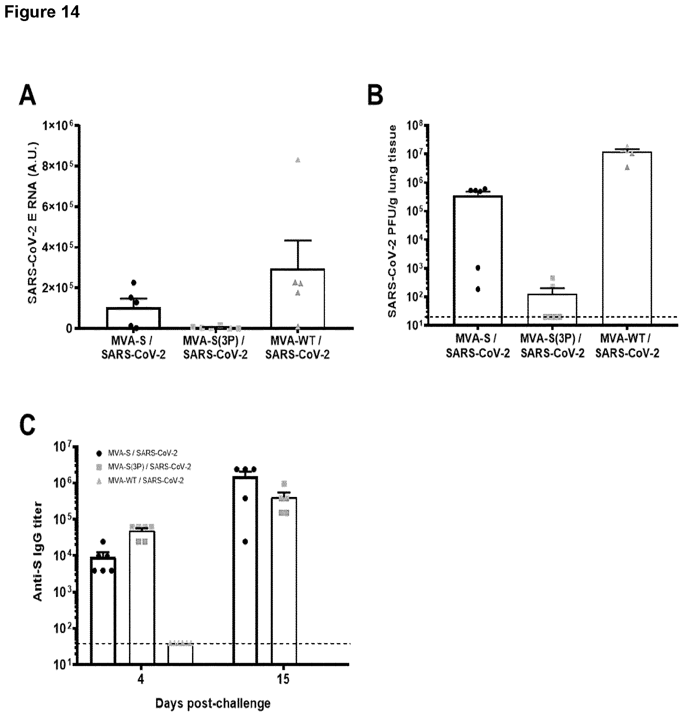

**Fig. 14****. MVA-S(3P) vaccine candidate is highly efficacious in K18-hACE2 humanized mice, preventing SARS-CoV-2 virus replication, and induced high levels of IgG antibodies. (A)** SARS-CoV-2 RNA. Detected by RT-qPCR targeting subgenomic viral E gene with normalization to 28S in lungs obtained at 4 days post-challenge (n = 6) from mice vaccinated and infected as in Fig. 13A. Mean RNA levels (in arbitrary units [A.U.]) and SEM from duplicates of each lung sample is represented for each immunization group; relative values are referred to uninfected mice. **(B)** SARS-CoV-2 infectious virus. Detected by a plaque assay in Vero E6 cells in homogenate lung samples obtained at 4 days postchallenge (n = 6) from mice vaccinated and infected as in Fig. 13A. Mean levels (in PFU/g of lung tissue) and SEM from triplicates of each lung sample is represented for each immunization group. **(C)** Titers of IgG antibodies specific for the S protein. Titers were determined by ELISA in individual mouse serum samples collected at days 4 and 15 post-challenge from immunized K18-hACE2 mice and were calculated as the serum dilution at which the absorbance at 450 nm was at least three times higher than a naive serum value in each of the indicated immunization regimens. Mean values and SEM is represented for each immunization group. The dashed line represents the detection limit.

### General definitions

It must be noted that, as used herein, the singular forms "a", "an", and "the", include plural references unless the context clearly indicates otherwise. Thus, for example, reference to "an antigenic determinant" includes one or more antigenic determinants and reference to "the method" includes reference to equivalent steps and methods known to those of ordinary skill in the art that could be modified or substituted for the methods described herein.

Unless otherwise indicated, the term "at least" preceding a series of elements is to be understood to refer to every element in the series. Those skilled in the art will recognize or be able to ascertain using no more than routine experimentation, many equivalents to the specific embodiments of the invention described herein. Such equivalents are intended to be encompassed by the present invention.

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step. When used herein the term "comprising" can be substituted with the term "containing" or "including" or sometimes when used herein with the term "having". Any of the aforementioned terms (comprising, containing, including, having), whenever used herein in the context of an aspect or embodiment of the present invention may be substituted with the term "consisting of", though less preferred.

When used herein "consisting of" excludes any element, step, or ingredient not specified in the claim element. When used herein, "consisting essentially of does not exclude materials or steps that do not materially affect the basic and novel characteristics of the claim.

As used herein, the conjunctive term "and/or" between multiple recited elements is understood as encompassing both individual and combined options. For instance, where two elements are conjoined by "and/or", a first option refers to the applicability of the first element without the second. A second option refers to the applicability of the second element without the first. A third option refers to the applicability of the first and second elements together. Any one of these options is understood to fall within the meaning, and therefore satisfy the requirement of the term "and/or" as used herein. Concurrent applicability of more than one of the options is also understood to fall within the meaning, and therefore satisfy the requirement of the term "and/or."

Unless otherwise noted, the expression "at least" or "at least one of" as used herein includes individually each of the recited objects after the expression and the various combinations of two or more of the recited objects unless otherwise understood from the context and use.

The term "subject" as used herein is a living multi-cellular vertebrate organism, including, for example, humans, non-human mammals and (non-human) primates. The term "subject" may be used interchangeably with the term "animal" herein.

As used herein, the term "enhanced" when used with respect to an immune response against SARS-CoV-2, such as an antibody response (e.g., neutralizing antigen specific antibody response), a cytokine response or a CD8 or CD4 T cell response (e.g., immunodominant T cell response), refers to an increase in the immune response in an animal administered with recombinant MVA according to the present invention relative to the corresponding immune response observed from the animal administered with a vector that does not express any SARS-CoV-2 proteins, e.g., a MVA-WT vector.

"Modified vaccinia virus Ankara (MVA) vector" as used herein is an attenuated replication-deficient vaccinia virus that has been genetically modified to include in its genome foreign DNA (that is, DNA that does not naturally belong to poxviruses). In particular, the MVA vector of the present invention has been genetically modified to express SARS-CoV-2 protein(s). Thus, the MVA vector of the present invention refers to viral particles of MVA that comprise inside the viral particle the MVA genome, wherein at least one nucleic acid encoding an antigenic protein of at least one SARS-CoV-2 virus subtype has been introduced in the MVA genome.

As used herein, term "antigenic protein" or "antigenic determinant" refers to any molecule that stimulates a host's immune system to make an antigen-specific immune response, whether a cellular response or a humoral antibody response. Antigenic determinants may include proteins, polypeptides, antigenic protein fragments, antigens, and epitopes which still elicit an immune response in a host and form part of an antigen, homologues or variants of proteins, polypeptides, and antigenic protein fragments, antigens and epitopes including, for example, glycosylated proteins, polypeptides, antigenic protein fragments, antigens and epitopes, and nucleotide sequences encoding such molecules. Thus, proteins, polypeptides, antigenic protein fragments, antigens and epitopes are not limited to particular native nucleotide or amino acid sequences but encompass sequences identical to the native sequence as well as modifications to the native sequence, such as deletions, additions, insertions and substitutions.

The term "epitope" refers to a site on an antigen to which B- and/or T-cells respond, either alone or in conjunction with another protein such as, for example, a major histocompatibility complex ("MHC") protein or a T-cell receptor. Epitopes can be formed both from contiguous amino acids or noncontiguous amino acids juxtaposed by secondary and/or tertiary folding of a protein. Epitopes formed from contiguous amino acids are typically retained on exposure to denaturing solvents, while epitopes formed by tertiary folding are typically lost on treatment with denaturing solvents. An epitope typically includes at least 5, 6, 7, 8, 9, 10 or more amino acids - but generally less than 20 amino acids - in a unique spatial conformation. Methods of determining spatial conformation of epitopes include, for example, x-ray crystallography and 2-dimensional nuclear magnetic resonance. See, e.g., "Epitope Mapping Protocols" in Methods in Molecular Biology, Vol. 66, Glenn E. Morris, Ed (1996).

Preferably, a homologue or variant has at least about 50%, at least about 60% or 65%, at least about 70% or 75%, at least about 80%, 81 %, 82%, 83%, 84%, 85%, 86%, 87%, 88%, or 89%, more typically, at least about 90%, 91 %, 92%, 93%, or 94% and even more typically at least about 95%, 96%, 97%, 98% or 99%, most typically, at least about 99% identity with the referenced protein, polypeptide, antigenic protein fragment, antigen and epitope at the level of nucleotide or amino acid sequence.

Techniques for determining sequence identity between nucleic acids and amino acids are known in the art. Two or more sequences can be compared by determining their "percent identity." The percent identity of two sequences, whether nucleic acid or amino acid sequences, is the number of exact matches between two aligned sequences divided by the length of the shorter sequences and multiplied by 100.

"Percent (%) amino acid sequence identity" with respect to proteins, polypeptides, antigenic protein fragments, antigens and epitopes described herein is defined as the percentage of amino acid residues in a candidate sequence that are identical with the amino acid residues in the reference sequence (i.e., the protein, polypeptide, antigenic protein fragment, antigen or epitope from which it is derived), after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for example, using publically available computer software such as BLAST, ALIGN, or Megalign (DNASTAR) software. Those skilled in the art can determine appropriate parameters for measuring alignment, including any algorithms needed to achieve maximum alignment over the full-length of the sequences being compared.

The same applies to "percent (%) nucleotide sequence identity", mutatis mutandis.

For example, an appropriate alignment for nucleic acid sequences is provided by the local homology algorithm of Smith and Waterman, (1981), Advances in Applied Mathematics 2:482-489. This algorithm can be applied to amino acid sequences by using the scoring matrix developed by Dayhoff, Atlas of Protein Sequences and Structure, M. O. Dayhoff ed., 5 suppl. 3:353-358, National Biomedical Research Foundation, Washington, D.C., USA, and normalized by Gribskov (1986), Nucl. Acids Res. 14(6):6745-6763. An exemplary implementation of this algorithm to determine percent identity of a sequence is provided by the Genetics Computer Group (Madison, Wis.) in the "BestFit" utility application. The default parameters for this method are described in the Wisconsin Sequence Analysis Package Program Manual, Version 8 (1995) (available from Genetics Computer Group, Madison, Wis.). A preferred method of establishing percent identity in the context of the present invention is to use the MPSRCH package of programs copyrighted by the University of Edinburgh, developed by John F. Collins and Shane S. Sturrok, and distributed by IntelliGenetics, Inc. (Mountain View, California). From this suite of packages, the Smith-Waterman algorithm can be employed where default parameters are used for the scoring table (for example, gap open penalty of 12, gap extension penalty of one, and a gap of six). From the data generated the "Match" value reflects "sequence identity." Other suitable programs for calculating the percent identity or similarity between sequences are generally known in the art, for example, another alignment program is BLAST, used with default parameters. For example, BLASTN and BLASTP can be used using the following default parameters: genetic code=standard; filter=none; strand=both; cutoff=60; expect=10; Matrix=BLOSUM62; Descriptions=50 sequences; sort by=HIGH SCORE; Databases=non-redundant, GenBank+EMBL+DDBJ+PDB+ GenBank CDS translations+Swiss protein+Spupdate+PIR. Details of these programs can be found at the following internet address: http://wvw.ncbi.nlm.gov/cqi-bin/BLAST.

The term "subtype" herein includes strains, isolates, clades, lineages, linages, and/or variants of any severe acute respiratory syndrome coronavirus, namely SARS-CoV-2. The terms "subtype" "strain" "clade", "lineage or linage", "isolate" and/or "variant" are technical terms, well known to the skilled person, referring to the taxonomy of microorganisms, that is, referring to all characterized microorganisms into the hierarchic order of Families, Genera, Species, Strains. While the criteria for the members of a Family is their phylogenetic relationship, a Genera comprises all members which share common characteristics, and a Species is defined as a polythetic class that constitutes a replicating lineage and occupies a particular ecological niche. The term "strain" or "clade" describes a microorganism, for instance, a virus, which shares common characteristics with other microorganisms, like basic morphology or genome structure and organization, but varies in biological properties, like host range, tissue tropism, geographic distribution, attenuation or pathogenicity. The term "variant" describes a microorganism, in the present invention, a virus, which replicates and introduces one or more new mutations into its genome which results in differences from the original virus. In the present invention, the terms "variant" and "subtype" are synonymous and are used interchangeably. The term "lineage" or "linage" describes a cluster of viral sequences derived from a common ancestor, which are associated with an epidemiological event, for instance, an introduction of the virus into a distinct geographic area with evidence of onward spread. Lineages are designed to capture the emerging edge of a pandemic and are at a fine-grain resolution suitable to genomic epidemiological surveillance and outbreak investigation. The SARS-CoV-2 lineage nomenclature is described for example, in Rambaut A. et al. A dynamic nomenclature proposal for SARS-CoV-2 lineages to assist genomic epidemiology. Nat Microbiol. 2020; 5(11): 1403-1407. Thus, by "at least one variant of severe acute respiratory syndrome coronavirus 2 (SARS-CoV-2)" is meant at least one variant, strain, isolate, lineage or linage, and/or clade of the SARS-CoV-2 virus. The different variants of SARS-CoV-2 can be found in databases such as Emma B. Hodcroft. 2021. "CoVariants: SARS-CoV-2 Mutations and Variations of Interest" (covariants.org/variants) or O'Toole A. et al., 2020 "A dynamic nomenclature proposal for SARS-CoV-2 lineages to assist genomic epidemiology", PANGO lineages (cov-lineages.org/).

In the context of multiple immunization regiments, an "homologous prime-boost combination" is referred herein as the administration of at least two doses of a vaccine, wherein the vaccine is the same in every dose. By "heterologous prime-boost combination" is referred herein as the administration of at least two doses of a vaccine, wherein the first dose comprises a vaccine that is different from the vaccine comprised in the second or subsequent doses. By "different" is meant herein that either the immunogen is different (different antigen) or that, even if the antigen is the same, it is carried by a different vector, or both (different vector and different antigen). For example, in the case of different vector, a heterologous prime-boost combination may be a first dose with a DNA-based vaccine, and a second dose with a MVA-based vaccine. By "Immunogen" is referred herein to a protein or a portion thereof that is capable of inducing an immune response in a mammal, such as a mammal infected or at risk of infection with a pathogen.

Several documents are cited throughout the text of this specification. Each of the documents cited herein (including all patents, patent applications, scientific publications, manufacturer's specifications, instructions, etc.), whether supra or infra, are hereby incorporated by reference in their entirety. To the extent the material incorporated by reference contradicts or is inconsistent with this specification, the specification will supersede any such material. Nothing herein is to be construed as an admission that the invention is not entitled to antedate such disclosure by virtue of prior invention.

### Recombinant MVA virus vaccine compositions

Vaccinia virus (VACV) is amongst the most extensively evaluated live vectors and has particular features in support of its use as a recombinant vaccine: It is highly stable, cheap to manufacture, easy to administer, and it can accommodate large amounts of foreign DNA. It has the advantage of inducing both antibody and cytotoxic T cell responses and allows presentation of antigens to the immune system in a more natural way, and it was successfully used as vector vaccine protecting against several infectious diseases in a broad variety of animal models. Additionally, vaccinia vectors are extremely valuable research tools to analyse structure-function relationships of recombinant proteins, determine targets of humoral and cell- mediated immune responses, and investigate the type of immune parameters needed to protect against a specific disease.

However, VACV is infectious for humans and its use as expression vector in the laboratory has been affected by safety concerns and regulations. Furthermore, possible future applications of recombinant VACV e.g. to generate recombinant proteins or recombinant viral particles for novel therapeutic or prophylactic approaches in humans, are hindered by the productive replication of the recombinant VACV vector. Most of the recombinant VACVs described in the literature are based on the Western Reserve (WR) strain of VV. On the other hand, it is known that this strain is highly neurovirulent and is thus poorly suited for use in humans and animals (Morita et al., Vaccine 5, 65-70 [1987]).

Concerns with the safety of standard strains of VACV have been addressed by the development of VACV vectors from highly attenuated virus strains which are characterized by their restricted replicative capacity in vitro and their avirulence in vivo. Strains of viruses specially cultured to avoid undesired side effects have been known for a long time. Thus, it has been possible, by long-term serial passages of the chorioallantoic VACV Ankara (CVA) strain on chicken embryo fibroblasts, to culture MVA (for review see Mayr, A., Hochstein-Mintzel, V. and Stickl, H. (1975) Infection 3, 6-14; Swiss Patent No. 568 392). The MVA virus was deposited in compliance with the requirements of the Budapest Treaty at CNCM (Institut Pasteur, Collectione Nationale de Cultures de Microorganisms, 25, rue de Docteur Roux, 75724 Paris Cedex 15) on Dec. 15, 1987 under Depositary No. 1-721.

The MVA virus has been analysed to determine alterations in the genome relative to the wild type CVA strain. Six major deletions (deletion I, II, III, IV, V, and VI) have been identified (Meyer, H., Sutter, G. and Mayr A. (1991) J. Gen. Virol. 72, 1031-1038). This MVA has only low virulence, that is to say it is followed by no side effects when used for vaccination. Hence it is particularly suitable for the initial vaccination of immunocompromised subjects. The excellent properties of the MVA strain have been demonstrated in a number of clinical trials (Mayr et al., Zbl. Bakt. Hyg. I, Abt. Org. B 167, 375-390 [1987], Stickl et al., Dtsch. med. Wschr. 99, 2386-2392 [1974]). Thus, MVA is a valuable tool as safe viral vector for expression of recombinant genes and can be used for such different purposes as the in vitro study of protein functions or the *in vivo* induction of antigen-specific cellular or humoral immune responses. A major advantage of MVA is to allow for high level gene expression despite being replication defective in human and most mammalian cells. MVA as a vaccine has an excellent safety track-record, can be handled under biosafety level 1 conditions and has proven to be immunogenic and protective when delivering heterologous antigens in animals, and first human candidate vaccines have proceeded into clinical trials.

Although unable to multiply in most mammalian cell lines, MVA retains its genome plasticity that allows the insertion of large amounts of foreign DNA (heterologous genes) (Sutter and Moss, 1992). Absence of pathogenicity for humans, inherent avirulence even in immunocompromised hosts, high-level expression of foreign antigens and adjuvant effect for immune responses make recombinant MVA (rMVA), expressing heterologous genes, an ideal vector for both prophylactic and therapeutic vaccination, as demonstrated by the wide use in prime-boost immunization strategies

Indeed, recombinant MVA (rMVA)-based vaccines comprising heterologous genes and therefore expressing heterologous proteins are able to elicit both humoral and cell-mediated adaptive immune responses (Ramirez et al., 2000) and have been proved to be protective in animal models of several infectious diseases (García-Arriaza et al., 2014, Journal of Virology (Chikungunya) Perez et al., 2018, Scientific Reports (Zika) Lázaro-Frías et al., 2018 (Ebola) Gómez et al., 2013; Marín et al., 2019, Journal of Virology (Hepatitis C) Garcia.-Arriaza et al., 2021 (SARS-CoV-2)).

Standard procedures to generate recombinant VACV (rVACV) have been described in detail (Earl et al., 1998) and rely on in vivo homologous recombination between acceptor VACV DNA and the transfected transfer plasmid, in which the foreign gene is flanked by VACV sequences. Additional approaches for the generation of recombinant MVA are, for example, disclosed in WO 04074493, WO 03023040 and WO 9702355.

Highly immunogenic viral proteins, such as the S protein of SARS-CoV-2 virus, are usually elusive and present several mechanisms to mislead the immune system and avoid the straightforward generation of neutralizing antibodies. Some of the mechanisms used to evade the immune system of the host imply conformational changes that may also be important for viral cycle. Among them, it is particularly relevant the transition between the prefusion state of the S protein, that is naturally present in the viral particles when they are free in the media, to the post-fusion state, that occurs after the binding of the S protein to the host cell receptor. The S protein is first produced as a precursor that trimerizes and is thought to be cleaved by a furin-like protease into two fragments: the receptor-binding fragment S1 and the fusion fragment S2. Binding through the receptor-binding domain (RBD) comprised in the S1 to a host cell and further proteolytic cleavage at a second site in S2 are believed to trigger dissociation of S1 and irreversible refolding of S2 into a postfusion conformation, a trimeric hairpin structure formed by heptad repeat 1 (HR1) and heptad repeat 2 (HR2). These large structural rearrangements bring together the viral and cellular membranes, ultimately leading to fusion of the two bilayers.

Although this mechanism is necessary for viral attachment to the cell response, antibodies against the post-fusion S protein have less neutralizing capability since the S protein can only be found in said state when the virus is bound to the cellular receptor. These antibodies would thus not target efficiently the protein when the viral particles are released from an infected cell. Therefore, vaccines able to elicit neutralizing antibodies that target the prefusion state of the S protein represent an urgent need.

An important question is thus whether rMVA-based vaccines would or not be capable of generating a strong immunogenic and protective immune response against the stabilized prefusion S protein from SARS-CoV-2 virus in a subject in need thereof. In this regard, the authors of the present invention have redesigned the S protein of SARS-CoV-2 virus by introducing modifications into the wildtype S amino acid sequence and they have found and demonstrated that a vaccine comprising an immunologically effective amount of a MVA vector expressing a prefusion stabilized SARS-CoV-2 S protein (termed MVA-CoV2-S(3P), MVA-CoV2-S(3P) (which will also be referred to as MVA-S(3P) throughout the text and figures) is capable of generating a strong immunogenic and protective immune response against the SARS-CoV-2 virus in a subject in need thereof, remarkably only after one dose of the vaccine. The prefusion-stabilized S protein disclosed herein comprises at least the substitutions R682G, R683S, R685S, A942P, K986P and V987P.

In particular, the results presented herein show that mice vaccinated with one dose of MVA-S(3P) generated higher neutralizing antibodies and higher anti-S and anti-RBD IgG antibodies than the parental MVA-CoV2-S vector (Fig. 5, 7, 13B and 13C), in which the S protein does not comprise the substitutions R682G, R683S, R685S, A942P, K986P and V987P and therefore the vector MVA-CoV2-S does not produce a prefusion-stabilized S protein. Interestingly, although one single dose of both vaccine candidates (MVA-CoV2-S and MVA-S(3P)) conferred full protection (100% survival) after challenge with SARS-CoV-2 virus, mice vaccinated with MVA-S(3P) did not present a reduction in body weight due to the infection, while mice vaccinated with MVA-CoV2-S suffered loss of body weight, indicating that the virus caused an infection in these animals (see Fig. 13D). Furthermore, vaccination with one dose of MVA-S(3P) was highly effective to prevent SARS-CoV-2 replication and virus yields at 4 days postchallenge (Fig. 14A and 14B). It is noted that the lack of body weight loss and symptoms in SARS-CoV-2 challenged animals is also relevant from a clinical and sociological point of view since the lack of symptoms indicates that the vaccinated subject is controlling viral replication at early times post infection and would thus be less contagious than subject presenting symptoms due to viral infection. These results lead to the conclusion that MVA-S(3P) expressing a prefusion-stabilized S protein not only elicits higher humoral immune response than its parental vector MVA-CoV2-S in a one dose regimen, but it is also able to fully protect the animals while avoiding signs of disease and body weight loss.

In line with the above, the study underlying the present invention has also found that the use of a homologous prime/boost vaccine combination comprising two doses of the MVA-S(3P) vector also elicited higher antibody titers 10 days post prime than two doses of the MVA-S parental vector (Fig. 5B). These superior levels of antibodies elicited by MVA-S(3P) were mainly due to an increase in IgG1, IgG2b, IgG2c and Ig3 subtypes (Fig. 6A) Further, both vectors elicited comparable levels of IgG at 10 days post boost (Fig. 5C and 6B). Moreover, the vector MVA-S(3P) is able to generate higher neutralizing antibody titers than parental MVA-S after one dose (10 days post prime). All these higher humoral immune responses elicited by MVA-S(3P) are in accordance with the findings described above that the vector MVA-S(3P) protects more efficiently and without the course of symptoms than MVA-S after a single dose of the vaccine prior a SARS-CoV-2 challenge. In line with this, the present invention thus provides a homologous prime/boost vaccine combination comprising:
a. a composition comprising an immunologically effective amount of the rMVA vector provided herein; and
b. a composition comprising an immunologically effective amount of the rMVA vector provided herein;
wherein one of the compositions is a priming composition and the other composition is a boosting composition. Such response is particularly strong when the composition comprises the MVA-S(3P) provided herein.

Furthermore, in view of the results obtained and presented herein, it is also plausible that a single dose (either as a prime or as a boost) of an immunologically effective amount of MVA-S(3P) combined with an immunologically effective amount of at least another immunogen also elicits protective immune responses against a lethal dose of SARS-CoV-2 virus. In line with this, the present invention also provides a heterologous prime/boost vaccine combination comprising:
c. a composition comprising an immunologically effective amount of the rMVA vector provided herein; and
d. a composition comprising an immunologically effective amount of at least one immunogen of at least one SARS-CoV-2 variant;
wherein one of the compositions is a priming composition and the other composition is a boosting composition, in any order. Such response may be particularly strong when the composition comprising the MVA-S(3P) provided herein is given as a boost.

Thus, generally, the invention provides, for the first time, enhanced and effective vaccines or vaccine combinations for use in generating a protective immune response against infections by at least one SARS-CoV-2 variant and vaccines or vaccine combinations which can be used for manufacturing of a vaccine against at least one SARS-CoV-2 variant.

Thus, a first aspect of the present invention provides a vaccine composition comprising an immunologically effective amount of a modified vaccinia virus Ankara (MVA) vector comprising at least one nucleic acid encoding a SARS-CoV-2 spike (S) protein, or an immunogenic fragment of said S protein comprising at least one epitope, of at least one SARS-CoV-2 variant, wherein said S protein or fragment comprise amino acid substitutions aimed at enhancing the stability of the prefusion state of the S protein or fragment therefrom, and wherein the MVA vector regulates the expression of the nucleic acid encoding said stabilized prefusion S protein or fragment thereof.

As detailed below, the redesigned S protein encoded by the genome of MVA contains amino acid substitutions that enhance stability of the prefusion S structure. These include mutations that inactivate the S1/S2 cleavage site, and mutations in HR1 that remove any strain in the turn region between HR1 and CH, i.e., to prevent the formation of a straight helix during fusion. In some embodiments, the resigned S protein can additionally contain a truncation of the HR2 motif. Truncation of the HR2 domain leads to disruption of the HR1/HR2 fusion core and also stabilizes the prefusion S structure.

In some embodiments, the S protein or an immunogenic fragment of said S protein comprising at least one epitope comprises at least one, two, preferably three, amino acid substitutions to inactivate the S1/S2 furin cleavage site. Inactivation of this cleavage site can be achieved by a number of sequence alterations (e.g., deletions or substitutions) within or around the site. One mutation that inactivates the cleavage site without otherwise impacting the structure of the protein is substitution of residues 682 RRAR 685. Please note that, here and throughout the whole document, the amino acid numbering used as reference is the one based on cryo-EM model PDB ID 6VSB or GenBank accession number MN908947.3. Thus, in a preferred embodiment, said inactivation of the S1/S2 cleavage site is achieved by introducing at least one, two, or three of the amino acid substitutions selected from the list consisting of R682G, R683S or R685S. More preferably, the S protein or the immunogenic fragment thereof comprises at least the three amino acid substitutions R682G, R683S and R685S.

In addition the inactivation of the cleavage site, the soluble SARS-CoV-2 immunogen polypeptides can additionally contain a double mutation in the HR1 region that remove strain in the turn region (between HR1 and CH motifs) during fusion by preventing the formation of a straight helix. Thus, in an embodiment, the S protein, or an immunogenic fragment thereof, encoded by the nucleic acid comprised in the MVA vector of the present invention further comprises other amino acid substitutions aimed at maintaining the quaternary structure of the S protein in a prefusion state or form. As referred herein, a "prefusion protein" or a "protein in a prefusion state or form" is meant a S protein or a fragment of said protein that are designed to be stabilized in a prefusion conformation, preventing structural rearrangements that usually take place when the protein interacts with its receptor or other protein. A strategy to stabilize the S protein in a prefusion conformation is eliminating the causes of metastability by amino acid substitutions in various regions of S, particularly HR1 and in HR2. In an embodiment, amino acid substitutions to proline are performed in the S protein to provide a prefusion S protein. In an embodiment, said amino acid substitutions aimed at maintaining the prefusion quaternary structure of the S protein are selected from the group including, but not limited to, T961D, G769E, L938F, F817P, A942P, S884C, A892P, A899P, A893C, T859K, Q957E, Q895P, T912P, K921P, G946P, or S975P, or any combination thereof.

In some embodiments, additional mutations of the wildtype soluble S sequence can be introduced to destabilize the postfusion S structure. In some embodiments, one or more proline and/or glycine substitution can be engineered in the region of HR1 that interacts with HR2 to form the fusion core. These substitutions function to disrupt the six-helix-bundle fusion core. In various embodiments, the mutations can include A942P, S943P, A944P, A942G, S943G and A944G. In further embodiments, the substitutions function to disrupt the six-helix-bundle fusion core. In various embodiments, the mutations can include S924P, T925P, A926P, S924G, T925G, and A926G. In some embodiments, one or more extra amino acid residues can be inserted into the region of HR1 that interacts with HR2 to form the fusion core. Similarly, these insertions also function to disrupt helical pattern of the fusion core. In various embodiments, the insertions can include insertion of G or GS between any residues in A942-A944. In some embodiments, the substitutions may be double amino acid substitutions such as K986GN987G, K986P/V987P, K986G/V987P or K986P/V987G.

In a preferred embodiment, the S protein or the immunogenic fragment thereof comprise at least one, two or three of the substitutions A942P, K986P and V987P. Most preferably, the S protein or the immunogenic fragment thereof encoded by the nucleic acid comprised in the MVA vector provided herein comprise at least the three substitutions A942P, K986P and V987P.

In an embodiment, the S protein or the immunogenic fragment thereof encoded by the nucleic acid comprised in the MVA vector provided herein comprises the above mentioned substitutions to avoid the furin cleavage in combination with the above mentioned substitutions to maintain the S protein in a prefusion state. Thus, in a most preferred embodiment, the vaccine composition according to the first aspect or any of its embodiments comprises an immunologically effective amount of a MVA vector comprising at least one nucleic acid encoding the S protein, or a fragment of said S protein comprising at least one epitope, of at least one SARS-CoV-2 variant, wherein said S protein or fragment thereof, comprises at least the substitutions R682G, R683S, R685S, A942P, K986P and V987P, and wherein the MVA vector regulates the expression of the nucleic acid encoding the S protein, or the fragment thereof.

In a preferred embodiment, the S protein encoded by the nucleic acid comprised in the MVA vector comprises, consists or consists essentially of the full-length S protein of at least a SARS-CoV-2 variant. The full-length S protein comprises or consists of amino acids 1 to 1273. In an embodiment, the S protein of at least a SARS-CoV-2 virus encoded by the nucleic acid comprised in the MVA-S(3P) vector according to the first aspect of the present invention comprises, consists, or consists essentially of SEQ ID NO: 1 or a sequence with at least 50%, 60%, 70%, 80%, 85%, 90%, 91%, 92%, 93%, 94%, 95%, 96%, 97%, 98%, 99% or 100% sequence identity over the full length with SEQ ID NO: 1. In the context of the present invention, the nucleic acid of the vaccine composition described herein may also optionally encode antigenic domains or antigenic protein fragments rather than the entire antigenic S protein. These fragments can be of any length sufficient to be antigenic or immunogenic as long as they comprise at least the amino acid substitutions R682G, R683S, R685S, A942P, K986P and V987P. Fragments can be at least 300 amino acids long, but can be longer, such as, e.g., at least 350, 400, 500, 600, 700, 800, 900, 1000, 1200 amino acids long, or any length in between. In some embodiments, at least one nucleic acid fragment encoding an antigenic S protein fragment or immunogenic S polypeptide thereof is inserted into the genome of the recombinant viral vector of the invention. In another embodiment, about 2-6 different nucleic acids encoding different antigenic proteins are inserted into the genome of the recombinant viral vector. In some embodiments, multiple immunogenic fragments or subunits of various proteins can be inserted. For example, several different epitopes from different sites of a single protein or from different proteins of the same SARS-CoV-2 strain, or from a S protein orthologue from different strains can be expressed from the vectors.

In some embodiments, the S protein consists of a truncated protein lacking at least 5, 10, 15, 20, 15, 30, 35, 40, 45, 50, 60, 70, 80, 90, 100, 200, 300, 400, or more than 400 amino acid of the full-length S protein. In some embodiments, the S protein consists of a truncated protein where the N-terminal domain has been removed. In some embodiments, the S protein consists of a truncated protein where the C-terminal domain has been removed. In some embodiments, the S protein consists of a truncated protein consisting of only the S1 and S2 subunits. In some embodiments, the S protein consists of a truncated protein where the cytosolic domain has been removed. In some embodiments, the S protein consists of a truncated protein where the transmembrane domain has been removed. In some embodiments, the S protein consists of a soluble S protein wherein the transmembrane and the cytosolic domains has been removed.

Thus, a "fragment" of the S protein of at least a SARS-CoV-2 virus according to the present invention is a partial amino acid sequence of the SARS-CoV-2 S protein or a functional equivalent of such a fragment. A fragment is shorter than the full-length complete SARS-CoV-2 S protein and preferably comprises or consists of the amino acids 682-987 of the full-length S protein. A fragment of the SARS-CoV-2 S protein also includes peptides having at least 15, 20 or 25 contiguous amino acid residues having at least about 70%, at least about 80%, at least about 90%, preferably at least about 95%, more preferably at least 98% sequence identity with at least about 15, 20 or 25 contiguous amino acid residues of SEQ ID NO: 2 having about the same length as said peptides.

A fragment that "corresponds substantially to" a fragment of the SARS-CoV-2 S protein is a fragment that has substantially the same amino acid sequence and has substantially the same functionality as the specified fragment of the SARS-CoV-2 S protein. A fragment that has "substantially the same amino acid sequence" as a fragment of the SARS-CoV-2 S protein typically has more than 90% amino acid identity with this fragment. Included in this definition are conservative amino acid substitutions.

In another preferred embodiment of the first aspect of the invention, the antigenic protein is the structural protein S of SARS-CoV-2 resulting in vaccine compositions comprising the MVA-S(3P) vector, preferably the antigenic protein is the protein of SEQ ID NO 2, more preferably the antigenic protein is the protein of SEQ ID NO 2 encoded by the nucleic acid consisting of SEQ ID NO 1, or a sequence with at least 50%, 60%, 70%, 80%, 85%, 90%, 91%, 92%, 93%, 94%, 95%, 96%, 97%, 98%, 99% or 100% sequence identity over the full length with SEQ ID NO: 1.

In a preferred embodiment, the nucleic acid encoding the antigenic protein is incorporated or inserted into a non-essential region of the genome of the MVA. In an embodiment of the present invention, the nucleic acid encoding the antigenic protein is incorporated into the MVA by substituting a non-essential region of the genome of the MVA. In a preferred embodiment of the present invention, the nucleic acid encoding the S protein is incorporated or inserted into the MVA thymidine kinase (TK) locus or hemagglutinin (HA) locus. Integration of the nucleic acid encoding the antigenic protein is performed preferentially by homologous recombination of the flanking regions of a so-called transfer vector, which initially comprises the nucleic acid encoding the antigenic protein according to the invention prior to its integration into the parental MVA genome. In a preferred embodiment, the transfer vector is the pCyA vector. The pCyA vector has been previously described in WO 2014/162031 A1.

In a preferred embodiment, the vaccine composition comprises an immunologically effective amount of a MVA vector comprising at least one nucleic acid encoding the S protein, or a fragment of said S protein comprising at least one epitope, of at least one SARS-CoV-2 variant, wherein said S protein or fragment thereof, comprises at least the substitutions R682G, R683S, R685S, A942P, K986P and V987P, wherein the S protein or the fragment thereof are inserted into the TK locus of the MVA genome, and wherein the MVA vector regulates the expression of the nucleic acid encoding the S protein, or the fragment thereof.

Upon homologous recombination by means of an infection with parental virus followed by a transfection with the plasmid transfer vector, selection and purification of recombinant viruses encoding the antigenic protein is performed. In an embodiment, the intermediate MVA recombinant viruses also expresses marker genes that facilitate the tracking and selection of the desired final recombinant viruses. For instance, the intermediate recombinant MVA viruses might express β-galactosidase gene, a fluorescent protein such as the green fluorescent protein (GFP), or an antibiotic resistance gene. Methods for generating recombinant MVAs by homologous recombination by means of intermediate MVAs encoding for selectable genes are described in WO 2014/162031 A1 and WO 2016/086980 A1.

On the other hand, early expression of antigens by VACV vectors is crucial for efficient antigen-specific CD8 and/or CD4 T cell responses. The specific properties of the early elements poxviral promoters might thus be crucial for induction of an antigen-specific T cell response.

Therefore, in a further embodiment, the nucleic acid encoding for the S protein, or an immunogenic fragment thereof, comprising at least the substitutions R682G, R683S, R685S, A942P, K986P and V987P and of at least one SARS-CoV-2 variant is operably linked and under the control of a VACV-specific promoter, an orthopox virus-specific promoter, a poxvirus-specific promoter or a synthetic promoter. A number of promoters may be used for the present invention. For the expression of the nucleic acid encoding an antigenic protein according to the invention several promoters, such the 30K and 40K promoters (US 5,747,324, A Strong Synthetic Early-Late Promoter (Sutter, et al., Vaccine (1994), 12, 1032-1040)), the P7.5 promoter (Endo et al., J. Gen. Virol. (1991) 72, 699-703), a promoter derived from the cow pox virus type A (inclusion ATI gene) (Lee et al., J. Gen. Virol. 1998), 79, 613), and the new synthetic late-early optimized (LEO) promoter (Di Pilato et al. J Gen Virol. 2013 Dec;94(Pt 12):2771-2776) or LEO160 promoter (Di Pilato et al. J Gen Virol. 2015 Aug;96(8):2360-2371) can be used. In a preferred embodiment, the promoter is the synthetic early-late (sE/L) promoter.

In another preferred embodiment, the nucleic acid encoding for the prefusion-stabilized S protein, or a fragment thereof, comprising at least the substitutions R682G, R683S, R685S, A942P, K986P and V987P of at least one SARS-CoV-2 variant is operably linked and under the control of a synthetic promoter. Synthetic promoters are DNA sequences that do not exist in nature and which are designed to regulate the activity of genes to which they are operably linked, controlling a gene's ability to produce its encoded protein. In a preferred embodiment, the synthetic promoter drives the expression of the antigenic protein early during infection. For instance, in an embodiment, the expression of the antigenic protein can be detected as early as 3-hour post-infection. In another embodiment, the synthetic promoter may also drive the expression of the antigenic protein late during infection. In a preferred embodiment, the expression of the antigenic protein is operably linked and under the control of a synthetic promoter that drives the expression of the antigenic protein both, early and late, during viral infection.

The synthetic promoter may be comprised by one or more elements driving early expression of said antigen. The synthetic promoter may be comprised by one or more elements driving late expression of said antigen. Different types of synthetic early/late promoters are further described in WO 2010/102822 A1. In another embodiment, the synthetic promoter is formed by both early and late elements, referred as sE/L promoter (Chakrabarti, S.; Sisler, J.R.; Moss, B. Compact, synthetic, vaccinia virus early/late promoter for protein expression. Biotechniques 1997, 23, 1094-1097) or the LEO160 promoter (Di Pilato, M.; Sanchez-Sampedro, L.; Mejías-Pérez, E.; Sorzano, C.O.S.; Esteban, M. Modification of promoter spacer length in vaccinia virus as a strategy to control the antigen expression. J. Gen. Virol. 2015, 96, 2360-2371. Perez et al., 2019, Vaccines 2019, 7, 208). In another embodiment, the early and late elements are overlapping.

In a preferred embodiment, the vaccine composition comprises an immunologically effective amount of a MVA vector comprising at least one nucleic acid encoding the S protein, or a fragment of said S protein comprising at least one epitope, of at least one SARS-CoV-2 variant, wherein said S protein or fragment thereof, comprises at least the substitutions R682G, R683S, R685S, A942P, K986P and V987P; wherein the nucleic acid encoding for the S protein or the fragment is operably linked to an early/late promoter from VACV, preferably the synthetic early/late promoter; and wherein the MVA vector regulates the expression of the nucleic acid encoding the S protein, or the fragment thereof.

In a preferred embodiment, the MVA vector provided in the first aspect or the present invention or in any of its embodiments comprises at least one nucleic acid encoding the S protein, or a fragment of said S protein comprising at least one epitope, of at least one SARS-CoV-2 variant, wherein said S protein or fragment comprises at least the substitutions R682G, R683S, R685S, A942P, K986P and V987P; wherein the nucleic acid encoding for the S protein or the fragment is operably linked to an early/late promoter from VACV, preferably the synthetic early/late promoter; wherein said nucleic acid is inserted into the TK locus of the MVA genome; and wherein the MVA vector regulates the expression of the nucleic acid encoding the S protein, or the fragment thereof.

On the other hand, the nucleic acid inserted in MVA genome according to the invention can further encode for one or more antigenic proteins that can be any protein(s) from any SARS-CoV-2 virus variant. In a preferred embodiment, the nucleic acid comprised in the MVA vector further encodes for one or more antigenic proteins selected from the group consisting of structural proteins E (envelope), receptor-binding domain (RBD), N (nucleocapsid) or M (membrane), or any combination thereof, from at least one SARS-CoV-2 variant. In an embodiment, said further antigenic proteins are inserted in a different locus of the MVA genome, preferably in the HA locus. In another embodiment, said further antigenic proteins are placed under the control of a synthetic VACV promoter, preferably the synthetic early/late promoter.

In an embodiment, the MVA vector comprising at least one nucleic acid encoding the S protein, or a fragment of said S protein comprising at least one epitope, of at least one SARS-CoV-2 variant according to the first aspect or any of its embodiments is derived from a variant of concern (VOC) as defined by the Centers for Disease Control and Prevention (CDC) "SARS-CoV-2 Variant Classifications and Definitions". The SARS-CoV-2 is observed to mutate, with certain combinations of specific point mutations proving to be more concerning than others. These mutations are the reason of the increased transmissibility, increased virulence, and possible emergence of escape mutations in new variants. The term "variant of concern" (VOC) is a designation used in newly emerged variants of SARS-CoV-2 with mutations that provide an increased transmissibility and/or morbidity and/or mortality and/or decreased susceptibility to antiviral or therapeutic drugs and/or have the ability to evade immunity and/or ability to infect vaccinated individuals, among others.

In a preferred embodiment, the S protein, or a fragment of said S protein comprising at least one epitope, of at least one SARS-CoV-2 variant is derived from the Wuhan-Hu-1 seafood market pneumonia virus isolate (GenBank accession number: MN908947). In another embodiment, the S protein, or a fragment of said S protein comprising at least one epitope, of at least one SARS-CoV-2 variant is derived from the United Kingdom SARS-CoV-2 variant VOC 202012/01 (Lineage B.1.1.7). According to WHO, on 14 December 2020, authorities of the United Kingdom reported to WHO a variant referred to by the United Kingdom as SARS-CoV-2 VOC 202012/01 (Variant of Concern, year 2020, month 12, variant 01) also known as Lineage B.1.1.7 or 501Y.V1. This variant is described in the scientific literature, see, e.g., in Wise, J. Covid-19: New coronavirus variant is identified in UK. BMJ 2020, 371, m4857. This variant contains 23 nucleotide substitutions and is not phylogenetically related to the SARS-CoV-2 virus circulating in the United Kingdom at the time the variant was detected. Among the variant's several mutations there is one in the RBD of the S protein that changes the asparagine at position 501 to tyrosine (N501Y). Another of the mutations in the VOC 202012/01 variant, the deletion at position 69/70 was found to affect the performance of some diagnostic PCR assays with an S gene target. As of 30 December, VOC-202012/01 variant has been reported in 31 other countries/territories/areas in five of the six WHO regions.

In another embodiment, the S protein, or a fragment of said S protein comprising at least one epitope, of at least one SARS-CoV-2 variant according to the first aspect or any of its embodiments is derived from the South African SARS-CoV-2 variant (Lineage B.1.351). On 18 December, national authorities in South Africa announced the detection of a new variant of SARS-CoV-2 that is rapidly spreading in three provinces of South Africa. South Africa has named this variant as Lineage B.1.351, also known as 501Y.V2. This variant is described in the scientific literature, see, e.g., Tegally et al. Emergence and rapid spread of a new severe acute respiratory syndrome-related coronavirus 2 (SARS-CoV-2) lineage with multiple S mutations in South Africa. medRxiv 2020. SARS-CoV-2 South African variant is characterised by three mutations K417N, E484K and N501Y in the RBD. While SARS-CoV-2 VOC 202012/01 from the UK also has the N501Y mutation, phylogenetic analysis has shown that the virus from South Africa are different virus variants.

In another embodiment, the S protein, or a fragment of said S protein comprising at least one epitope, of at least one SARS-CoV-2 variant according to the first aspect or any of its embodiments is derived from the Brazilian SARS-CoV-2 variant VOC-202101/02 (Linage B.1.1.28). Brazilian variant is also known as Lineage P.1, also known as 20J/501Y.V3, Variant of Concern 202101/02 (VOC-202101/02). This variant is described in the scientific literature, see, e.g., Faria, et al. Genomic Characterisation of an Emergent SARS-CoV-2 Lineage in Manaus: Preliminary Findings. This variant of SARS-CoV-2 has 17 unique amino acid changes, ten of which are in its S protein, including these three designated to be of particular concern: N501Y, E484K and K417T. This variant of SARS-CoV-2 was first detected by the National Institute of Infectious Diseases (NIID), Japan, on 6 January 2021 in four people who had arrived in Tokyo having visited Amazonas, Brazil four days earlier. It was subsequently declared to be in circulation in Brazil and spreading around the world.

Recently, a California variant has been also known as VOC CAL.20C. Thus, in another embodiment, the S protein, or a fragment of said S protein comprising at least one epitope, of at least one SARS-CoV-2 variant according to the first aspect or any of its embodiments is derived from the Californian SARS-CoV-2 (Linage B.1.427 or B.1.429). This variant is characterized by the mutations S131, W152C in the N-terminal domain (NTD) of the S protein and by the L452R mutation in the RBD of the S protein. This variant was originally detected in California (Linage B.1.427 or B.1.429).

Many other variants have been described, such as the B.1.207 variant in Nigeria, which has a mutation in the S protein (P681H) that is also found in the VOC 202012/01 variant, the B.1.617 variant in India (Linage B.1.617, Indian variant), which has the mutations P681R, E484Q and L425R in the S protein, or the Danish variant, referred to as the "Cluster 5" variant by Danish authorities, and which has a combination of mutations not previously observed. The rising variants worldwide can be easily found by the skilled person, e.g., in the website databases such as disclosed by Emma B. Hodcroft. 2021. "CoVariants: SARS-CoV-2 Mutations and Variations of Interest." (covariants.org/variants) or by O'Toole A. et al., 2020 "PANGO lineages" (cov-lineages.org/). It is thus to be understood that the present invention covers MVAs encoding for S proteins or fragments of said proteins that are derived from any strain or clade or variant or lineage or isolate of SARS-CoV-2.

In a preferred embodiment, the S protein, or a fragment of said S protein comprising at least one epitope, of at least one SARS-CoV-2 variant according to the first aspect or any of its embodiments is derived from a SARS-CoV-2 variant selected from the group including, but not limited to, Wuhan-Hu-1 seafood market pneumonia virus isolate (GenBank accession number: MN908947), Linage B.1.1.28 (Brazilian variant), Linage B.1.351 (South African variant), Linage B.1.427 or Linage B.1.429 (California variant), Linage B.1.617 (Indian variant), or Linage B.1.1.7 (United Kingdom variant), or any combination thereof. In a preferred embodiment, the S protein, or a fragment of said S protein comprising at least one epitope, of at least one SARS-CoV-2 variant according to the first aspect or any of its embodiments is derived from a SARS-CoV-2 variant selected from the group consisting of Wuhan-Hu-1 seafood market pneumonia virus isolate (GenBank accession number: MN908947), Linage B.1.1.28 (Brazilian variant), Linage B.1.351 (South African variant), Linage B.1.427 or Linage B.1.429 (California variant), Linage B.1.617 (Indian variant), or Linage B.1.1.7 (United Kingdom variant), or any combination thereof.

Rational design of S-based vaccines also provides the generation of chimeric S proteins comprising the most dominant antigenic epitopes from more than one SARS-CoV-2 variants. Furthermore, it is also possible to generate a synthetic, i.e., not "naturally occurring", S protein that comprises a combination of the sequences of two or more SARS-CoV-2 variants. Thus, in a possible embodiment, the S protein, or a fragment of said S protein comprising at least one epitope, comprises or consists of the sequences of two or more SARS-CoV-2 variants. Preferably, the S protein, or a fragment of said S protein comprising at least one epitope, comprises or consists of a consensus sequence derived from two or more different SARS-CoV-2 variants.

It is noted that the recombinant MVA viruses described herein are highly replication restricted and, thus, highly attenuated, and thus they are ideal candidates for the treatment of a wide range of mammals including humans and even immune-compromised humans. Hence, provided herein are pharmaceutical compositions and vaccines, as described above in the first aspect of the invention, suitable for inducing an immune response in a living animal body, including a human against the SARS-CoV-2 virus.

The vaccines of the first aspect preferably comprise the MVA vector described herein formulated in solution in a concentration range of from 1 × 10⁷ to 1 × 10⁸ PFUs per dosis. This is illustrated in Figures 5-14 which show that immunogenicity was induced in vaccinated mice with a dose of 1 × 10⁷ PFUs per dose (see immunization schedule in Figures 5 and 13). Further, as shown in Figure 13, mice were vaccinated with 1×10⁷ PFUs of MVA-S(3P) virus per dose, which indicates that this dose is sufficient to induce protective immunity that leads to 100% survival upon a lethal challenge with SARS-CoV-2. As discussed above, it is important to highlight that mice vaccinated with MVA-S(3P) did not show any weight loss nor disease symptoms, indicating that the vaccine is not only able to protect mice from a lethal challenge, but it is able to stop and/or reduce viral infection and the symptoms associated to it at early times upon challenge, as it is described in Figure 14A and 14B.

The vaccine compositions provided herein in the first aspect may generally include one or more pharmaceutically acceptable and/or approved carriers, additives, antibiotics, preservatives, adjuvants, diluents and/or stabilizers. Such auxiliary substances can be water, saline, glycerol, ethanol, wetting or emulsifying agents, pH buffering substances, or the like. Suitable carriers are typically large, slowly metabolized molecules such as proteins, polysaccharides, polylactic acids, polyglycolic acids, polymeric amino acids, amino acid copolymers, lipid aggregates, or the like.

For the preparation of vaccines, the recombinant MVA viruses provided herein can be converted into a physiologically acceptable form. This can be done based on experience in the preparation of poxvirus vaccines used for vaccination against smallpox as described by H. Stickl et al., Dtsch. med. Wschr. 99:2386-2392 (1974).

For example, purified viruses can be stored at -80°C with a titer of 5×10⁸ a PFU/ml formulated in about 10 mM Tris, 140 mM NaCl pH 7.4. For the preparation of vaccine shots, e.g., 10²-10⁸ or 10²-10⁹ particles of the virus can be lyophilized in 100 ml of phosphate-buffered saline (PBS) in the presence of 2% peptone and 1% human albumin in an ampoule, preferably a glass ampoule. Alternatively, the vaccine shots can be produced by stepwise freeze-drying of the virus in a formulation. This formulation can contain additional additives such as mannitol, dextran, sugar, glycine, lactose or polyvinylpyrrolidone or other aids such as antioxidants or inert gas, stabilizers or recombinant proteins (e.g., human serum albumin) suitable for in vivo administration. The glass ampoule is then sealed and can be stored between 45°C and room temperature for several months. However, as long as no need exists, the ampoule is stored preferably at temperatures below -20°C.

For vaccination or therapy, the lyophilizate can be dissolved in an aqueous solution, preferably physiological saline or Tris buffer, and administered either systemically or locally, i.e., parenteral, subcutaneous, intravenous, intramuscular, intranasal, or any other path of administration known to the skilled practitioner. The mode of administration, the dose and the number of administrations can be optimized by those skilled in the art in a known manner. However, most commonly a patient is vaccinated with a second shot about one month to six weeks after the first vaccination shot, although booster doses can also be administered years after the first dose.

In addition, for working the present invention any avirulent MVA vector suitable for human vaccination could be used. "Derivatives" or "variants" of MVA are also suitable for working the present invention. In preferred embodiments, the MVA vector is further modified by deletion of one or more vaccinia immunomodulatory genes; preferably one, two, or three of the genes C6L, K7R, and A46R. Most preferably, all three genes are deleted. Deletion of these genes has been shown to enhance the innate immune response triggered by the viral vector, and also adaptive antigen-specific immune responses. Thus, in an alternative embodiment of the first aspect, the present invention provides a vaccine composition comprising an immunologically effective amount of a MVA vector comprising at least one nucleic acid encoding the S protein, or a fragment of said S protein comprising at least one epitope, of at least one SARS-CoV-2 variant, wherein said S protein or fragment thereof, comprises at least the substitutions R682G, R683S, R685S, A942P, K986P and V987P, wherein the MVA vector regulates the expression of the nucleic acid encoding the S protein, or the fragment thereof, and wherein the MVA vector is further modified by deletion of one or more vaccinia immunomodulatory genes; preferably one, two, or three of the genes C6L, K7R, and A46R.

In the context of the present invention, "derivatives" or "variants" of MVA refer to viruses exhibiting essentially the same replication characteristics as MVA but exhibiting differences in one or more parts of their genomes. These "derivatives" or "variants" of MVA must fail to reproductively replicate in vivo in humans and mice, even in severely immune suppressed mice. Tests and assays for the properties of MVA variants are described in WO 02/42480 (US 2003/0206926) and WO 03/048184 (US 2006/0159699).

The term "fails to reproductively replicate" refers to a virus that has a virus amplification ratio at 4 days after infection of less than 1. Assays described in WO 02/42480 or in U.S. Patent No. 6,761 ,893 are applicable for the determination of the virus amplification ratio.

The amplification or replication of a virus is normally expressed as the ratio of virus produced from an infected cell (output) to the amount originally used to infect the cell in the first place (input) referred to as the "amplification ratio". An amplification ratio of "1" defines an amplification status where the amount of virus produced from the infected cells is the same as the amount initially used to infect the cells, meaning that the infected cells are permissive for virus infection and reproduction. In contrast, an amplification ratio of less than 1, i.e., a decrease in output compared to the input level, indicates a lack of reproductive replication and therefore attenuation of the virus.

The advantages of MVA-based vaccine include their safety profile as well as availability for large scale vaccine production. In a preferred embodiment, the recombinant MVA vector of any of the embodiments used for generating the recombinant virus is a MVA virus or a derivative having the capability of reproductive replication in vitro in chicken embryo fibroblasts (CEF) cells and in established chick cells DF-1, but no capability of reproductive replication in human cervix adenocarcinoma cell line HeLa. Also preferably, the recombinant MVA vector of any of the embodiments used for generating the recombinant virus is a MVA vector may be further modified by deletion of one or more vaccinia immunomodulatory genes; preferably one, two, or three of the genes C6L, K7R, and A46R. Most preferably, all three genes are deleted.

MVA vectors useful for the present invention can be prepared using methods known in the art, such as those described in WO 02/042480 and WO 02/24224, both of which are incorporated by reference herein.

In another aspect, a MVA viral strain suitable for generating the recombinant viruses of the present invention may be strain MVA-572, MVA-575 or any similarly attenuated MVA strain. Also suitable may be a mutant MVA, such as the deleted chorioallantoic vaccinia virus Ankara (dCVA). A dCVA comprises del I, del II, del III, del IV, del V, and del VI deletion sites of the MVA genome. The sites are particularly useful for the insertion of multiple heterologous sequences. The dCVA can reproductively replicate (with an amplification ratio of greater than 10) in a human cell line (such as human 293, 143B, and MRC-5 cell lines), which then enable the optimization by further mutation useful for a virus-based vaccination strategy (see WO 201 1/092029).

### Combination Vaccines Using Homoloqous/Heterologous Prime-Boost Regimens

The Combination Vaccines and methods described herein may be used as part of a homologous prime-boost regimen. In the homologous prime-boost, a first priming vaccination is given followed by one or more subsequent boosting vaccinations. The boosting vaccinations are configured to boost the immune response generated in the first vaccination by administration of the same recombinant MVA that was used in the first vaccination.

In one exemplary embodiment a homologous prime-boost regimen may be employed wherein a MVA viral vector as defined herein in the first aspect of the invention or in any of its embodiments is administered in a first dosage. One or more subsequent administrations of a MVA viral vector as defined herein in the first aspect or any of its embodiments can be given to boost the immune response provided in the first administration. Preferably, the one or more antigenic determinants are the same or similar to those of the first administration. In a preferred embodiment, the MVA vector provided herein is administered as a boost (i.e., as a second, third, fourth, or successive vaccinations).

The MVA recombinant viral vectors according to the present invention may also be used in prime-boost regimens in combination with other immunogens (heterologous prime/boost combination=, such as in combination with a DNA or RNA vector or another viral vector, such as an adenoviruses. In this case, one or more subsequent boosting vaccinations may be done with the MVA vector provided herein and not used in the prime vaccination, e.g., if another immunogenic vector is given in a prime, then subsequent boosting vaccinations may be performed with the MVA provided herein, and vice versa.

Thus, a **second aspect** of the invention refers to a vaccine combination (from hereinafter referred to as homologous vaccine combination) comprising:
a. a composition comprising an immunologically effective amount of a MVA vector as defined in the first aspect or any of its embodiments; and
b. a composition comprising an immunologically effective amount of a MVA vector as defined in the first aspect or any of its embodiments;
wherein one of the compositions is a priming composition and the other composition is a boosting composition, preferably wherein the boosting composition comprises or consists of two or more doses of the vector of the boosting composition.

Furthermore, in an alternative second aspect of the invention refers to a vaccine combination (from hereinafter referred to as heterologous vaccine combination) comprising:
a. a composition comprising an immunologically effective amount of a MVA vector as defined in the first aspect or any of its embodiments; and
b. a composition comprising an immunologically effective amount of at least one immunogen of at least one SARS-CoV-2 variant, preferably selected from the group consisting of viral vectors, recombinant proteins or polypeptides, nucleic acids, virus-like particles, nanoparticles, or any combination thereof,
wherein one of the compositions is a priming composition and the other composition is a boosting composition, in any order. Preferably, the composition comprising the MVA vector as defined in the first aspect or any of its embodiments is administered as a boost, after a prime with a composition comprising an immunologically effective amount of an immunogen of at least one SARS-CoV-2 variant. Preferably, the boost composition comprises or consists of two or more doses of the vector or immunogen of the boosting composition.

It is noted that the recombinant heterologous or homologous vaccine combinations may be either monovalent, i.e., comprising only one heterologous sequence encoding an antigenic determinant of SARS-CoV-2, or multivalent, i.e., comprising at least two heterologous sequences encoding antigenic determinants of SARS-CoV-2, such as those selected from the list consisting of structural proteins S, RBD, E, N or M of SARS-CoV-2, preferably such antigenic proteins are those selected from any of SEQ ID NO 2, SEQ ID NO 4, SEQ ID NO 6, SEQ ID NO 8 and SEQ ID NO 10, respectively encoded by nucleic acid sequences SEQ ID NO 1, SEQ ID NO 3, SEQ ID NO 5, SEQ ID NO 7 and SEQ ID NO 9.

In one preferred embodiment of the second aspect of the invention, the immunologically effective amount of an immunogen of at least one SARS-CoV-2 variant comprises an antigenic protein or nucleic acid encoding for an antigenic protein, wherein the antigenic protein is selected from any of the group of structural proteins S, RBD, E, N or M of SARS-CoV-2, or any combination or fragments thereof. In an embodiment, the at least one immunogen of at least one SARS-CoV-2 variant is a nucleotide sequence of a DNA or RNA vector that encodes for an antigenic protein of at least one SARS-CoV-2 variant. In an embodiment, said nucleotide sequence encoding for an antigenic protein is preferably the structural protein S of SARS-CoV-2, or a fragment thereof such as the RBD.

As regards the heterologous vaccine compositions it is important to note that upon injection, preferably intramuscularly, of the DNA/RNA vaccine, cells, preferably muscle cells, may be efficiently transfected and produce a relatively large amount of antigen that may be secreted or otherwise released. Once the plasmid or the RNA reaches the cell, the antigen of interest is produced. Next, the transfected cell displays on its surface the foreign antigen in both histocompatibility complex (MHC) classes I and class II molecules. The antigen-presenting cell primed with the antigen travels to the lymph nodes and presents the antigen peptide and costimulatory molecules, initiating the immune response.

DNA/RNA-based vectors suitable for used as vaccines, as disclosed herein for the heterologous regimes or heterologous vaccine combinations, are known in the art. For instance, Hobernik and Bros Int J Mol Sci. 2018 Nov 15;19(11):3605 summarize the knowledge on the course of action of DNA vaccines and the methods for DNA vaccine immunogenicity optimization. Lee et al. Acta Biomater. 2018 Oct 15;80:31-47 relates to engineering DNA vaccines against infectious diseases.

The DNA/RNA-based vaccine vector encode for one or more antigens against which the immune response is to be elicited. Further, the DNA/RNA vector can also encode other co-stimulatory molecules, such as interleukins, cytokines, etc.

If the DNA/RNA-based vaccine is to be administrated to a mammal, the promoter operably linked to the antigen of interest would need to be recognized by mammal cellular transcription machinery. Promoters for expressing proteins in mammalian cells are also known in the art. For instance, some viral promoters might be used, such as cytomegalovirus promoter or T7 phage promoter.

Any DNA, linear or plasmid, able to express the antigen in a mammalian cell may be used to construct a DNA/RNA vaccine. Mammalian expression DNA and RNA vectors are widely known in the art.

In a preferred embodiment of the heterologous vaccine combination, the other immunogen that is combined with the MVA vector provided herein is a mammalian expression vector (a DNA plasmid vector) and produces high levels of the antigen. For instance, suitable mammalian expression plasmid vectors that can be used in the present inventions are, but not limited to, pCDNA, pCAGGS, etc. The mammalian expression vector can be grown in bacterial cells for high-yield production.

In a preferred embodiment of the heterologous vaccine combination, the other immunogen that is combined with the MVA vector provided herein is a RNA molecule. In a preferred embodiment of the heterologous vaccine combination, the other immunogen that is combined with the MVA vector provided herein is an adenovirus. In a preferred embodiment of the heterologous vaccine combination, the other immunogen that is combined with the MVA vector provided herein is a recombinant protein or polypeptide.

Methods for generating, producing and isolating DNA/RNA vaccine vectors are known in the art. In a preferred embodiment of the present invention, the DNA/RNA-based vaccine has been produced by endo-free means, for instance, by using EndoFree Plasmid Kits by Qiagen.

### Vaccines and Kits Comprising Recombinant MVA Viruses

Also provided herein are vaccines and kits comprising any one or more of the recombinant MVAs described herein. The kit can comprise one or multiple containers or vials of the recombinant MVA, together with instructions for the administration of the recombinant MVA to a subject at risk of SARS-CoV-2 infection. In certain embodiments, the subject is a human. In certain embodiments, the instructions indicate that the recombinant MVA is administered to the subject in a single dose, or in multiple (i.e., 2, 3, 4, etc.) doses. In certain embodiments, the instructions indicate that the recombinant MVA virus is administered in a single dose to naive or non-naive subjects. Thus, in a preferred embodiment, the vaccines and kits comprising recombinant MVA vectors or vaccine combinations as described in the first or second aspects or any of their embodiments comprise a single dose to be administrated to a subject in need thereof. In certain embodiments, the instructions indicate that the recombinant MVA virus is administered in a first (priming) and second (boosting) administration to naive or non-naive subjects. In certain embodiments, the instructions indicate that the recombinant MVA virus is administered as a boost to naive or non-naive subjects. Preferably, a kit comprises at least two vials or container for prime/boost immunization comprising the recombinant MVAs as described herein for a first inoculation ("priming inoculation") in a first vial/container and for an at least second and/or third and/or further inoculation ("boosting inoculation") in a second and/or further vial/container. More preferably, the vaccines and kits described herein comprise a first (priming) and second (boosting) dose of an immunologically effective amount of the recombinant MVAs as described herein, for use in homologous or heterologous prime boost inoculations.

Thus, a **third aspect** of the invention refers to kits comprising one, two, or more doses of any of the vaccine compositions as defined in the first aspect of the invention or any of its embodiments, or of any of the homologous or heterologous vaccine combinations of the second aspect of the invention or any of its embodiments. Preferably, the kit comprises an immunologically effective amount of a MVA-based vaccine composition, preferably MVA-S(3P) vector according to the first aspect of the invention in a first vial or container for a first administration (priming) and in a second vial or container for a second administration (boosting).

A preferred embodiment of the third aspect of the invention refers to a kit comprising an immunologically effective amount of a MVA vector according to the first aspect or any of its embodiment in a container for a first or a second or subsequent administrations, wherein the kit further comprises at least another container comprising an immunologically effective amount of another immunogen of at least one SARS-CoV-2 variant for a first or a second or subsequent administrations. A preferred embodiment refers to kits comprising an immunologically effective amount of at least one immunogen of at least one SARS-CoV-2 variant in a first vial or container for a first administration (priming), and an immunologically effective amount of a MVA vector according to the first aspect or any of its embodiments, in a second vial or container for a second administration (boosting).

In another preferred embodiment of the third aspect of the invention, any of the kits referred to herein, comprise a third, fourth or further vial or container comprising any of the vaccine compositions indicated throughout the present invention for a third, fourth or further administration. Preferably, the MVA vector provided herein is used in a second, third, fourth, or further administrations.

In a preferred embodiment of the third aspect, the combination of the combination of compositions contained in the containers is capable of producing at least the S protein, or a fragment thereof, comprising the substitutions R682G, R683S, R685S, A942P, K986P and V987P derived from at least one SARS-CoV-2 variant, in the subject to be treated, and generating an immunogenic and/or protective immune response against at least one SARS-CoV-2 variant in a subject in need thereof.

In an embodiment, the vaccines compositions and kits provided in any of the previous aspects are **for use** in generating a protective immune response against at least one SARS-CoV-2 subtype, wherein the kits or vaccine composition comprise one dose of an immunologically effective amount of the vaccine compositions provided herein. Preferably, the vaccines compositions and kits provided in any of the previous aspects are for use in generating a protective immune response against at least one SARS-CoV-2 variant, wherein the kits or vaccine composition comprise at least one dose of an immunologically effective amount of the vector MVA-S(3P).

In a preferred embodiment, the vaccine composition according to the first aspect or any of its embodiment or the kits of the third aspect or any of its embodiments is for use in generating an immunogenic and/or protective immune response in a subject in need thereof, preferably in a human subject, wherein said immune response is against at least the S protein, or a fragment of said S protein, comprising the substitutions R682G, R683S, R685S, A942P, K986P and V987P and derived from at least one SARS-CoV-2 variant, wherein said composition is used for priming and/or for boosting said immune response, and wherein the MVA is capable of producing said S protein, or fragment thereof, in the subject to be treated.

In a further preferred embodiment the vaccines, vaccine combinations and kits provided in any of the previous aspects are for use in generating a protective immune response against at least one SARS-CoV-2 variant, wherein if the kits or vaccine combinations are used, the first composition is used for priming said immune response and the second composition is used for boosting said immune response or for use in generating a protective immune response against at least one SARS-CoV-2 variant, wherein the second composition is used for priming said immune response and the first composition is used for boosting said immune response. In any of the vaccines, vaccine combinations and kits provided herein the boosting composition can comprise two or more doses of the vector of the boosting composition.

Thus, in a preferred embodiment, the vaccine combination according to the second aspect or any of its embodiments is for use in generating an immunogenic and/or protective immune response in a subject in need thereof, preferably in a human subject, wherein said immune response is against at least the S protein, or a fragment of said S protein, comprising the substitutions R682G, R683S, R685S, A942P, K986P and V987P and derived from at least one SARS-CoV-2 variant, and wherein said combination is capable of producing said S protein, or fragment thereof, in the subject to be treated.

In a preferred embodiment, the vaccine composition according to the first aspect or the vaccine combinations according to the second aspect or any of their embodiments are used to induce an enhanced immune response against at least one SARS-CoV-2 variant in a subject in need thereof, preferably in a human subject, wherein the MVA is capable of producing one or more proteins, or fragments thereof, selected from the group consisting of proteins S, E, RBD, N and/or M, or any combination thereof, derived from at least one SARS-CoV-2 variant, in the subject to be treated.

As stated above, the present invention provides heterologous vaccine programs that consists of a combination vaccine and/or vaccination kit which comprises:
a. a composition comprising an immunologically effective amount of a MVA vector as defined in the first aspect or any of its embodiments, and
b. a composition comprising an immunologically effective amount of at least one immunogen of at least one SARS-CoV-2 variant,
wherein one of the compositions is a priming composition and the other composition is a boosting composition, in any order, and preferably wherein the boosting composition comprises two or more doses. Preferably, the MVA vector as defined in the first aspect or any of its embodiments is the boosting composition.

The present invention also provides homologous vaccination regimes using two equal non-replicating viral vectors. For homologous vaccine programs, the present invention thus provides a combination vaccine and/or vaccination kit which comprises:
a. a composition comprising an immunologically effective amount of a MVA vector as defined in the first aspect or any of its embodiments, and
b. a composition comprising an immunologically effective amount of a MVA vector as defined in the first aspect or any of its embodiments,
wherein one of the compositions is a priming composition and the other composition is a boosting composition, preferably wherein the boosting composition comprises two or more doses.

In this embodiment, the combination vaccines and/or kit comprises at least two vials or containers for prime/boost immunization comprising the recombinant MVA according to the first aspect or any of its embodiments for a first inoculation ("priming inoculation") in a first vial or container and for an at least second and/or third and/or further inoculation ("boosting inoculation") in a second and/or further vial or container.

The heterologous combination vaccine and/or kit can comprise multiple containers or vials of the recombinant MVA/other immunogen, together with instructions for the administration of the recombinant MVA/other immunogen to a subject at risk of SARS-CoV-2 infection. In certain embodiments, the subject is a human. In certain embodiments, the instructions indicate that the recombinant MVA/other immunogen is administered to the subject in a single dose, or in multiple (i.e., 2, 3, 4, etc.) doses. In certain embodiments, the instructions indicate that the recombinant MVA/other immunogen virus is administered in a first (priming) and second (boosting) administration to naive or non-naive subjects. As indicated above, the other immunogen of at least one SARS-CoV-2 variant is selected from the group including, but not limited to, viral vectors, recombinant proteins or polypeptides, nucleic acids, virus-like particles, nanoparticles, or any combination thereof. Preferably, said immunogen is selected from the group consisting of viral vectors, recombinant proteins or polypeptides, nucleic acids, virus-like particles, nanoparticles, or any combination thereof

The first and/or second composition or MVA and/or other immunogen of any combination vaccine, vaccination kit and/or any homologous or heterologous vaccine program of the invention can comprise any of the MVA vector described herein and any combination thereof.

### Methods and Uses of Recombinant MVA Viruses

Also provided herein are methods of use the vaccine compositions, as described in the first aspect of the invention or any of its embodiments, for immunizing a subject animal or for affecting an immune response in a subject against SARS-CoV-2 infection. Also covered are uses of the said recombinant vaccine composition described herein for the preparation of a medicament or pharmaceutical for the immunization of a subject animal, in particular for the preparation of a medicament or vaccine for treating and/or preventing a SARS-CoV-2-caused disease in a subject. Provided are also said vaccine compositions according to any embodiment herein for use in priming or boosting an immune response against a SARS-CoV-2 infection, preferably wherein the recombinant MVA is administered once, twice, three times or four times.

Further covered herein are vaccine combinations of any of the embodiments for use as a medicament or vaccine for inducing an enhanced immune response against a SARS-CoV-2 infection wherein the combination is capable of producing the antigenic determinant, and/or SARS-CoV-2 -like particles in the subject to be treated, preferably, wherein the combination vaccine is producing the encoded antigenic determinant in the subject to be treated.

Accordingly, a **fourth aspect** of the present invention provides a method of generating an immunogenic and/or protective immune response against at least one SARS-CoV-2 variant in a subject in need thereof, preferably in a human subject, the method comprising administering to the subject the recombinant MVA vaccine composition as described in the first aspect of the invention or any of its embodiments; or a kit or any of the vaccine combinations, including the homologous or heterologous vaccination regimes, as described in any of the second or third aspects of the invention or any of their embodiments. In an embodiment, said method is for priming the immune response and/or for boosting said immune response.

As used herein, the term "immunogenic and protective immune response, "protective immunity" or "protective immune response" means that the vaccinated subject is able to control an infection with the pathogenic agent against which the vaccination was done. Usually, the subject having developed a "protective immune response" develops only mild to moderate clinical symptoms or no symptoms at all. Usually, a subject having a "protective immune response" or "protective immunity" against a certain agent will not die as a result of the infection with said agent. In certain embodiments, the subject animal is a mammal. The mammal may be an adult cow, a calf, in particular a juvenile calf, a rat, rabbit, pig, mouse, but preferably a human, and the method comprises administering a dose of the recombinant MVA or combinations thereof as described in the fourth aspect of the invention.

In certain embodiments of the fourth aspect of the invention, the subject is a human. In certain embodiments, the subject is an adult. In certain embodiments, the adult is immune-compromised. In certain embodiments, the adult is over the age of 10, 15, 20, 25, 30, 25, 40, 45, 50, 55, 60, 65, 70, 75, 80, or 85 years.

The recombinant MVA provided in the first aspect of the invention, or any of the kits or vaccine combinations, including the homologous or heterologous vaccination regimes, as described in any of the second or third aspects of the invention, may be preferably administered to the subject at a dose of 1 × 10⁷, 5 × 10⁷, 1 × 10⁸ or 2 × 10⁸ PFU/dose.

In certain embodiments, any of the recombinant MVA provided in the first aspect of the invention, or any of the kits or vaccine combinations, including the homologous or heterologous vaccination regimes, as described in any of the second or third aspects of the invention, are administered to the subject at any of the doses provided herein prior to SARS-CoV-2 virus exposure as, e.g., 1 , 2, 3, or 4 weeks or 1, 2, 3, 4, 5, 6, 7, 8, 9, 10, 11, or 12 months before SARS-CoV-2 virus exposure. In certain embodiments, any of the said vaccine compositions or kits provided herein is administered to the subject at any of the doses provided herein after SARS-CoV-2 virus exposure as, e.g., 1, 2, 3, 4, 5, 6, 7, 8, 9. 10, 11, 12, 13, 14, 15, 16, 17, 18, 19, 20, 21, 22, 23, or 24 hours or 1, 2, 3, 4, 5, 6, or 7 days after SARS-CoV-2 exposure.

In certain embodiments, the recombinant MVA provided in the first aspect of the invention, or any of the kits or vaccine combinations, including the homologous or heterologous vaccination regimes, as described in any of the second or third aspects of the invention, are administered to the subject in a single dose, or in multiple (i.e., 2, 3, 4, etc.) doses. In certain embodiments, the said kits or vaccine compositions provided herein are administered in a first (priming) and second (boosting) administration.

In an embodiment, a single dose of MVA viral vector as defined herein in the first aspect of the present invention may be given to induce protective immune response, as shown in Figure 13 (vaccinated mice Group 2) and Figure 14. In a further embodiment, more than one dose of an immunologically effective amount of an MVA viral vector as defined herein in the first aspect of the present invention may be given to induce protective immune response. In particular, a single dose of an immunologically effective amount of MVA-S(3P) is preferred, although two doses might be a choice for longer term immune responses.

Boosting compositions are generally administered once or multiple times weeks or months after administration of the priming composition, for example, about 1 or 2 weeks or 3 weeks, or 4 weeks, or 6 weeks, or 8 weeks, or 16 weeks, or 20 weeks, or 24 weeks, or 28 weeks, or 32 weeks or one to two years. Preferably, the initial boosting inoculation is administered 1-12 weeks or 2-12 weeks after priming, more preferably 1, 2, 3, 4 or 8 weeks after priming. In a preferred embodiment, the initial boosting inoculation is administered 4 or 8 weeks after priming. In additional preferred embodiments, the initial boosting is conducted at least 2 weeks or at least 4 weeks after priming. In still another preferred embodiment, the initial boosting is conducted 4-12 weeks or 4-8 weeks after priming.

The recombinant MVA provided in the first aspect of the invention, or any of the kits or vaccine combinations, including the homologous or heterologous vaccination regimes, as described in any of the second or third aspects of the invention, or in any of their embodiments, can be administered systemically or locally. In certain embodiments, the said recombinant vaccine compositions are administered parenterally, subcutaneously, intravenously, intramuscularly, or intranasally, in particular intranasally or intramuscularly. Preferably, the recombinant MVA or the containers comprising the recombinant MVA are administered intranasally. In other embodiments, the said recombinant vaccine compositions are administered by any other path of administration known to the skilled practitioner. In a further preferred embodiment, the said recombinant vaccine composition is administered intramuscularly, preferably the recombinant vaccine composition is administered intramuscularly in a volume ranging between about 0.10 and 1.0 ml, preferably containing concentrations of e.g., about 1 × 10⁷ to 2 × 10⁸ PFU/ml. Preferably, the said recombinant vaccine composition is administered in a volume ranging between 0.25 and 1.0 ml. More preferably, the said recombinant vaccine composition is administered in a volume of about 0.5 ml.

Other embodiments of the invention will be apparent to those skilled in the art from consideration of the specification and practice of the invention disclosed herein. It is intended that the specification and examples be considered as exemplary only, with a true scope and spirit of the invention being indicated by the appended claims.

### Examples

### MATERIALS AND METHODS

### 1. Materials

### 1.1. Culture media

DF-1 and HeLa cells were grown in Dulbecco's modified Eagle's medium (DMEM) (Gibco) supplemented with non-essential amino acids (1X, Sigma-Aldrich) and L-glutamine (2 mM, Merck) and with 10 % fetal calf serum (FCS) (Gibco). Opti-MEM medium (Gibco) was used as a transient culture medium during plasmid transfection. DMEM was used as a medium for absorbing the virus and was removed after 1 hour, and DMEM-2 % FCS was used as a medium for incubating infections. The solid infection medium was used to select the recombinant virus plaques and consisted of a 1:1 ratio of DMEM 2X-4 % FCS and 1.9 % previously melted agarose (Conda). All the cells were kept in a humidified incubator at 37°C and 5 % CO₂.

### 1.2. Bacteria

The bacterial strains used for plasmid growth and transformations during cloning were chemically competent *Escherichia coli* DH5a cells (CNB) and electrocompetent *Escherichia coli* DH10B cells (New England Biolabs). The bacteria were cultured in Luria-Bertani (LB) medium supplemented with 1 % bacto-tryptone (BD Biosciences), 1 % NaCl (Sigma-Aldrich), and 0.5 % yeast extract (BD Biosciences) at pH 7 in the presence of ampicillin (100 µg/ml, Merck) or kanamycin (100 µg/ml, Merck) (Green MR, Sambrook J. 2012. Molecular Cloning: a laboratory manual, 4 ed. Cold Spring Harbor Laboratory Press, New York, USA)

### 1.3. Coronavirus SARS-CoV-2 S antigen (SEQ ID NO 1 and SEQ ID NO 2)

The SARS-CoV-2 antigen used to generate the recombinant MVA-CoV2-S(3P), also referred as abbreviated MVA-S(3P) vaccine candidate, is a prefusion-stabilized full-length SARS-CoV-2 S gene (Wuhan seafood market pneumonia virus isolate Wuhan-Hu-1. GenBank accession number: MN908947.3). The nucleotide sequence of this S protein was edited with codons optimized for humans by GeneArt (Thermo Fisher Scientific), and contains three mutations in the furin cleavage site (R682G, R683S, R685S) that avoid the cleavage of the S protein in S1 and S2, and three additional mutations in the S2 region that stabilizes the S protein in a prefusion conformation (A942P, K986P, and V987P).

### 1.4. Virus

### 1.4.1. MVA-based virus

The parental MVA used for generating MVA-CoV2-S(3P) vaccine candidate was a wild-type MVA (MVA-WT). The S gene was inserted in the viral TK locus (gene J2R) to generate the MVA-CoV2-S(3P) vaccine candidate. The S gene is under the control of the synthetic early/late viral promoter (pE/L).

### 1.4.2. SARS-CoV-2 virus

SARS-CoV-2 strain MAD6 (kindly provided by Jose M. Honrubia and Dr. Luis Enjuanes, CNB-CSIC, Madrid, Spain) is a virus isolated from a patient from Hospital 12 de Octubre in Madrid. The virus was isolated, plaque cloned three times and amplified at CNB-CSIC. Full-length virus genome was sequenced, and it was found to be identical to SARS-CoV-2 reference sequence (Wuhan-Hu-1 isolate, GenBank: MN908947), except the silent mutation C3037>T, and two mutations leading to amino acid changes: C14408>T (in nsp12) and A23403>G (D614G in S protein).

### 1.5. Plasmid transfer vector used to generate recombinant MVA-CoV2-S(3P)

A plasmid transfer vector pCyA was used for inserting the coronavirus SARS-CoV-2 prefusion-stabilized S(3P) gene into the MVA genome. The cells were first infected with the parental MVA-WT virus and then transfected with this plasmid. The recombinant MVA virus was subsequently selected after several consecutive plaque purification steps. The process whereby a heterologous gene is inserted into the MVA genome is called homologous recombination, a type of genetic recombination in which nucleotides sequences are exchanged between two similar or identical DNA molecules. In this case, recombination occurs between the genetic sequences of the transfer plasmid and the viral genome. In order to insert the heterologous genes into the MVA genome, the transfer plasmid must contain the following elements:
- Flanking regions of the desired insertion area: These are sequences including the left (L) and right (R) regions of the MVA TK gene such that they allow recombination processes whereby the antigens of interest are inserted in the TK locus.
- Antigen of interest: This must be located between the L and R flanking regions of the TK gene, such that as a consequence of recombination, the antigen of interest is inserted in the viral genome. This antigen was inserted into the plasmid multiple cloning site (MCS) under the control of the VACV synthetic early/late (sE/L) promoter cloned into that same site.
- Selectable marker gene in cell culture: This is located in the area adjacent to the genes of the antigens of interest and within the flanking regions, such that it is inserted into the viral genome next to the genes of interest. Another area of recombination (L flanking region) necessary for the later removal of the marker gene by additional recombination processes is found to be repeated between the antigen of interest and the marker. Recombinant MVAs containing only the antigen of interest are therefore generated, which is necessary for use as vaccine candidates in humans. In this invention, the LacZ (β-galactosidase) marker gene was used to select in cell culture the intermediary recombinant viruses forming blue lysis plaques, after insertion in the TK locus.
- Selectable gene in bacterial culture: To clone and grow plasmids, the ampicillin resistance gene (β-Lactamase) is included in such plasmids in order to select the bacteria transformed with that gene.

The plasmid transfer vector used to introduce the coronavirus SARS-CoV-2 S protein gene into the MVA TK locus is briefly described below:
▪ **pCyA-S(3P):** Plasmid transfer vector pCyA-S was used to generate MVA-S(3P) recombinant virus and was developed by the CNB. It directs the insertion of the coronavirus SARS-CoV-2 prefusion-stabilized S gene sequence, under the control of the VACV sE/L promoter, in the TK locus of the parental MVA-WT virus. We provided the pCyA vector to GeneArt (Thermo Fisher Scientific) for optimization of codons, synthesis of the prefusion-stabilized S gene of SARS-CoV-2 and insertion into the plasmid vector. More specifically, the S gene was introduced between the VACV TK-L and TK-R flanking regions, under the control of the VACV sE/L promoter, in plasmid pCyA-20 MCS (Gómez et al., 2013, J Virol 87:7282-7300). Furthermore, this plasmid also contains the selectable marker genes for ampicillin and β-galactosidase (LacZ gene). Polymerase chain reaction (PCR) and sequencing techniques confirmed that plasmid pCyA-S(3P) (11,322 bp) had been generated correctly.

### 1.6. Mice

Female mice of strain C57BL/6JOlaHsd (Envigo Laboratories) 6-8 weeks of age at the start of the experiment were used for the immunogenicity studies. These tests were approved by the Ethics Committee for Animal Testing (CEEA, *Comité Ético de Experimentación Animal*) of the CNB (Spanish National Center for Biotechnology), and by the Division of Animal Protection of the Comunidad de Madrid (PROEX 49/20), in accordance with national and international guidelines and Spanish Royal Decree (RD 53/2013), and were carried out in the animal facility of the National Center for Biotechnology (CNB; Madrid, Spain) in a pathogen-free area.

Male and female transgenic humanized K18-hACE2 mice, expressing human ACE2, were obtained from the Jackson Laboratory (034860-B6.Cg-Tg(K18-ACE2)2Prlman/J) and efficacy experiments were performed at the BSL-3 laboratory of CISA-INIA (Madrid, Spain). The experiments performed in K18-hACE2 humanized transgenic mice at the CNB-CSIC and CISA-INIA were approved by the Ethical Committee of Animal Experimentation (CEEA) of the CNB-CSIC and CISA-INIA (Madrid, Spain) and by the Division of Animal Protection of the Comunidad de Madrid (PROEX 169.4/20). Animal procedures were conformed to international guidelines (European Union (EU) Directive 2010/63EU and Recommendation 2007/526/EC) and to the Spanish law under the Royal Decree (RD 53/2013).

### 2. Reagents

### 2.1. Oligonucleotides

The following table (**Table 1**) shows the different oligonucleotides used for recombinant virus cloning, sequencing, and generation processes and those used in the PCR for detecting mycoplasma contamination.

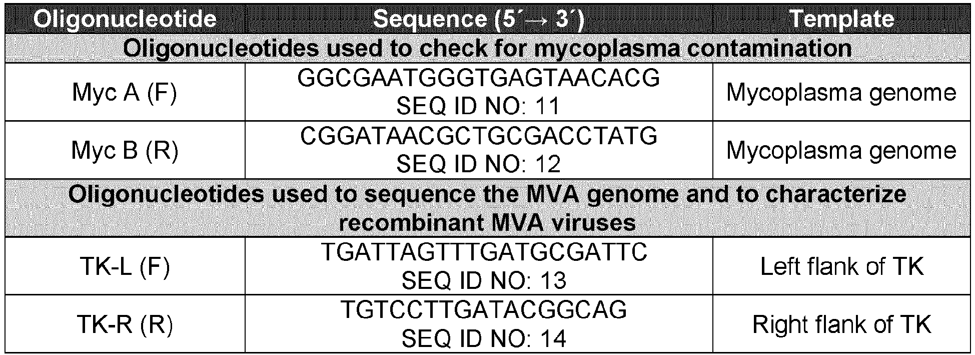

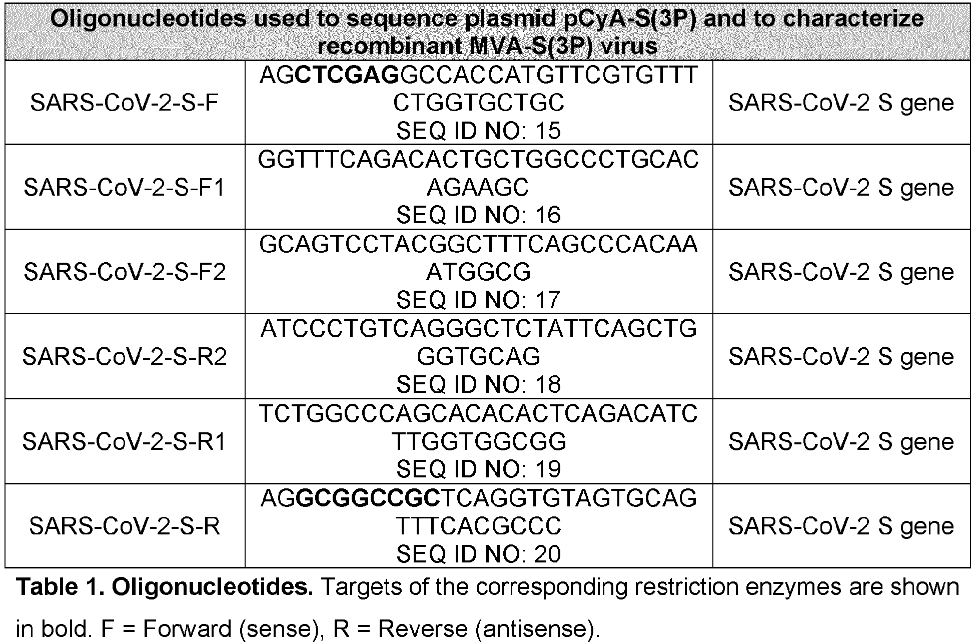

### 2.2. Peptides and proteins

### 2.2.1. Peptides

Various coronavirus SARS-CoV-2 S peptides were used for the stimulation of splenocytes isolated from mice immunized with vaccine candidates:
■ **SARS-CoV-2 S peptide pools:** These peptides are grouped into two mixtures, S1 (158 peptides) and S2 (157 peptides) (JPT). They are overlapping peptides of the coronavirus SARS-CoV-2 S-protein formed by 15 amino acids (15-mers), 11 of which are overlapping. Each peptide pool (S1 or S2) is at a concentration of 500 µg/ml.

### 2.2.2. Proteins

The coronavirus SARS-CoV-2 S and RBD proteins were used in enzyme-linked immunosorbent assays (ELISA) at a concentration of 2 µg/ml. Those proteins were provided by Dr. Jose Maria Casasnovas (CNB, Madrid)

### 2.3. Antibodies

The antibodies used in this invention are summarized in the following table **(Table 2):**

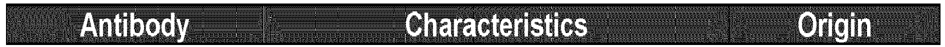

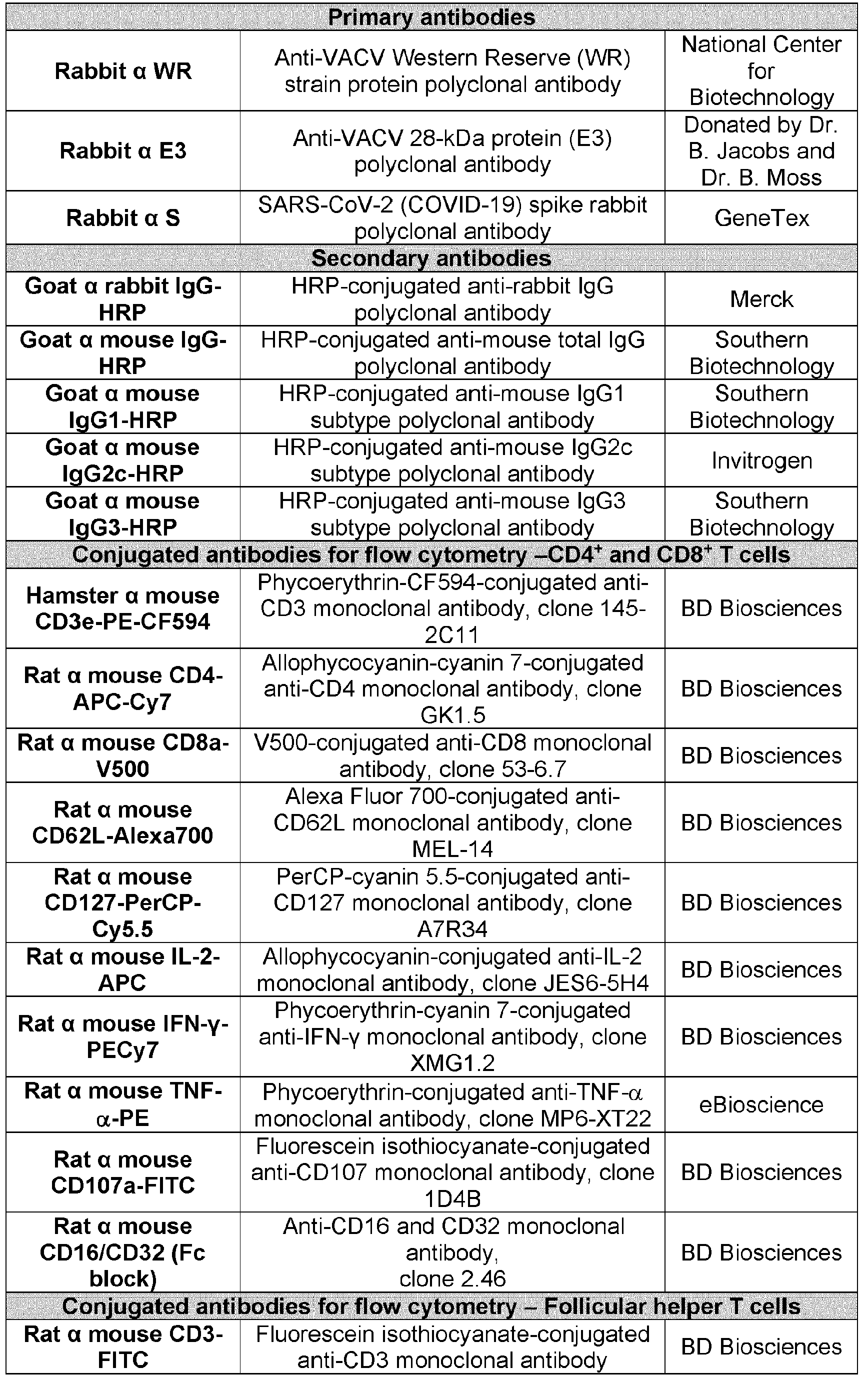

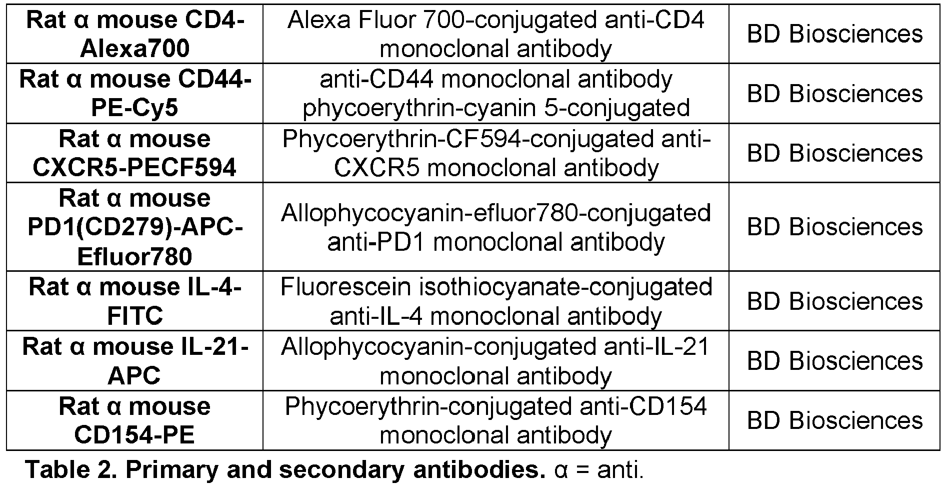

### 3. Methodology

### 3.1. DNA manipulation techniques

The DNA manipulation techniques used were carried out according to the methodology described in the book *Molecular cloning: a laboratory manual* (Green MR, Sambrook J. 2012. Molecular Cloning: a laboratory manual, 4 ed. Cold Spring Harbor Laboratory Press, New York, USA).

### 3.1.1. DNA purification

The methodology used for DNA purification differed according to the source of the genetic material:
- Purification of plasmid DNA from bacterial cultures: To extract plasmid DNA from a bacterial culture, the bacteria were previously grown in LB and then DNA was then extracted by means of different commercial kits. The purification of plasmid DNA during the molecular cloning process was performed using the *Qiagen Plasmid Mini kit* (Qiagen), from 2 ml of positive bacterial clone cultures, following the manufacturer's instructions. Larger amount of purified plasmid DNA were obtained (from 200-ml bacterial cultures) for use in transfections and infections-transfections in cell cultures using the *Qiagen Plasmid Maxi kit* (Qiagen), following the manufacturer's protocol. The plasmid DNAs used for *in vivo* experiments were purified by means of the *Qiagen Plasmid endo-free Mega kit* (Qiagen) from 500-ml bacterial cultures, following the manufacturer's recommendations.
- Purification of DNA fragments: The purification of DNA from agarose gels or PCR reactions was carried out with the *Wizard*^{®} *Genomic DNA purification Kit* (Promega), following the manufacturer's recommendations. The purification of these DNA fragments was performed during the plasmid cloning processes and for sequencing regions of interest in plasmids and recombinant viruses to verify that they are generated correctly.
- Purification of genomic DNA of cells infected in culture: This technique was used to obtain the total DNA of cells infected with the recombinant viruses and to check for purity and that such viruses were generated correctly. For that purpose, the infected cells were collected when a cytopathic effect was observed and centrifuged at 3000 revolutions per minute (rpm) for 5 min at room temperature. The supernatant was removed, the precipitate was resuspended in 50 mM Tris-HCI pH 8, and proteinase K (200 µg/ml, Roche) in its corresponding buffer was added. After 1 hour of incubation at 55°C, RNAse A (40 µg/ml, PanReac AppliChem) was added, and it was incubated for 30 min at 37°C. Then saturated NaCl was added. It was mixed by inverting the tube and centrifuged at 13000 rpm for 10 min at room temperature. The supernatant was collected, mixed with isopropanol at a ratio of 1:0.7 to precipitate the DNA, and centrifuged at 13,000 rpm for 10 min at room temperature. The precipitate was washed with 75 % ethanol and centrifuged again at 13,000 rpm for 10 min at room temperature. Lastly the supernatant was carefully removed and the precipitated DNA was left to dry at room temperature, after which it was resuspended in sterile distilled water.

In all cases, DNA concentrations were measured in a NanoDrop^{®} ND-1000 full-spectrum spectrophotometer (Thermo Fisher Scientific). Isolated DNA was later used for different experiments, including PCR, sequencing, transfections, infections-transfections, and *in vivo* assays in mice.

### 3.1.2. Polymerase chain reaction (PCR)

The PCR technique was used to amplify DNA fragments for later cloning or to check for the gene insertion in transfer plasmids or in the recombinant MVA genome. The polymerase used was *Phusion^{®} High Fidelity* (New England Biolabs) with its corresponding buffers and reagents and following the manufacturer's recommendations. About 10-100 ng of template DNA together with 0.4 mM of the corresponding oligonucleotides, 1-2.5 units of *Phusion^{®} High Fidelity* polymerase with the corresponding buffer, and 0.2 mM of each of the four deoxynucleotide triphosphates (dNTPs) (Roche Diagnostics GmbH) were used for each reaction. The temperature used for annealing was selected according to the oligonucleotides used, and the extension time used depended on the length of the fragment to be amplified. The reactions were carried out in a 96-well VeritiTM thermocycler (Applied Biosystems).

### 3.1.3. DNA detection

To separate the different DNA fragments, the electrophoresis technique in 1 % agarose gel (Conda), with 1 % *SYBR* Safe (Invitrogen) as an intercalating agent was used. The DNA fragments were then viewed by means of the Gel Doc 2000 UV light transilluminator system (Bio-Rad) and QuantityOne software (Bio-Rad).

### 3.1.4. Gene cloning into plasmids transfer vectors

Coronavirus SARS-CoV-2 S gene was cloned into transfer plasmid pCyA to generate plasmid transfer vector pCyA-S by GeneArt (Thermo Fisher Scientific).

### 3.2. Protein manipulation techniques

### 3.2.1. Protein analysis by means of sodium dodecyl sulfate-polyacrylamide gel (SDS-PAGE) electrophoresis and membrane immunodetection (Western blot)

The protein samples of the cell extracts and supernatants were analyzed by means of one-dimensional sodium dodecyl sulfate-polyacrylamide gel (SDS-PAGE) electrophoresis according to standard protocols previously described (Green MR, Sambrook J. 2012. Molecular Cloning: a laboratory manual, 4 ed. Cold Spring Harbor Laboratory Press, New York, USA). In detail, the infected or transfected cell samples were collected, centrifuged at 2,000 rpm for 5 min at room temperature, and the precipitate was resuspended in Laemmli 1X-β-mercaptoethanol loading buffer. The cell supernatants were also centrifuged at 2,000 rpm for 5 min at 4°C to separate them from T cells that might be present. Then the proteins were precipitated from the supernatant with 10 % trichloroacetic acid (TCA) (Sigma-Aldrich), centrifuged at 13,000 rpm for 15 min at room temperature, and the precipitate was resuspended in Laemmli 1X-β-mercaptoethanol. After that, both the cell extract and supernatant samples were denatured at 95°C for 10 min and were loaded in 7 % polyacrylamide gels in the presence of SDS. The proteins were separated by means of one-dimensional electrophoresis at 100 V and at room temperature for about 90 min.

Once electrophoresis ended, the SDS-PAGE gels were transferred to nitrocellulose membranes (GE Healthcare), following the wet system protocol recommended by the manufacturer (Mini Trans-Blot^{®} Cell, Bio-Rad). The gel and previously hydrated nitrocellulose membrane were arranged between two Whattman-3MM^{®} filter papers (GE Healthcare), which were also hydrated. They were then mounted in the transfer system in the presence of the transfer buffer, and the transfer was performed at a current intensity of 100 V for 50 min. Once this ended, checking for the presence of proteins was performed by staining the membrane with the reversible Ponceau S stain (0.2 % Ponceau in 3 % TCA; Sigma-Aldrich).

The nitrocellulose membrane was then blocked with a 5 % skim milk powder solution prepared in PBS 1X-0.05 % Tween20 (Sigma-Aldrich) (PBS-T) at room temperature for 1 hour with gentle stirring. Next, the corresponding primary antibodies, prepared at the desired dilution in the blocking buffer, were added and incubated under stirring at 4°C overnight. After four washes with PBS-T, the membrane was incubated with the appropriate secondary antibody (suitably diluted in the same blocking buffer) for 1 hour at room temperature and was then washed again four times with PBS-T. The membranes were developed with the ECL^{®} luminol system (Amersham), exposing *Carestream Kodak BioMax XAR* autoradiography film (Kodak) or directly in the *ChemiDoc*^{™} *Imaging System* (Bio-Rad). The proteins bands were analyzed and quantified by means of *Image Lab software* (Bio-Rad).

**3.2.2. Expression of SARS-CoV-2 S protein from recombinant MVA-S and MVA-S(3P) by flow cytometry.** Monolayers of HeLa cells were mock infected or infected at 5 PFU/cell with MVA-WT, MVA-S, or MVA-S(3P). At 24 hpi, cells were collected by scraping and centrifuged at 2,000 rpm for 5 min, and the cellular pellets were resuspended in phosphate-buffered saline (PBS) staining solution (1X PBS, 0.5% bovine serum albumin [BSA], 1% FCS, 0.065% sodium azide, 2 mM EDTA) and added to a 96-well plate at a rate of 200,000 cells per well. Then, Fc block (BD Pharmingen) was added for 20 min at 4°C. For the staining, mouse monoclonal antibodies against S and RBD proteins were added to the cells for 30 min at 4°C, and then anti-mouse IgG fluorescein isothiocyanate (FITC)-conjugated antibodies (diluted 1:100; eBioscience) were added to the cells as secondary antibodies for 20 min in the dark at 4°C. Finally, cells were fixed with 4% paraformaldehyde (PFA) for 20 min in the dark at 4°C and were acquired using a Gallios flow cytometer (Beckman Coulter). Data were analyzed using FlowJo software (version 8.5.3; Tree Star, Ashland, OR).

### 3.3. Virus manipulation techniques

### 3.3.1. Viral infections

All the viruses were kept at -70°C and thawed at 37°C in a bath prior to use. Once thawed, they were stirred with a vortex and sonicated with a 3-cycle 10-second sonication and 10-second pause program (Misonix Incorporated S-3000 Sonicator, Cole-Parmer). The viruses were then added to the cell monolayer with the minimum volume of (serum-free) DMEM needed to cover the cell monolayer at the desired MOI. After 1 hour of adsorption at 37°C, the inoculum was removed and fresh DMEM-2 % FCS medium was added. The time the different infections were maintained varied with the objective of the infection, and the manner in which the cells were collected also varied.

### 3.3.2. Generation of recombinant MVA-S(3P) virus

In this invention, a recombinant MVA virus expressing coronavirus SARS-CoV-2 prefusion-stabilized S protein [MVA-S(3P)] was generated in DF-1 cells according to the previously described standard infection/transfection protocol (Earl et al., 2001, Curr Protoc Mol Biol, 16:16 17), with certain modifications. To generate vaccine candidate MVA-S(3P), MVA-WT was used as the parental virus, as previously described (Garcia-Arriaza et al., 2014, J Virol 88:3527-3547).

In detail, the DF-1 cell monolayer cultured in p60 dishes (Nunc) at 80-90 % confluence was infected at a MOI of 0.05 PFU/cell with MVA-WT parental virus. In the meantime, a mixture of DNA-lipofectamine 2000 was prepared by mixing 10 µg of the corresponding transfer plasmid with the suitable volume of lipofectamine 2000 (Invitrogen) in Opti-MEM (Gibco) (1.5 µl lipofectamine 2000/µg of DNA) for 20 min at room temperature to form DNA-liposome complexes. After 1 hour of adsorption of the virus, the inoculum was removed, the cells were washed two times with Opti-MEM and incubated with 1 ml of the DNA-lipofectamine mixture for 4-6 hours at 37°C and 5 % CO₂.Next the transfection medium was removed, the cells were washed two times with Opti-MEM and incubated with DMEM-2 % FCS medium at 37°C and 5 % CO₂. When a cytopathic effect was observed (usually after 3 days), the cells were collected, centrifuged at 1,500 rpm for 5 min at room temperature, and resuspended in 500 µl of serum-free DMEM. The cells were then frozen (at -70°C) and thawed (at 37°C) three times, stirring with a vortex between each freeze-thaw cycle to lyse them and release the virus into the supernatant, which was obtained after centrifuging at 3,000 rpm for 5 min at room temperature. The virus was then used to infect confluent monolayers of DF-1 cells grown in 6-well plates (M6) (Nunc) at different serial dilutions (from 10⁻¹ to 10⁻⁶) in serum-free DMEM. After 1 hour of infection, the inoculum was removed and replaced with 3 ml of solid infection medium consisting of a mixture of 1.9 % (previously melted) agarose (Conda) with DMEM 2X-4 % FCS medium at a 1:1 ratio. After 48 hours of infection, 1 ml of the 1.9 % agarose-DMEM 2X-4 % FCS 1:1 mixture and 1.2 mg/ml of X-Gal substrate (Sigma-Aldrich) was added to view the lysis plaques. After 24 hours, the blue plaques that were more isolated from other lysis plaques were selected. The selected lysis plaques were obtained with a 150-mm glass Pasteur pipette (Deltalab) and added to 500 µl of serum-free DMEM. After three freeze-thaw cycles and subsequent stirring with a vortex and sonication, these plaques were used as an inoculum to infect monolayers of confluent cells in 24-well plates (M24) (Nunc) as explained in section 3.3.1. These infections were performed to re-grow the virus, and they were collected when a cytopathic effect was observed. These regrown plaques were next used to re-infect monolayers of confluent cells in M24 plates, and once a cytopathic effect was observed, these cells were collected and lysed for PCR and Western Blot analysis to check that the recombinant MVA virus was generated correctly. Oligonucleotides TK-L and TK-R were used in the PCR to amplify the antigens inserted in the TK locus (oligonucleotides described in **Table 1**). Anti-S-protein rabbit polyclonal antibody (described in **Table 2**) was used to analyze expression of the S protein by Western blot.

Once the presence of the antigen and its correct expression by the different plaques were confirmed, one of them was selected and used as an inoculum to infect monolayers of confluent cells in M6 plates at dilutions from 10⁻¹ to 10⁻⁶. This purification process was carried out three times selecting blue plaques, and three more times selecting colorless plaques. A purified recombinant virus containing the antigen of interest in the TK locus and without the LacZ marker, which was lost through homologous recombination, is therefore ultimately obtained.

Three types of viral stocks are distinguished in this invention. The initial viral stock (stock P1) consists of the generated recombinant virus originating from cell monolayers infected with the final plaque and collected after observing a cytopathic effect. Once having verified the expression of the antigen of interest by means of Western Blot and the purity of the recombinant virus by means of PCR and sequencing, it was used to generate the working viral stock (stock P2). For that purpose, 5 p150 plates (Nunc) containing confluent DF-1 cells were infected at an MOI of 0.05 PFU/cell and cells were collected when a cytopathic effect was observed. Stock P2 was used in all the experiments for characterizing the virus *in vitro.* Lastly, for the *in vivo* assays, the purified viral stock (stock P3) was generated from stock P2 (described in section 3.3.4.).

### 3.3.3. Characterization of the recombinant MVA-S(3P) virus

The experiments performed to characterize the recombinant MVA-S(3P) virus include titration of the viruses, viral growth kinetics, and stability analysis of the inserted S antigen. As mentioned above, stock P2 was used to perform these characterization experiments.

### • Viral titration

Titration of the MVA-S(3P) virus was carried out according to the previously described standard protocols (Ramírez et al., 2000, J. Virol, 74:923-933). DF-1 cells seeded in M6 plates were infected in duplicate with serial dilutions of the virus in serum-free DMEM, as described in section 3.3.1. The medium was removed 30-40 hours post-infection and the cells were fixed for 3 min using a 1:1 mixture of methanol and acetone and the titer was determined by means of an immunostaining assay which allows detecting and counting lysis plaques formed by the virus. The monolayers were thereby incubated with a rabbit anti-VACV WR polyclonal primary antibody (diluted 1:1.000 in PBS-3 % FCS; CNB) for 1 hour at room temperature; it was then washed three times with PBS 1X and incubated for 1 hour at room temperature with the HRP-conjugated goat anti-rabbit secondary antibody (diluted 1:1.000 in PBS-3 % FCS; Sigma-Aldrich). After three additional washes with PBS 1X, the plates were developed using 1 mg/ml of 3,3'-diaminobenzidine tetrahydrochloride (DAB; Sigma-Aldrich), as the HRP substrate, with 30 % hydrogen peroxide (Sigma-Aldrich) and 3 % nickel sulfate (NiSO₄) (Sigma-Aldrich). After counting the lysis plaques, the titer was referenced as PFU/ml.

### • Replication kinetics

To evaluate the replication capacity of the recombinant MVA-S(3P) virus and analyze if the insertion of the S gene affects their replication capacity, monolayers of DF-1 cells were infected with each virus at an MOI of 0.01 PFU/cell, in duplicate. After 1 hour of adsorption, the inoculum was removed and was added to the DMEM-2 % FCS infection medium monolayer. The cells were collected at different times post-infection (0, 24, 48, and 72 hours), subjected to three freeze-thaw cycles (stirred with a vortex between each cycle), and kept at -70°C until titration. Lastly, the viruses were sonicated and titrated using the method described in the preceding section.

### • Genetic stability analysis

The genetic stability of vaccine candidates MVA-S(3P) was analyzed. Monolayers of DF-1 cells were infected with MVA-S(3P) recombinant virus at an MOI of 0.05 PFU/cell. The cells were collected 72 hours post-infection, frozen-thawed three times, and briefly sonicated. The cell extracts were then centrifuged at 1,500 rpm for 5 minutes, and the supernatant was used for a new round of infection at a low MOI. The same method was repeated eight times. Expression of the coronavirus SARS-CoV-2 S protein was analyzed by means of Western blot (see section 3.2.3.) after infecting DF-1 cells with the viral stocks from each passage.

### 3.3.4. Virus purification

Purified virus stocks (stock P3) were used for the *in vivo* assays according to the method first described by Joklik (Joklik, 1962, Virology, 18:9-18) and modified by Esteban (Esteban, 1984, Virology, 133:220-227). In summary, 30 p150 plates containing confluent CEF cells were infected with a stock P2 of MVA-S(3P) at an MOI of 0.01 PFU/cell. The cells were collected when a cytopathic effect was observed (usually after 3 days), centrifuged at 1,500 rpm for 5 min at 4°C, and the precipitate was washed with PBS and resuspended in 10 mM Tris-HCI buffer pH 9. The infected cells were then lysed by means of three sonication cycles, stirring with a vortex, and centrifugation to release the viral particles into the supernatant. Next, this supernatant was centrifuged for 1 hour at 20,000 rpm at 4°C in the SW28 rotor (Beckman) on a 36 % sucrose pad in 10 mM Tris-HCI pH 9. The obtained precipitate was resuspended in 10 mM Tris-HCI pH 9 and centrifuged again on another 36 % sucrose pad under the same conditions. Lastly, the precipitate obtained in this second centrifugation was resuspended in 10 mM Tris-HCI pH 9, aliquoted, and frozen at -70°C until use.

The purified virus was tittered in duplicate and checked for the absence of mycoplasma contamination (through PCR with mycoplasma-specific oligonucleotides; see **Table 1**), bacteria (by means of growth in LB agar plates), and fungi (by means of growth in blood agar plates, Oxoid). Furthermore, checking for the correct expression of the S antigen of stock P3 was performed by means of PCR and Western blot before use in the *in vivo* assays.

### 3.4. In vivo immunological techniques

### 3.4.1. Immunization protocols

In assays for analyzing adaptive immune responses 6 female C57BL/6 mice per group were inoculated by intramuscular route using a homologous prime-boost immunization protocol, which consisted of a first inoculation with 1 × 10⁷ PFU of vaccine candidates MVA-S and MVA-S(3P), followed by a 1 × 10⁷ PFU booster dose of the same vaccine candidates MVA-S and MVA-S(3P) 2 weeks later. The mice were sacrificed 10 days after the booster dose. All the preparations of the inocula were performed in endotoxin-free PBS (Gibco). Intramuscular immunization was performed in the triceps of both hind legs in a total volume of 100 µl of PBS (50 µl/leg), using a BD Micro-Fine (30 G) × 8 mm insulin syringe (BD Biosciences). At the end of each immunization study the corresponding animals were sacrificed using CO₂, and blood samples were drawn and spleens removed, as appropriate in each experiment.

### 3.4.2. Analysis of the adaptive T cell-mediated immune response by means of ELISpot

The ELISpot assay was used to detect S antigen-specific IFNy-secreting T cells. First, 96-well nitrocellulose-bottom plates (Millipore) were covered with 75 µl/well of a solution of the rat anti-mouse IFNγ monoclonal antibody (Pharmingen, San Diego, California) at a concentration of 6 µg/ml in PBS. After incubating overnight at room temperature, the wells were washed three times with RPMI medium and blocked with RPMI-10 % FCS for at least one hour at 37°C in 5 %CO₂ atmosphere. Moreover, the spleens of each group of immunized animals were collected in RPMI-10 % FCS medium and processed together to obtain the splenocytes as previously described (García-Arriaza et al., 2013, PLoS One, 8:e66894; Nájera et al., 2010, PLoS One, 5:e11406). In summary, the spleens were homogenized by means of mechanical disintegration through a 40-µm cell strainer (Falcon). The disintegrated cells were centrifuged for 5 min at 1,500 rpm at 4°C and washed with RPMI-10 % FCS. Next, the erythrocytes were lysed by adding 0.1 M NH₄Cl (2 ml/spleen) for 5 min in ice. After this time, the cells were washed three times with RPMI-10 % FCS to eliminate fat and resuspended in 12 ml of RPMI-10 % FCS, and the number of living splenocytes was counted by means of staining with trypan blue (4 % in water; Sigma-Aldrich).

To evaluate the specific response to the different antigens, 10⁶ splenocytes per condition were re-stimulated with 1 µg/ml of the S1 or S2 peptide pool. As a control of the response to the MVA vector, 10⁶ splenocytes were stimulated with 2.5 µg/ml of VACV E3 peptide. The plates were incubated with the peptides for 48 hours at 37°C in 5 % CO₂ atmosphere, washed five times with PBS-T, and incubated with 2 µg/ml of biotinylated rat anti-mouse IFNγ monoclonal antibody XMG1.2 (Pharmingen) diluted in PBS-T, for 2 hours at room temperature. The plates were then washed five times with PBS-T and a 1:800 dilution of HRP-conjugated streptavidin (0.5 mg/ml; Sigma-Aldrich) was added. After 1 hour at room temperature, it was washed three times with PBS-T and two times with PBS, and finally 1 µg/ml of the DAB substrate (Sigma-Aldrich), resuspended in 50 mM Tris-CI pH 7.5 and 0.015 % H₂O₂, was added to develop the plates. The reaction was stopped by washing the plate with abundant water and once it was dry, the spots were counted using the *ELISpot Reader System -ELR02-* plate reader (AID Autoimmun Diagnostika GmbH) with the aid of *AID ELISpot reader system software* (Vitro).

### 3.4.3. Analysis of the adaptive cell-mediated immune response by means of flow cytometry

### 3.4.3.1. Analysis of the specific responses of CD4⁺ and CD8⁺ T cells

To study the response of SARS-CoV-2-specific T cells generated (see section 3.4.1.), 6 mice per group were immunized and sacrificed 10 days after the last immunization. After sacrificing the animals, the spleens were removed from the immunized animals in RPMI-10 % FCS medium and processed as described in the preceding section. The splenocytes were then resuspended in RPMI-10 % FCS medium with 2 µl/ml of Monensin 1X (Thermo Fisher Scientific), 2 µl/ml of brefeldin A (BFA) (*Protein Transport Inhibitor BD GolgiPlug*, BD Biosciences), and a 1:300 dilution of anti-CD107a-FITC antibody. 4 × 10⁶ cells were added in M96 conical bottom wells and were incubated for 6 hours in the presence of 1 µg/ml of the S1 or S2 peptide pool, or with 5 µg/ml of VACV E3 peptide to determine the response to the MVA vector. As controls, the splenocytes were stimulated with 2 µl/ml of LAC (Leukocyte Activation Cocktail, BD Biosciences) and Monensin 1X. After 6 hours of stimulation, the splenocytes were washed with IB buffer and incubated with *Violet Dye* (0.5 µl/ml, Invitrogen) for 20 min in the dark at 4°C to evaluate cell viability. Next, two washes were performed with IB buffer and the splenocytes were incubated for 20 min in the dark at 4°C with 50 µl of the primary surface antibodies (described in the **Table 2)** diluted as indicated for each batch. The splenocytes were then washed two times and 100 µl of *Cytofix*/*Cytoperm* (BD Biosciences) was added per well for 20 min in the dark to 4°C for fixing and permeabilizing the cells, and they were then left in IB buffer overnight at 4°C in the dark. A wash was performed the next day with *Permwash* 1X (BD Biosciences) and 25 µl of *Fc Block* diluted 1:100 in *Permwash* 1X were added for 5 min at 4°C in the dark. Next, 25 µl of the antibodies needed for intracellular cytokine staining (ICS) were added to the 25 µl of *Fc Block*, at different dilutions in *Permwash* 1X according to the batch of each antibody (see **Table 2**), with incubation being performed for 20 min at 4°C in the dark. Lastly, two washes were performed with *Permwash* 1X and the cells resuspended in 200 µl of IB buffer to later be analyzed by means of flow cytometry in a Gallios cytometer (Beckman Coulter) and by means of the *FlowJo* program (*Tree Star*, Ashland, Oregon). The analysis strategy was performed by selecting the living cells expressing CD3 on their surface; then those expressing CD4, CD8, CD127, or CD62L; and finally those secreting the different cytokines (IFN-y, TNF-α, and IL-2) or expressing degranulation marker CD107a.

### 3.4.3.2. Analysis of the specific responses of follicular helper T cells

To analyze the response of follicular helper T cells (Tfh) against SARS-CoV-2, spleens were extracted from sacrificed animals and processed as explained above. The splenocytes were then resuspended in RPMI-10% FCS medium with 1 µl/ml of BFA (BD Biosciences), 1 µl/ml of Monensin 1X (eBioscience), and a 1:100 dilution of the anti-CD154 (CD40L)-PE antibody. Next, 4 × 10⁶ splenocytes were stimulated with 5 µg/ml of S-protein and 1 µg/ml of S1 and S2 peptide pools for 6 hours at 37°C in a conical-bottom 96-well plate. The cells were then stained with Fixable Viability Stain (FVS) 520 (BD Biosciences) for 20 min at 4°C in the dark to analyze cell viability. The splenocytes were then washed two times with IB buffer and stained for 20 min at 4°C in the dark with 50 µl of the antibodies specific against the surface markers (described in **Table 2**), using the dilutions recommended by the manufacturer. Next, the splenocytes were again washed two times with IB buffer, fixed, and permeabilized with the *BD Cytofix*/*Cytoperm*^{™} *kit* (BD Biosciences) for 20 min at 4°C in the dark, and they were left in IB buffer overnight at 4°C in the dark. The cells were washed the next day with *Permwash* 1X (BD Biosciences) and the Fc receptors were blocked with 25 µl of *Fc Block* (diluted to 1:100 in *Permwash* 1X) for 5 min at 4°C in the dark. Next, 25 µl of the antibodies (diluted in *Permwash* 1X at the dilution indicated by the manufacturer) required for ICS (see **Table 2)** were added on 25 µl of *Fc Block*, incubating for 20 min at 4°C in the dark. Finally, the splenocytes were washed two times in *Permwash* 1X, resuspended in 200 µl of IB buffer, and passed through a Gallios flow cytometer (Beckman Coulter). The analysis was performed with the *FlowJo* software (*Tree Star*, Ashland, Oregon). The analysis strategy was performed by selecting living cells expressing CD3, CD4, and CD44 on their surface, followed by cells expressing CXCR5, PD1, and CD154 (CD40L), and finally cells secreting the different cytokines (IFN-y, IL-4, and IL-21).

### 3.4.4. Analysis of the humoral immune response

The humoral immune response was determined by analyzing the levels of IgG antibodies in the sera of immunized mice and the capacity of these antibodies to neutralize the SARS-CoV-2 virus. To obtain the sera, blood was collected from the animals by submandibular bleeding or after sacrificing them by means of intracardiac puncture. Next, the blood samples were maintained at 37°C for 1 hour and kept at 4°C overnight. The tubes of blood were centrifuged the next day at 3600 rpm for 20 min at 4°C and the serum was obtained, inactivated at 56°C for 30 min and kept at -20°C until use. Serial dilutions of each individual serum sample (or group of sera) were then performed to measure the titers of coronavirus SARS-CoV-2 S-protein-specific and RBD-specific antibodies by means of ELISA, or to measure their SARS-CoV-2 neutralizing capacity by means of a luciferase assay. These techniques are explained in the following sections.

### 3.4.4.1. Enzyme-linked immunosorbent assay (ELISA)

ELISA assays were performed to analyze the levels of antibodies in the serum of immunized animals. NUNC MaxiSorp^{™} 96-well plates (Thermo Fisher Scientific) were coated with 2 µg/ml of the coronavirus SARS-CoV-2 S and RBD proteins in PBS and incubated at 4°C overnight. The plates were washed three times the next day with PBS-T and blocked for 2 hours at room temperature with 5% skim milk prepared in PBS-T. After this time elapsed, the plates were washed three times with PBS-T, 50 µl of the serial dilutions of the serum samples diluted in PBS with 1% skim milk and 0.01% Tween20 were added, and the plates were incubated for 1.5 hours at room temperature. Next, the plates were again washed three times with PBS-T and incubated with 50 µl of HRP-conjugated mouse anti-IgG, -lgG1, -IgG2b, -IgG2c, or -IgG3 antibodies (diluted to 1:1000 in PBS with 1% skim milk and 0.01% Tween20) for 1 hour at room temperature. Finally, after another washing step, the plates were developed by adding 100 µl of TMB substrate (Sigma-Aldrich) and the reaction was stopped by adding 50 µl of 1 M H₂SO₄. Absorbance was read at 450 nm by means of an EZ Read 400 microplate reader (Biochrom). Total IgG titers were measured as the last dilution that gives an absorbance at least 3 times higher the absorbance of a naive serum.

### 3.4.4.2. Neutralization assay

The capacity of the sera obtained from mice immunized with vaccine candidates MVA-S and MVA-S(3P), to neutralize the SARS-CoV-2 S virus was determined using retrovirus-based pseudoparticles expressing the SARS-CoV-2 S protein. Pseudotyped viruses were produced by transfection of a mammalian DNA expression plasmid expressing the full-length S protein (DNA-S), together with packaging plasmids (kindly provided by Dr. F. L. Cosset (INSERM, Lyon)) in HEK-293T cells. Vero E6 cells were seeded in 96-well plates at 15,000 cells/well. Twenty-four hours post-seeding, a mix of retrovirus-based pseudoparticles expressing SARS-CoV-2 S protein and mice serum samples (different serial dilutions) were preincubated for 1 hour at 37°C and then added to the cells in triplicates. The DMEM-2%FCS medium was replaced 24 h after infection, and then, 24 hours later, cells were lysed in passive lysis buffer (Promega, Madison, WI) and the luciferase activity was measured in a luminometer (Thermo Appliskan Multimode Microplate Reader, Thermo Fisher Scientific). The ID50 titers were determined as the highest dilution of serum which resulted in a 50% reduction of luciferase units compared with serum samples from control group (pseudotyped viruses not incubated with serum).

Furthermore, live-virus SARS-CoV-2 neutralization antibody titers were assessed by a microneutralization test (MNT), using SARS-CoV-2 MAD6 strain in a BSL-3 laboratory at the CNB-CSIC. Serum samples were inactivated at 56°C for 30 min and then serially diluted two-fold in duplicate in serum-free DMEM and incubated at a 1:1 ratio with 100 TCID50 (50% Tissue Culture Infectious Dose) of SARS-CoV-2 MAD6 isolate at 37° C for 1 hour. The virus/serum mixture were added to VeroE6 cell monolayers, seeded in 96-well plates (40.000-50.000 cells per well), and incubated at 37° C, in a 5% CO₂ incubator for 3 days. Then, cells were fixed with 4% formaldehyde and stained with crystal violet. ID50 titers were calculated as the reciprocal dilution resulting in 50% inhibition of cell death. A WHO International Standard containing pooled plasma obtained from eleven individuals recovered from SARS-CoV-2 infection was used for the calibration and harmonisation of serological assays detecting anti-SARS-CoV-2 neutralizing antibodies (NIBSC code: 20/136).

### 3.5. Efficacy study in K18-hACE2 transgenic mice.

Male and female K18-hACE2 mice (9 weeks old at the beginning of the study) immunized with one dose of MVA-S and MVA-S(3P) were used to evaluate the efficacy of MVA-S(3P) vaccine candidate. Groups of animals (n = 11) received one dose of 1 × 10⁷ PFU of MVA-S or MVA-S(3P) by the i.m. route in 100 µl of PBS (50 µl/leg) at week 0. Mice primed with the same dose of non-recombinant MVA-WT were used as a control group. At week 4 mice were lightly anesthetized with isoflurane and infected intranasally with 1 × 10⁵ PFU of SARS-CoV-2 MAD6 in a total volume of 50 µl of PBS. Mice were monitored daily for body weight change and survival for 15 days postchallenge. Animals with more than a 25% of weight loss were euthanized. At day 4 postchallenge, six mice per group were euthanized, and lungs and serum samples were collected. One lung was divided longitudinally in two, with one part placed in RNALater stabilization reagent (Sigma-Aldrich) and stored at -80°C until RNA extraction, and the other part stored also at -80°C until analysis of virus yields. Blood was collected from the animals by submandibular bleeding, maintained at 37°C for 1 hour, kept at 4°C overnight, and centrifuged at 3600 rpm for 20 min at 4°C to obtain the serum samples, which was then inactivated at 56°C for 30 min and kept at -20°C until use. All experiments with SARS-CoV-2 were performed in a Biosafety Level 3 (BSL-3) Laboratory at the CISA-INIA.

**3.5.1. Analysis of SARS-CoV-2 RNA by quantitative RT-PCR (RT-qPCR).** Lungs from K18-hACE2 mice used in the efficacy study were harvested at 4 days post-challenge and stored in RNALater (Sigma-Aldrich) at -80°C until homogenized with a gentleMACS dissociator (Miltenyi Biotec) in 2 ml of RLT buffer (Qiagen) plus β-mercaptoethanol (Sigma-Aldrich) and aliquoted. Then, 600 µl of homogenized lung tissue was used to isolate total RNA using the RNeasy minikit (Qiagen), according to the manufacturer's specifications. First-strand cDNA synthesis and subsequent real-time PCR were performed in one step using NZYSpeedy One-step RT-qPCR Master Mix (NZYTech) according to the manufacturer's specifications using ROX as reference dye. SARS-CoV-2 viral RNA content was determined using previously validated set of primers and probes specific for the SARS-CoV-2 subgenomic RNA for the protein E, and the cellular 28S rRNA for normalization. Data were acquired with a 7500 real-time PCR system (Applied Biosystems) and analyzed with 7500 software v2.0.6. Relative RNA arbitrary units (A.U.) were quantified relative to the negative group (uninfected mice) and were performed using the 2-ΔΔCt method. All samples were tested in triplicate.

**3.5.2. Analysis of SARS-CoV-2 virus yields by plaque assay.** Lungs from K18-hACE2 mice used in the efficacy study were harvested at 4 days post-challenge, weighted, and stored directly at -80°C until homogenized with a gentleMACS dissociator (Miltenyi Biotec) in 2 ml of PBS buffer and aliquoted. Then, undiluted and serial ten-fold dilutions of homogenized lung tissue were added in triplicate to Vero-E6 cell monolayers seeded in 12-well plates at 5 × 10⁵ cells/well and after 1 hour of adsorption the inoculum was removed and plates were incubated at 37°C, 5% CO₂ in 2:1 DMEM 2X-4% fetal bovine serum (FBS):2% Agar. After 4 days, cells were fixed for 1 hour with 10% formaldehyde (Sigma-Aldrich), and then the agarose was removed and plaques were visualized by adding 0.5% crystal violet (Sigma-Aldrich). SARS-CoV-2 titers were determined in PFUs per gram of lung tissue.

### RESULTS

In this invention, a new MVA viral vector-based vaccine have been designed and developed against the coronavirus SARS-CoV-2 causing the COVID-19 pandemic. Once generated, this recombinant MVA virus have been characterized *in vitro* and their capacity to stimulate adaptive SARS-CoV-2-specific T cell and humoral immune responses *in vivo* in immunized mice has been analysed, as well as its efficacy in humanized K18-hACE2 mice.

### 1. Design, generation and in vitro characterization of vaccine candidate MVA-S(3P) expressing a prefusion-stabilized SARS-CoV-2 S protein

To generate novel vaccines against SARS-CoV-2, the causative agent of COVID-19 pandemic, that could stimulate SARS-CoV-2-specifc T- and B-cell immune responses, we have developed a new MVA-based vaccine candidate expressing the SARS-CoV-2 full-length prefusion-stabilized S structural gene (Wuhan seafood market pneumonia virus isolate Wuhan-Hu-1, Genbank number: MN908947.3) [termed MVA-S(3P)] (see Materials and Methods). Vaccine candidate MVA-S(3P) was generated following the insertion of the complete sequence of the coronavirus SARS-CoV-2 S gene (prefusion-stabilized) in the genome of the parental virus MVA-WT. The expression of the SARS-CoV-2 S gene is under the transcriptional control of a synthetic early/late (sE/L) promoter. MVA-CoV2-S(3P) vaccine candidate express a human codon optimized full-length S protein that contains three mutations in the furin cleavage site (R682G, R683S, R685S) that avoid the cleavage of the S protein in S1 and S2, and three additional mutations in the S2 region that stabilizes the S protein in a prefusion conformation (A942P, K986P, and V987P) (Figure 1A).

Figure 1A shows a diagram of the generated vaccine candidate MVA-S(3P). The methodology used for generating this vaccine candidate has been described above in Materials and Methods. The correct insertion of the coronavirus SARS-CoV-2 S gene and the purity of the generated vaccine candidate MVA-S(3P) was confirmed by means of PCR and sequencing, after amplifying the DNA obtained from DF-1 cells that were not infected (mock) or infected with 5 PFU/cell of MVA-WT, MVA-S, and MVA-S(3P). Oligonucleotides (described in **Table 3** of Materials and Methods) which hybridize in the flanking regions of the MVA TK gene (J2R gene) were used in the PCR, and the obtained results confirmed the presence of the S gene in MVA-S and MVA-S(3P), without contamination with the parental MVA-WT (Figure 1B). The TK locus of parental virus MVA-WT was amplified as PCR control, with the TK gene being amplified. The correct presence of the S gene inserted in the TK locus was also confirmed by means of DNA sequencing.

To demonstrate that MVA-S(3P) constitutively express the prefusion-stabilized SARS-CoV-2 S protein, we performed a Western blot analysis of cell extracts from MVA-infective permissive chicken DF-1 cells infected at a MOI of 5 PFU/cell for 24 h with MVA-S (expressing a full-length non-stabilized S protein), MVA-S(3P), or MVA-WT and leave in the presence or absence of tunicamycin, an inhibitor of protein N-glycosylation, to analyze the glycosylation pattern of SARS-CoV-2 S protein expressed from MVA, using specific antibodies that recognize the SARS-CoV-2 S protein. The results proved that MVA-S and MVA-S(3P) vaccine candidates correctly expressed the SARS-CoV-2 S protein (of an expected molecular weight of about 180 kDa, corresponding to the full-length glycosylated S protein) at 24 h postinfection (hpi), being detected using a rabbit polyclonal antibody against SARS-CoV-2 (against SARS-CoV-2 S1 region) (Figure 1C). Furthermore, tunicamycin treatment inhibited SARS-CoV-2 S glycosylation and a single band of about 130 kDa, corresponding to the full-length unglycosylated S protein was detected (Figure 1C).

Next, we analyzed the ability of the SARS-CoV-2 S protein expressed from MVA-S(3P) recombinant to form oligomers, and compared to MVA-S. Thus, at 24 hpi, we collected cell extracts under nonreducing conditions (without β-mercaptoethanol) from DF-1 cells infected at a MOI of 5 PFU/cell with MVA-S, and MVA-S(3P), and then we analyzed by Western blotting the expression of SARS-CoV-2 S protein, using a rabbit polyclonal antibody against SARS-CoV-2 (Figure 1D). Immunoblots showed that under nonreducing conditions, the SARS-CoV-2 S protein expressed from MVA-S and MVA-S(3P) recombinants existed mainly as high-molecular-mass oligomers; presumably trimers (Figure 1D).

The secretion of the S protein in the supernatant was then analysed at different times post-infection. Cell extracts, or supernatants were collected from DF-1 cells infected with vaccine candidates MVA-S and MVA-S(3P), and with parental virus MVA-WT, at an MOI of 5 PFU/cell at 4, 7 and 24 hours post-infection. Next, the expression of the S protein in cell extracts and the secretion of said protein in the extracellular medium was analysed by means of Western blot (Figure 1E). The results showed that the expression of the S protein was detected from 4 hours post-infection in the cell extracts originating from vaccine candidates MVA-S and MVA-S(3P) (Figure 1E). However, the expression of the S protein in the supernatants was not detected (Figure 1E) because the S-protein inserted in the MVA genome is a transmembrane protein.

Moreover, we also analyzed the expression of the S protein in non-permissive HeLa cells infected with MVA-S and MVA-S(3P), and the results showed a similar pattern of S expression to the one described in permissive DF-1 cells (Figures 2A, 2B, 2C).

In conclusion, vaccine candidates MVA-S and MVA-S(3P), efficiently express the coronavirus SARS-CoV-2 S protein.

### 2. In vitro characterization of vaccine candidate MVA-S(3P)

### 2.1. Vaccine candidate MVA-S(3P) is stable in cell culture

To confirm that vaccine candidate MVA-S(3P) is stable and can be maintained in cultured cells without the loss of the S gene, DF-1 cells were infected with MVA-S(3P) with a low MOI for 8 consecutive serial passages, and the expression of the S-protein was determined by means of Western Blot. The results showed that MVA-S(3P) efficiently expressed the S protein for all the passages, which demonstrates that vaccine candidate MVA-S(3P) is genetically very stable (Figure 3A).

### 2.2. Vaccine candidate MVA-S(3P) expresses high levels of S protein in the cell membrane

To further quantify and determine differences in S expression levels between the MVA-S and MVA-S(3P) recombinants, we next analyzed by flow cytometry the S expression at 24 hpi in HeLa cells mock infected or infected with MVA-S, MVA-S(3P) and MVA-WT (Figure 3B). The results showed that MVA-S(3P) expressed higher levels of the S protein in the cell membrane than MVA-S, either using antibodies against the S or the RBD proteins (Figure 3B).

### 2.3. Vaccine candidate MVA-S(3P) replicates well in permissive cells

To determine if the expression of the coronavirus prefusion-stabilized SARS-CoV-2 S protein affects MVA replication in cell culture, DF-1 cells were infected with vaccine candidates MVA-S and MVA-S(3P), and parental virus MVA-WT, and cell extracts were collected at different times post-infection (0, 24, 48, and 72 hours), and the amount of virus present was determined. The results showed that the viral growth kinetics of vaccine candidates MVA-S and MVA-S(3P), was similar (Figure 3C), and similar to parental MVA.-WT, which demonstrates that the expression of the prefusion-stabilized S protein does not negatively affect MVA vector replication in permissive conditions. Moreover, similarly to parental MVA-WT virus and as expected, MVA-S and MVA-S(3P) does not replicate in human HeLa cells (data not shown).

### 2.4. Vaccine candidate MVA-S(3P) expresses a transmembrane S protein

Furthermore, the S protein expressed by MVA-S(3P) is present in the cell membrane, similar to what happen with MVA-S (Figure 4). In permeabilized cells, large amounts of S protein were detected in the cytoplasm of cells infected with MVA-S or MVA-S(3P) (Figure 4A). Remarkably, in nonpermeabilized cells, we observed S protein cell surface expression, which colocalized with the wheat germ agglutinin (WGA) cell membrane probe (Figure 4B). These results further confirm SARS-CoV-2 S protein expression in cells infected with both MVA-S and MVA-S(3P) vaccine candidates and showed that it was located at the cell surface and cytoplasm.

### 3. Analysis of the SARS-CoV-2-specific adaptive immune response of vaccine candidate MVA-S(3P) in C57BL/6 mice after homologous (MVA/MVA) immunization protocols

The immunogenicity of vaccine candidate MVA-S(3P) in C57BL/6 mice was then evaluated in comparison to MVA-S using homologous (MVA/MVA) prime-boost immunization regimens, and the coronavirus SARS-CoV-2 S-protein-specific adaptive cell-mediated immune responses and humoral immune responses induced by those vaccine candidates were analysed in detail. Therefore, 6 C57BL/6 mice per group were immunized with two doses of each vaccine candidate MVA-S or MVA-S(3P). At 10 days after the first dose, animals were bleed to obtain serum samples, and on day 10 after the boost mice were sacrificed for measuring adaptive SARS-CoV-2-specific cell-mediated immune responses and humoral immune responses (Figure 5A) (see Materials and Methods).

### 3.1. Immunization of C57BL/6 mice with MVA-S(3P) vaccine candidate induces higher levels of binding IgG antibodies against SARS-CoV-2 S and RBD proteins and of SARS-CoV-2-specific neutralizing antibodies than MVA-S after administration of one dose

Next, the total levels of coronavirus SARS-CoV-2 S-protein-specific and S-protein receptor binding domain (RBD)-specific IgG antibodies, the levels of subclasses thereof, i.e., IgG1, IgG2b, IgG2c, and IgG3, and the neutralizing antibodies present in the sera of C57BL/6 mice immunized with MVA-S and MVA-S(3P), were analyzed by means of ELISA 10 days after the prime and 10 days after the second immunization (boost). The animals immunized with MVA-WT were used as a control group.

The results showed that after one dose higher levels of binding IgG antibodies against S and RBD proteins were elicited by MVA-S(3P), compared to MVA-CoV2-S (Figure 5B). However, both vaccines induced similar binding IgG antibodies against S and RBD after the second dose (Figure 5C). The presence of antibodies against the RBD region suggest that those antibodies could be neutralizing antibodies, as this domain is the main region recognized by neutralizing antibodies.

The analysis of the levels of IgG isotypes against S and RBD proteins showed that both vaccine candidates induced higher IgG2c titers than IgG1, leading to a IgG2c/IgG1 ratio higher than 1, indicate of a Th1-like protective humoral response (Figure 6)

Finally, similarly, compared to MVA-S, MVA-S(3P) induced higher titers of neutralizing antibodies after one dose, but both vaccine candidates elicited similar titers of neutralizing antibodies after the second dose (Figure 7).

In conclusion, vaccine candidate MVA-S(3P) induced higher levels of total IgG, IgG1, IgG2b, IgG2c, and IgG3 antibodies against SARS-CoV-2 S- and RBD proteins, and higher neutralizing antibodies than MVA-S, after one dose, but similar after two doses.

### 3.1. Immunization of C57BL/6 mice with two doses of vaccine candidates MVA-S and MVA-S(3P) induces similar high levels of SARS-CoV-2 S-specific cells secreting IFN-γ

The coronavirus SARS-CoV-2 S-protein-specific adaptive cell-mediated immune response was first analysed by means of ELISpot (see Materials and Methods) 10 days after the boost in C57BL/6 mice (n = 6/group) immunized with two doses of MVA-S or MVA-S(3P). As a control group, mice were immunized with two doses of MVA-WT Splenocytes originating from the spleens of each group were stimulated *ex vivo* with a panel of peptide pools covering the complete sequence of the coronavirus SARS-CoV-2 S-protein (S1 and S2 peptide pools). The samples were stimulated with RPMI as a negative control. After 48 hours of stimulation, the cells were labeled with antibodies to identify IFN-γ-secreting cells specific for these peptides (Figure 8).

The results showed that two doses of vaccine candidates MVA-S and MVA-Δ-S(3P), induced similar high levels of S-specific IFN-γ-secreting cells (Figure 8), with the cellular response being directed to a greater extent against S1 peptide pools (data not shown).

### 3.2. Immunization of C57BL/6 mice with two doses of vaccine candidates MVA-S and MVA-S(3P) induces similar strong, broad, and polyfunctional SARS-CoV-2 S-specific adaptive CD4⁺ and CD8⁺ T cell-mediated immune responses

Next, splenocytes originating from the spleens of each immunization group were stimulated *ex vivo* with a panel of peptide pools covering the complete sequence of the coronavirus SARS-CoV-2 S-protein (S1 and S2 peptide pools). The samples were stimulated with RPMI as a negative control. After 6 hours of stimulation, the cells were labelled with specific antibodies to identify (CD4⁺ and CD8⁺) T cell populations and respondent cells (expressing CD107a on the surface of the T cells as an indirect cytotoxicity marker and/or producing cytokines IFN-γ, TNF-α, and IL-2) by ICS.

The results showed that two doses of MVA-S or MVA-S(3P) induced similar levels of coronavirus SARS-CoV-2 S-protein (S1 + S2)-specific adaptive CD4⁺ T cell-mediated (Figure 9A, left) and CD8⁺ T cell-mediated (Figure 9A, right) immune responses, with the levels of specific CD8⁺ T cells being greater than the levels of CD4⁺T cells in both groups. The detailed analysis of the response showed that, in both immunization groups, the coronavirus SARS-CoV-2 S-protein-specific CD4⁺ T cells and CD8⁺ T cells are similarly directed to a greater extent against S1 peptide pools, and also CD4⁺ T cells directed against S2 region are detected (Figure 9B).

These aforementioned responses can also be observed through the analysis of the percentages of S-protein (S1+S2)-specific CD4⁺ T cells (Figure 9C, left) or CD8⁺ T cells (Figure 9C, right) secreting each the cytokines (IFN-y, TNF-α, or IL-2) or expressing the cytotoxicity marker, CD107a. It must be pointed out that specific CD8⁺ T cell responses are characterized by a low IL-2 secretion.

Finally, the polyfunctionality of the CD4⁺ and CD8⁺ T cell-mediated immune response generated by both immunization groups was analyzed by measuring the simultaneous production pattern of cytokines (IFN-y, TNF-α, and/or IL-2) plus the cytotoxic potential thereof (CD107a as a degranulation marker). The coronavirus SARS-CoV-2 S-protein (S1+S2)-specific cell-mediated responses generated by both immunization groups were characterized by a similar high polyfunctionality (Figure 10). The CD4⁺ T cell-mediated response was mainly characterized by cells which are capable of carrying out all 4 functions (secreting all 3 cytokines and expressing the marker, CD107a) (Figure 10A). On the other hand, the CD8⁺ T cell-mediated response was mainly characterized by cells which are capable of carrying out 3 functions (CD107a, IFN-γ and TNF-α) (Figure 10B). In conclusion, two doses of vaccine candidates MVA-S or MVA-S(3P) are capable of inducing in mice similar robust, broad and polyfunctional coronavirus SARS-CoV-2 S-protein-specific adaptive CD4⁺ and CD8⁺ T cell-mediated immune responses.

### 3.3. Immunization of C57BL/6 mice with two doses of vaccine candidates MVA-S and MVA-S(3P) induces similar adaptive SARS-CoV-2 S-specific CD4⁺ and CD8⁺ T cell-mediated immune response with effector memory phenotype

Next, we determined the phenotype of the adaptive SARS-CoV-2-specific CD4+ and CD8+ T cells by measuring the expression of CD127 and CD62L surface markers, which defined different memory subpopulations: T central memory (TCM, CD127+/CD62L+), T effector memory (TEM, CD127+/CD62L⁻), and T effector (TE, CD127⁻/CD62L⁻) cells, and determined the sums of the individual responses expressing CD107a, IFN-γ, TNF-α, and/or IL-2 obtained for the S1, and S2 peptide pools (Figure 11). The results showed that in both vaccinated groups, adaptive SARS-CoV-2-specific CD4+ (Figure 11A) and CD8+ (Figure 11B) T cells were mainly of the TEM phenotype (Figure 11) and with a similar magnitude in both groups.

### 3.4. Immunization of C57BL/6 mice with two doses of vaccine candidates MVA-S and MVA-S(3P) induces adaptive SARS-CoV-2 S-specific CD4⁺ T follicular helper (Tfh) cell-mediated immune response

Next, the proportion of coronavirus SARS-CoV-2 S-specific CD4⁺ T follicular helper (Tfh) cells was analyzed by means of ICS 10 days after the boost in C57BL/6 mice immunized with two doses of MVA-S or MVA-S(3P), as described above.

Splenocytes isolated from the spleen were stimulated *ex vivo* with a combination of the coronavirus SARS-CoV-2 S-protein, together with the S1 and S2 peptide pools covering the complete sequence of said protein. The samples were stimulated with RPMI as a negative control. After 6 hours of stimulation, the cells were labelled with specific antibodies to identify the population of S-specific Tfh cells (CD4⁺, CXCR5⁺, and PD1⁺) expressing the marker CD40L (marker related to T cell effector functions) and/or producing the cytokines IFN-γ and/or IL-21.

The results showed that in both vaccinated groups SARS-CoV-2-specific CD4⁺ Tfh cells expressing CD40L and/or secreting IFN-γ and/or IL-21 were induced (Figure 12A), with MVA-S inducing higher magnitude than MVA-S(3P).

The pattern of SARS-CoV-2-specific CD4⁺ Tfh cells showed that in both vaccinated groups most of the CD4⁺ Tfh secreted IFN-γ and IL-21, followed by the presence of CD40L (Figure 12B).

Finally, the polyfunctionality of the Tfh cell-mediated immune response generated by both immunization groups was analyzed by measuring the simultaneous production pattern of cytokines (IFN-γ and/or IL-21) plus the marker, CD40L. The coronavirus SARS-CoV-2 S-protein-specific Tfh cell-mediated responses generated by both immunization groups were characterized by a high polyfunctionality (Figure 12C). The Tfh cell-mediated response was mainly characterized by cells which are capable of carrying out all 2 functions (secreting IFN-γ and IL-21), followed by cells capable of carrying out 3 functions (secreting the 2 cytokines and expressing the marker, CD40L) (Figure 12C). In conclusion, vaccine candidates MVA-S and MVA-S(3P) induce Tfh cell-mediated responses.

### 4. Analysis of the efficacy of MVA-S(3P) in humanized K18-h ACE2 mice

### 4.1 Morbidity and mortality induced by SARS-CoV-2 in humanized K18-hACE2 mice are completely prevented by vaccination with one dose of MVA-S(3P)

Next, we evaluated the ability of one single dose of MVA-S(3P) to protected against SARS-CoV-2 infection. Thus, for morbidity and mortality evaluation, groups of K18-hACE2 mice (n = 11) were immunized with one dose (1 × 10⁷ PFU by the intramuscular route) of MVA-S or MVA-S(3P) at week 0 and then challenged at week 4 with 1 × 10⁵ PFU of SARS-CoV-2 (MAD6 isolate) by the intranasal route (Figure 13A). Mice primed with MVA-WT were used as control group.

First, to determine the ability of the MVA-S(3P) vaccine candidate to induce SARS-CoV-2-specific humoral immune responses, serum samples from K18-hACE2 humanized mice vaccinated according to the schedule represented in Figure 13A were first obtained at 14 days after the immunization (and previous to the SARS-CoV-2 challenge) and binding IgG antibodies against S and RBD proteins and neutralizing antibodies were analyzed. The results showed that one dose of MVA-S(3P) elicited higher titers of IgG anti-S and anti-RBD antibodies than MVA-S (Figure 13B), similarly to what we observed in the immunogenicity study in wild-type C57BL/6 mice. Moreover, one single dose of MVA-S(3P) also induced significant higher neutralizing antibody titers than MVA-S (Fig. 13C). Next, we analyzed the efficacy of one dose of MVA-S(3P) in challenged K18-hACE2 mice, which were monitored for change in body weight and mortality for 15 days postchallenge (Figure 13D). All the K18-hACE2 mice immunized with one dose of MVA-S(3P) and challenged with SARS-CoV-2 did not loss body weight and survived (Figure 13D), compared with mice immunized with one dose of MVA-S that lost body weight during the first days post-challenge, but by day 4, they recovered and all survived (Figure 13D). MVA-WT-immunized and challenged K18-hACE2 mice showed loss body weight (more than 25%) and died within 6-7 days postchallenge (Figure 13D). Thus, one dose of the vaccine MVA-S(3P) [also known as MVA-CoV2-S(3P)] fully protected (100%) humanized mice against the morbidity and lethality effects of SARS-CoV-2 infection.

**4.2. MVA-S(3P) vaccine candidate is highly efficacious in K18-hACE2 humanized mice, preventing SARS-CoV-2 virus replication, and induced high levels of binding IgG antibodies against S protein.** Next, to examine the effect of the MVA-S(3P) vaccine candidate in virus replication, six mice per group were sacrificed at day 4 after SARS-CoV-2 virus challenge, lung tissue samples were collected and processed, and we analysed the presence of SARS-CoV-2 genomic RNA (Fig. 14A) and of live infectious virus (Fig. 14B). One dose of MVA-S(3P) was highly effective to completely prevent SARS-CoV-2 replication and virus yields at 4 days postchallenge, in comparison with MVA-S or in comparison to the high levels of SARS-CoV-2 RNA and infectious virus detected in control infected mice (Fig. 14A and 14B).

The analysis of the SARS-CoV-2-specific humoral immunogenicity triggered by the MVA-S(3P) vaccine candidate after SARS-CoV-2 challenge showed that mice immunized with one dose of MVA-S(3P) elicited high titers of S-specific binding IgG antibodies at days 4 and 15 post-challenge (Fig. 14C). The IgG titers elicited by MVA-S(3P) were higher than those induced by MVA-S at day 4 post-challenge (similarly to what happened at the prechallenged state, Fig. 13B), whereas at day 15 postchallenge the MVA-S vaccine induced higher titers than MVA-S(3P). In both groups an anamnestic response was observed.

### SEQUENCE LISTING

The full-length SARS-CoV-2 S sequence inserted in vaccine candidate MVA-S(3P) described in the present examples was synthetized by GeneArt, was human codon optimized, and contains three mutations in the furin cleavage site (R682G, R683S, R685S) that avoid the cleavage of the S protein in S1 and S2, and three additional mutations in the S2 region that stabilizes the S protein in a prefusion conformation (A942P, K986P, and V987P). The sequence was derived from Wuhan seafood market pneumonia virus isolate Wuhan-Hu-1 (GenBank: MN908947.3, https://www.ncbi.nlm.nih.gov/nuccore/MN908947). The sequence is reproduced herein below as SEQ ID NO 1.
- The S nucleotide sequence reads as follows (SEQ ID NO 1): 
ATGTTCGTGTTTCTGGTGCTGCTGCCTCTGGTGTCCAGCCAGTGTGTGAACCTGAC
CACCAGAACACAGCTGCCTCCAGCCTACACCAACAGCTTTACCAGAGGCGTGTAC
TACCCCGACAAGGTGTTCAGATCCAGCGTGCTGCACTCTACCCAGGACCTGTTCC
TGCCTTTCTTCAGCAACGTGACCTGGTTCCACGCCATCCACGTGTCCGGCACCAAT
GGCACCAAGAGATTCGACAACCCCGTGCTGCCCTTCAACGACGGGGTGTACTTTG
CCAGCACCGAGAAGTCCAACATCATCAGAGGCTGGATCTTCGGCACCACACTGGA
CAGCAAGACCCAGAGCCTGCTGATCGTGAACAACGCCACCAACGTGGTCATCAAA
GTGTGCGAGTTCCAGTTCTGCAACGACCCCTTCCTGGGCGTCTACTACCACAAGA
ACAACAAGAGCTGGATGGAAAGCGAGTTCCGGGTGTACAGCAGCGCCAACAACTG
 
CACCTTCGAGTACGTGTCCCAGCCTTTCCTGATGGACCTGGAAGGCAAGCAGGGC
AACTTCAAGAACCTGCGCGAGTTCGTGTTCAAGAACATCGACGGCTACTTCAAGAT
CTACAGCAAGCACACCCCTATCAACCTCGTGCGGATCTGCCTCAGGGCTTCTCT
GCTCTGGAACCCCTGGTGGATCTGCCCATCGGCATCAACATCACCCGGTTTCAGA
CACTGCTGGCCCTGCACAGAAGCTACCTGACACCTGGCGATAGCAGCAGCGGAT
GGACAGCTGGTGCCGCCGCTTACTATGTGGGCTACCTGCAGCCTAGAACCTTCCT
GCTGAAGTACAACGAGAACGGCACCATCACCGACGCCGTGGATTGTGCTCTGGAT
CCTCTGAGCGAGACAAAGTGCACCCTGAAGTCCTTCACCGTGGAAAAGGGCATCT
ACCAGACCAGCAACTTCCGGGTGCAGCCCACCGAATCCATCGTGCGGTTCCCCAA
TATCACCAATCTGTGCCCCTTCGGCGAGGTGTTCAATGCCACCAGATTCGCCTCTG
TGTACGCCTGGAACCGGAAGCGGATCAGCAATTGCGTGGCCGACTACTCCGTGCT
GTACAACTCCGCCAGCTTCAGCACCTTCAAGTGCTACGGCGTGTCCCCTACCAAG
CTGAACGACCTGTGCTTCACAAACGTGTACGCCGACAGCTTCGTGATCCGGGGAG
ATGAAGTGCGGCAGATTGCCCCTGGACAGACAGGCAAGATCGCCGACTACAACTA
CAAGCTGCCCGACGACTTCACCGGCTGTGTGATTGCCTGGAACAGCAACAACCTG
GACTCCAAAGTCGGCGGCAACTACAATTACCTGTACCGGCTGTTCCGGAAGTCCA
ATCTGAAGCCCTTCGAGCGGGACATCTCCACCGAGATCTATCAGGCCGGCAGCAC
CCCTTGTAACGGCGTGGAAGGCTTCAACTGCTACTTCCCACTGCAGTCCTACGGC
TTTCAGCCCACAAATGGCGTGGGCTATCAGCCCTACAGAGTGGTGGTGCTGAGCT
TCGAACTGCTGCATGCCCCTGCCACAGTGTGCGGCCCTAAGAAAGCACCAATCT
CGTGAAGAACAAATGCGTGAACTTCAACTTCAACGGCCTGACCGGCACCGGCGTG
CTGACAGAGAGCAACAAGAAGTTCCTGCCATTCCAGCAGTTTGGCCGGGATATCG
CCGATACCACAGACGCCGTTAGAGATCCCCAGACACTGGAAATCCTGGACATCAC
CCCTTGCAGCTTCGGCGGAGTGTCTGTGATCACCCCTGGCACCAACACCAGCAAT
CAGGTGGCAGTGCTGTACCAGGACGTGAACTGTACCGAAGTGCCCGTGGCCATTC
ACGCCGATCAGCTGACACCTACATGGCGGGTGTACTCCACCGGCAGCAATGTGTT
TCAGACCAGAGCCGGCTGTCTGATCGGAGCCGAGCACGTGAACAATAGCTACGAG
TGCGACATCCCCATCGGCGCTGGCATCTGTGCCAGCTACCAGACACAGACAAACA
GCCCCGGGAGTGCAAGTTCTGTGGCCAGCCAGAGCATCATTGCCTACACAATGTC
TCTGGGCGCCGAGAACAGCGTGGCCTACTCCAACAACTCTATCGCTATCCCCACC
AACTTCACCATCAGCGTGACCACAGAGATCCTGCCTGTGTCCATGACCAAGACCA
GCGTGGACTGCACCATGTACATCTGCGGCGATTCCACCGAGTGCTCCAACCTGCT
GCTGCAGTACGGCAGCTTCTGCACCCAGCTGAATAGAGCCCTGACAGGGATCGCC
GTGGAACAGGACAAGAACACCCAAGAGGTGTTCGCCCAAGTGAAGCAGATCTACA
AGACCCCTCCTATCAAGGACTTCGGCGGCTTCAATTTCAGCCAGATTCTGCCCGAT
 
CCTAGCAAGCCCAGCAAGCGGAGCTTCATCGAGGACCTGCTGTTCAACAAAGTGA
CACTGGCCGACGCCGGCTTCATCAAGCAGTATGGCGATTGTCTGGGCGACATTGC
CGCCAGGGATCTGATTTGCGCCCAGAAGTTTAACGGACTGACAGTGCTGCCTCCT
CTGCTGACCGATGAGATGATCGCCCAGTACACATCTGCCCTGCTGGCCGGCACAA
TCACAAGCGGCTGGACATTTGGAGCTGGCGCCGCTCTGCAGATCCCCTTTGCTAT
GCAGATGGCCTACCGGTTCAACGGCATCGGAGTGACCCAGAATGTGCTGTACGAG
AACCAGAAGCTGATCGCCAACCAGTTCAACAGCGCCATCGGCAAGATCCAGGACA
GCCTGAGCAGCACACCAAGCGCCCTGGGAAAGCTGCAGGACGTGGTCAACCAGA
ATGCCCAGGCACTGAACACCCTGGTCAAGCAGCTGTCCTCCAACTTCGGCGCCAT
CAGCTCTGTGCTGAACGATATCCTGAGCAGACTGGACCCACCTGAAGCCGAGGTG
CAGATCGACAGACTGATCACCGGAAGGCTGCAGTCCCTGCAGACCTACGTTACCC
AGCAGCTGATCAGAGCCGCCGAGATTAGAGCCTCTGCCAATCTGGCCGCCACCAA
GATGTCTGAGTGTGTGCTGGGCCAGAGCAAGAGAGTGGACTTTTGCGGCAAGGG
CTACCACCTGATGAGCTTCCCTCAGTCTGCCCCTCACGGCGTGGTGTTTCTGCAC
GTGACATACGTGCCCGCTCAAGAGAAGAATTTCACCACCGCTCCAGCCATCTGCC
ACGACGGCAAAGCCCACTTTCCTAGAGAAGGCGTGTTCGTGTCCAACGGCACCCA
TTGGTTCGTGACCCAGCGGAACTTCTACGAGCCCCAGATCATCACCACCGACAAC
ACCTTCGTGTCTGGCAACTGCGACGTCGTGATCGGCATTGTGAACAATACCGTGTA
CGACCCTCTGCAGCCCGAGCTGGACAGCTTCAAAGAGGAACTGGATAAGTACTTT
AAGAACCACACAAGCCCCGACGTGGACCTGGGCGATATCAGCGGAATCAATGCCA
GCGTCGTGAACATCCAGAAAGAGATCGACCGGCTGAACGAGGTGGCCAAGAATCT
GAACGAGAGCCTGATCGACCTGCAAGAACTGGGGAAGTACGAGCAGTACATCAAG
TGGCCCTGGTACATCTGGCTGGGCTTTATCGCCGGACTGATTGCCATCGTGATGG
TCACAATCATGCTGTGTTGCATGACCAGCTGCTGTAGCTGCCTGAAGGGCTGTTGT
AGCTGTGGCAGCTGCTGCAAGTTCGACGAGGACGATTCTGAGCCCGTGCTGAAG
GGCGTGAAACTGCACTACACCTGAGCGGCCGC
- The S protein sequence reads as follows (SEQ ID NO 2): 
MFVFLVLLPLVSSQCVNLTTRTQLPPAYTNSFTRGVYYPDKVFRSSVLHSTQDLFLPFF
SNVTWFHAIHVSGTNGTKRFDNPVLPFNDGVYFASTEKSNIIRGWIFGTTLDSKTQSLLI
VNNATNVVIKVCEFQFCNDPFLGVYYHKNNKSWMESEFRVYSSANNCTFEYVSQPFL
MDLEGKQGNFKNLREFVFKNIDGYFKIYSKHTPINLVRDLPQGFSALEPLVDLPIGINITR
FQTLLALHRSYLTPGDSSSGWTAGAAAYYVGYLQPRTFLLKYNENGTITDAVDCALDP
LSETKCTLKSFTVEKGIYQTSNFRVQPTESIVRFPNITNLCPFGEVFNATRFASVYAWN
 
RKRISNCVADYSVLYNSASFSTFKCYGVSPTKLNDLCFTNVYADSFVIRGDEVRQIAPG
QTGKIADYNYKLPDDFTGCVIAWNSNNLDSKVGGNYNYLYRLFRKSNLKPFERDISTEI
YQAGSTPCNGVEGFNCYFPLQSYGFQPTNGVGYQPYRVVVLSFELLHAPATVCGPKK
STNLVKNKCVNFNFNGLTGTGVLTESNKKFLPFQQFGRDIADTTDAVRDPQTLEILDIT
PCSFGGVSVITPGTNTSNQVAVLYQDVNCTEVPVAIHADQLTPTWRVYSTGSNVFQTR
AGCLIGAEHVNNSYECDIPIGAGICASYQTQTNSPGSASSVASQSIIAYTMSLGAENSVA
YSNNSIAIPTNFTISVTTEILPVSMTKTSVDCTMYICGDSTECSNLLLQYGSFCTQLNRAL
TGIAVEQDKNTQEVFAQVKQIYKTPPIKDFGGFNFSQILPDPSKPSKRSFIEDLLFNKVT
LADAGFIKQYGDCLGDIAARDLICAQKFNGLTVLPPLLTDEMIAQYTSALLAGTITSGWT
FGAGAALQIPFAMQMAYRFNGIGVTQNVLYENQKLIANQFNSAIGKIQDSLSSTPSALG
KLQDVVNQNAQALNTLVKQLSSNFGAISSVLNDILSRLDPPEAEVQIDRLITGRLQSLQT
YVTQQLIRAAEIRASANLAATKMSECVLGQSKRVDFCGKGYHLMSFPQSAPHGVVFLH
VTYVPAQEKNFTTAPAICHDGKAHFPREGVFVSNGTHWFVTQRNFYEPQIITTDNTFV
SGNCDVVIGIVNNTVYDPLQPELDSFKEELDKYFKNHTSPDVDLGDISGINASVVNIQKE
IDRLNEVAKNLNESLIDLQELGKYEQYIKWPWYIWLGFIAGLIAIVMVTIMLCCMTSCCS
CLKGCCSCGSCCKFDEDDSEPVLKGVKLHYT
- The RBD nucleotide sequence described in the specification as SEQ ID NO 3 reads as follows (SEQ ID NO 3): 
ATGGAAACCGACACACTGCTGCTGTGGGTGCTGCTTTTGTGGGTGCCAGGCAGCA
CAGGCGATTACCCCGGGTCTAGATTCCCCAACATCACCAATCTGTGCCCCTTCGG
CGAGGTGTTCAATGCCACAAGATTTGCCAGCGTGTACGCCTGGAACCGGAAGAGA
ATCAGCAACTGCGTGGCCGACTACAGCGTGCTGTACAATAGCGCCAGCTTCAGCA
CCTTCAAGTGCTACGGCGTGTCCCCTACCAAGCTGAACGACCTGTGCTTCACCAAT
GTGTACGCCGACAGCTTCGTGATCAGAGGCGACGAAGTTCGGCAGATCGCTCCTG
GACAGACAGGCAAGATCGCCGATTACAACTACAAGCTGCCCGACGACTTCACCGG
CTGCGTGATCGCCTGGAATAGCAACAACCTGGACAGCAAAGTCGGCGGCAACTAC
AACTACCTGTACCGGCTGTTCCGGAAGTCCAACCTGAAGCCTTTCGAGCGGGACA
TCAGCACCGAGATCTATCAGGCCGGCAGCACCCCTTGTAATGGCGTGGAAGGCTT
CAACTGCTACTTCCCACTGCAGTCCTACGGCTTCCAGCCTACAAACGGCGTGGGC
TACCAGCCTTATAGAGTGGTGGTGCTGAGCTTCGAACTGCTGCATGCCCCTGCTA
CCGTGTGTGGCCCTAAGAAGTCCACCAACTGA
- The RBD protein sequence described in the specification as SEQ ID NO 4 reads as follows (SEQ ID NO 4): 
METDTLLLWVLLLWVPGSTGDYPGSRFPNITNLCPFGEVFNATRFASVYAWNRKRISN
CVADYSVLYNSASFSTFKCYGVSPTKLNDLCFTNVYADSFVIRGDEVRQIAPGQTGKIA
DYNYKLPDDFTGCVIAWNSNNLDSKVGGNYNYLYRLFRKSNLKPFERDISTEIYQAGS
TPCNGVEGFNCYFPLQSYGFQPTNGVGYQPYRVVVLSFELLHAPATVCGPKKSTN
- The E nucleotide sequence described in the specification as SEQ ID NO 5 reads as follows (SEQ ID NO 5): 
ATGTACAGCTTCGTGTCCGAGGAAACCGGCACACTGATCGTGAACAGCGTGCTGC
TGTTCCTGGCCTTCGTGGTGTTCCTGCTGGTTACCCTGGCTATCCTGACAGCCCTG
AGACTGTGCGCCTACTGCTGCAACATCGTGAACGTGTCCCTCGTGAAGCCCAGCT
TCTACGTGTACAGCAGAGTGAAGAACCTGAACTCCAGCAGGGTGCCCGACCTGCT
GGTCTAA
- The E protein sequence described in the specification as SEQ ID NO 6 reads as follows (SEQ ID NO 6): 
MYSFVSEETGTLIVNSVLLFLAFVVFLLVTLAILTALRLCAYCCNIVNVSLVKPSFYVYSR
VKNLNSSRVPDLLV
- The N nucleotide sequence described in the specification as SEQ ID NO 7 reads as follows (SEQ ID NO 7): 
ATGAGCGACAACGGCCCTCAGAATCAGCGGAACGCCCCTAGAATCACATTTGGCG
GCCCTAGCGATAGCACCGGCAGCAATCAGAATGGCGAGAGAAGCGGCGCCAGAA
GCAAACAGAGAAGGCCTCAGGGCCTGCCTAACAACACCGCCTCTTGGTTTACAGC
CCTGACACAGCACGGCAAAGAGGACCTGAAGTTTCCTAGAGGCCAGGGCGTGCC
CATCAACACCAATAGCAGCCCTGATGACCAGATCGGCTACTACAGACGGGCCACC
AGAAGAATTAGAGGCGGCGACGGCAAGATGAAGGATCTGAGCCCCAGATGGTACT
TCTACTACCTCGGAACAGGACCCGAGGCCGGACTTCCTTATGGCGCCAACAAGGA
TGGCATCATCTGGGTTGCAACAGAAGGCGCCCTGAACACCCCTAAGGACCACATC
GGAACCAGAAATCCCGCCAACAATGCCGCCATTGTGCTGCAGTTGCCACAGGGCA
 
CAACCCTGCCTAAGGGCTTTTATGCCGAGGGCTCTAGAGGCGGAAGCCAGGCCTC
TAGCAGAAGCAGCAGCAGAAGCCGGAACTCCAGCAGAAATAGCACCCCTGGAAG
CTCCAGAGGCACATCTCCTGCCAGAATGGCCGGAAATGGCGGAGATGCTGCTCTT
GCTCTGCTGCTCCTGGATCGGCTGAACCAGCTGGAAAGCAAGATGTCTGGAAAGG
GCCAGCAGCAACAGGGCCAGACCGTGACAAAAAATCTGCCGCCGAGGCCAGCA
AGAAGCCCAGACAGAAGAGAACCGCCACCAAGGCCTACAACGTGACCCAGGCCTT
TGGAAGAAGAGGCCCAGAGCAGACCCAGGGCAATTTCGGCGATCAAGAGCTGAT
CAGACAGGGCACCGACTACAAGCACTGGCCTCAGATTGCCCAGTTTGCCCCTAGC
GCCTCCGCCTTCTTTGGCATGTCTAGAATCGGCATGGAAGTGACCCCTAGCGGCA
CCTGGTTGACATACACAGGCGCCATCAAGCTGGACGACAAGGACCCCAACTTCAA
GGACCAAGTGATCCTGCTGAACAAGCACATCGACGCCTACAAGACATTCCCTCCA
ACCGAGCCTAAGAAGGACAAGAAGAAGAAGGCCGACGAGACACAGGCCCTGCCA
CAGAGACAGAAAAACAGCAGACAGTGACCCTGCTGCCTGCCGCTGATCTGGACG
ATTTTTCCAAGCAGCTGCAGCAGTCCATGAGCAGCGCAGATAGCACACAGGCCTG
A
- The N protein sequence described in the specification as SEQ ID NO 8 reads as follows (SEQ ID NO 8): 
MSDNGPQNQRNAPRITFGGPSDSTGSNQNGERSGARSKQRRPQGLPNNTASWFTA
LTQHGKEDLKFPRGQGVPINTNSSPDDQIGYYRRATRRIRGGDGKMKDLSPRWYFYY
LGTGPEAGLPYGANKDGIIWVATEGALNTPKDHIGTRNPANNAAIVLQLPQGTTLPKGF
YAEGSRGGSQASSRSSSRSRNSSRNSTPGSSRGTSPARMAGNGGDAALALLLLDRL
NQLESKMSGKGQQQQGQTVTKKSAAEASKKPRQKRTATKAYNVTQAFGRRGPEQT
QGNFGDQELIRQGTDYKHWPQIAQFAPSASAFFGMSRIGMEVTPSGTWLTYTGAIKLD
DKDPNFKDQVILLNKHIDAYKTFPPTEPKKDKKKKADETQALPQRQKKQQTVTLLPAAD
LDDFSKQLQQSMSSADSTQA
- The M nucleotide sequence described in the specification as SEQ ID NO 9 reads as follows (SEQ ID NO 9): 
ATGGCCGATAGCAATGGCACCATCACCGTGGAAGAACTGAAGAAACTGCTGGAAC
AGTGGAACCTCGTGATCGGCTTCCTGTTCCTGACCTGGATCTGCCTGCTGCAGTT
CGCCTACGCCAACCGGAACAGATTCCTGTATATCATCAAGCTGATCTTTCTGTGGC
 
TGCTGTGGCCCGTGACACTGGCCTGTTTTGTGCTGGCCGCCGTGTACAGAATCAA
CTGGATCACCGGCGGAATCGCCATTGCCATGGCTTGTCTTGTGGGCCTGATGTGG
CTGAGCTACTTTATCGCCAGCTTCCGGCTGTTCGCCCGGACCAGATCCATGTGGT
CCTTCAATCCCGAGACAAACATCCTGCTGAACGTGCCCCTGCACGGCACCATTCT
GACAAGACCTCTGCTGGAAAGCGAGCTGGTTATCGGCGCCGTGATCCTGAGAGGA
CACCTGAGAATTGCCGGCCACCACCTGGGCAGATGCGATATCAAGGACCTGCCTA
AAGAAATCACAGTGGCCACCAGCAGAACCCTGTCCTACTATAAGCTGGGCGCCTC
TCAGAGAGTGGCCGGCGATAGCGGATTTGCCGCCTACAGCAGATACCGGATCGG
CAACTACAAGCTGAACACCGACCACAGCTCCAGCAGCGACAATATCGCACTGCTG
GTGCAGTGA
- The M protein sequence described in the specification as SEQ ID NO 10 reads as follows (SEQ ID NO 10):

MADSNGTITVEELKKLLEQWNLVIGFLFLTWICLLQFAYANRNRFLYIIKLIFLWLLWPVT
LACFVLAAVYRINWITGGIAIAMACLVGLMWLSYFIASFRLFARTRSMWSFNPETNILLN
VPLHGTILTRPLLESELVIGAVILRGHLRIAGHHLGRCDIKDLPKEITVATSRTLSYYKLGA
SQRVAGDSGFAAYSRYRIGNYKLNTDHSSSSDNIALLVQ

## Claims

1. A **vaccine composition** comprising an immunologically effective amount of a modified vaccinia virus Ankara (MVA) vector comprising at least one nucleic acid encoding the Spike (S) protein, or a fragment of said S protein comprising at least one epitope, of at least one SARS-CoV-2 variant, wherein said S protein or fragment thereof, comprises at least the substitutions R682G, R683S, R685S, A942P, K986P and V987P, and wherein the MVA vector regulates the expression of the nucleic acid encoding the S protein, or the fragment thereof.

2. The vaccine composition according to claim 1, wherein the nucleic acid encoding for the S protein, or a fragment thereof, of at least one SARS-CoV-2 variant is inserted into the thymidine kinase (TK) locus of the MVA genome.

3. The vaccine composition according to any of claims 1 and 2, wherein the nucleic acid encoding for the S protein, or a fragment thereof, of at least one SARS-CoV-2 variant is operably linked to an early/late promoter from Vaccinia Virus (VACV), preferably the synthetic early/late promoter.

4. The vaccine composition according to any of claims 1 to 3, wherein the nucleic acid encoding for the S protein, or a fragment thereof, of at least one SARS-CoV-2 variant is inserted into the TK locus of the MVA genome and is operably linked to an early/late promoter from VACV, preferably the synthetic early/late promoter.

5. The vaccine composition according to any of claims 1 to 4, wherein the nucleic acid comprised in the MVA vector further encodes for one or more antigenic proteins selected from the group consisting of structural proteins E (envelope), receptor-binding domain (RBD), N (nucleocapsid) or M (membrane), or any combination thereof, from at least one SARS-CoV-2 variant.

6. The vaccine composition according to claims 1 to 5, wherein at least one SARS-CoV-2 variant is selected from the group consisting of Wuhan-Hu-1 seafood market pneumonia virus isolate (GenBank accession number: MN908947), Linage B.1.1.28 (Brazilian variant), Linage B.1.351 (South African variant), Linage B.1.427 or Linage B.1.429 (California variant), Linage B.1.617 (Indian variant) or Linage B.1.1.7 (United Kingdom variant), or any combination thereof.

7. The vaccine composition according to any of claims 1 to 6, wherein the vaccine further comprises one or more pharmaceutically acceptable carriers, additives, antibiotics, preservatives, adjuvants, diluents and/or stabilizers.

8. A **vaccine combination** comprising:
a. a first composition comprising an immunologically effective amount of a MVA vector as defined in any claims 1 to 7; and
b. a second composition comprising an immunologically effective amount of a MVA vector as defined in any of claims 1 to 7;
wherein one of the compositions is a priming composition and the other composition is a boosting composition.

9. A **vaccine combination** comprising:
a. a first composition comprising an immunologically effective amount of a MVA vector as defined in any of claims 1 to 7; and
b. a second composition comprising an immunologically effective amount of at least one immunogen derived from at least one SARS-CoV-2 variant;
wherein one of the compositions is a priming composition and the other composition is a boosting composition.

10. The vaccine combination according to claim 9, wherein the at least one immunogen derived from at least one SARS-CoV-2 variant is selected from the group consisting of viral vectors, recombinant proteins or polypeptides, nucleic acids, virus-like particles, nanoparticles, or any combination thereof.

11. The vaccine composition according to any of claims 1 to 7, **for use** in generating an immunogenic and/or protective immune response in a subject in need thereof, preferably in a human subject, wherein said immune response is against at least the S protein, or a fragment of said S protein, comprising the substitutions R682G, R683S, R685S, A942P, K986P and V987P and derived from at least one SARS-CoV-2 variant, wherein said composition is used for priming and/or for boosting said immune response, and wherein the MVA is capable of producing said S protein, or fragment thereof, in the subject to be treated.

12. The vaccine combination according to any of claims 8 to 10, **for use** in generating an immunogenic and/or protective immune response in a subject in need thereof, preferably in a human subject, wherein said immune response is against at least the S protein, or a fragment of said S protein, comprising the substitutions R682G, R683S, R685S, A942P, K986P and V987P and derived from at least one SARS-CoV-2 variant, and wherein said combination is capable of producing said S protein, or fragment thereof, in the subject to be treated.

13. The vaccine composition according to any of claims 1 to 7 or the vaccine combination according to claims 8 to 10, **for use** to induce an enhanced immune response against at least one SARS-CoV-2 variant in a subject in need thereof, preferably in a human subject, wherein the MVA is capable of producing one or more proteins, or fragments thereof, selected from the group consisting of proteins S, E, RBD, N and/or M, or any combination thereof, derived from at least one SARS-CoV-2 variant, in the subject to be treated.

14. The vaccine composition for use according to any of claims 11 to 13 wherein the MVA vector/s of the vaccine composition/s is/are to be administered once, twice, three or more times.

15. The vaccine composition or combination for use according to any of claims 11 to 14, wherein said composition or combination is administered via the intramuscular or intranasal route.

16. The vaccine composition or combination for use according to any of claims 11 to 15, wherein said composition or combination are administered as a boosting composition at least 2 weeks after administering a priming composition.

17. The vaccine composition or combination for use according to any of claims 11 to 15, wherein said composition or combination are administered as a boosting composition 2-12 weeks, preferably 2, 3 or 4 weeks, after administering the priming composition.

18. A kit comprising one, two, or more doses of the vaccine composition as defined in any of claims 1 to 7, or the vaccine combination as defined in any of claims 8 to 10.

19. A kit comprising an immunologically effective amount of a MVA vector according to any of claims 1 to 7 in a first container for a first administration (priming) and in a second container for a second administration (boosting).

20. A kit comprising an immunologically effective amount of a MVA vector according to any of claims 1 to 7 in a container for a first or a second administration, wherein the kit further comprises at least another container comprising an immunologically effective amount of another immunogen derived from at least one SARS-CoV-2 variant for a first or a second administration.

21. A kit comprising an immunologically effective amount of an immunogen derived from at least one SARS-CoV-2 variant in a first container for a first administration (priming), and an immunologically effective amount of a MVA vector according to any of claims 1 to 7 in a second container for a second administration (boosting).

22. The kit according to any of claims 18 to 21, comprising in a third, fourth or further containers a MVA vector according to any of claims 1 to 8 for a third, fourth or further administration.

23. The kit according to claims 18 to 22, for use in generating an immunogenic and/or protective immune response against at least one SARS-CoV-2 variant in a subject in need thereof, preferably in a human subject.

24. The kit according to claim 23, wherein the combination of compositions contained in the containers is capable of producing at least the S protein, or a fragment thereof, comprising the substitutions R682G, R683S, R685S, A942P, K986P and V987P derived from at least one SARS-CoV-2 variant, in the subject to be treated, and generating an immunogenic and/or protective immune response against at least one SARS-CoV-2 variant in a subject in need thereof.

25. The kit for use according to any of claims 23 or 24, wherein said compositions comprised in the containers are administered via the intramuscular or intranasal route.

26. The kit for use according to any of claims 23 to 25, wherein the boosting composition is administered at least 2 weeks after administering the priming composition.

27. The kit for use according to any of claims 28 to 25, wherein the boosting composition is administered 2-12 weeks, preferably 2, 3 or 4 weeks, after administering the priming composition.

28. A method of generating an immunogenic and/or protective immune response against the SARS-CoV-2 virus in a subject in need thereof, preferably in a human subject, the method comprising administering to the subject the vaccine compositions according to any of claims 1 to 7 or any of the vaccine combinations according to any of claims 8 to 10.

29. The method according to claim 28, wherein said method comprises administering the vaccine compositions according to any of claims 1 to 7 for priming said immune response and/or for boosting said immune response.

30. The method according to claim 28, wherein said compositions or combinations of compositions contained in the containers are administered via the intramuscular or intranasal route.

31. The method according to any of claims 28 to 30, wherein the boosting composition is administered at least 2 weeks after administering the priming composition.

32. The method according to any of claims 28 to 31, wherein the boosting composition is administered 2-12 weeks, preferably 2, 3 or 4 weeks, after administering the priming composition.

33. The method according to any of claims 28 to 32, wherein the boosting composition is administered two or more times to the subject.

34. A method for generating an immune response against at least one SARS-CoV-2 variants in a subject in need thereof, preferably a human subject, comprising administering to the subject any the vaccine composition according to any of claims 1 to 7 or any of the vaccine combinations according to any of claims 8 to 10.

35. The method according to claim 34, wherein said method comprises administering the vaccine compositions according to any of claims 1 to 8 for priming said immune response and/or boosting said immune response.

36. The method according to claim 33 or 34, wherein said compositions or combinations of compositions comprised in the containers are administered via the intramuscular or intranasal route.

37. The method according to any of claims 34 to 36, wherein the boosting composition is administered at least 2 weeks after administering the priming composition.

38. The method according to any of claims 34 to 36, wherein the boosting composition is administered 2-12 weeks, preferably 2, 3 or 4 weeks, after administering the priming composition.

39. The method according to any of claims 34 to 38, wherein the boosting composition is administered two or more times to the subject.

40. A method of inducing an enhanced immune response against at least one SARS-CoV-2 variant in a subject in need thereof, preferably in a human subject, comprising administering to the subject the vaccine composition according to any of claims 1 to 7 or any of the vaccine combinations according to any of claims 8 to 10.

41. The method according to claim 40, wherein said compositions or combinations of compositions comprised in the containers are administered via the intramuscular or intranasal route.
